(19) Europäisches Patentamt
European Patent Office
Office européen des brevets

(11) EP 4 410 316 A1

(12) EUROPEAN PATENT APPLICATION

(43) Date of publication:
**07.08.2024 Bulletin 2024/32**

(21) Application number: **23382091.9**

(22) Date of filing: **01.02.2023**

(51) International Patent Classification (IPC):
***A61K 47/69*** *(2017.01)*

(52) Cooperative Patent Classification (CPC):
**A61K 47/6929; A61K 47/6455; A61K 47/6911**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **4basebio UK Ltd**
  **Cambridge CB24 5QE (GB)**
- **4basebio, S.L.U.**
  **28049 Cantoblanco (Madrid) (ES)**

(72) Inventors:
- **LANCKRIET, Heikki**
  **Cambridge, CB24 5QE (GB)**
- **WALKER, Amy**
  **Cambridge, CB24 5QE (GB)**
- **PICHER, Ángel**
  **E-28049 Cantoblanco, Madrid (ES)**
- **YOUNG, Emily**
  **Cambridge, CB24 5QE (GB)**
- **LU, Yu**
  **Cambridge, CB24 5QE (GB)**

(74) Representative: **Boult Wade Tennant LLP**
**Salisbury Square House**
**8 Salisbury Square**
**London EC4Y 8AP (GB)**

Remarks:
The complete document including Reference Table(s) and the Sequence Listing(s) can be downloaded from the EPO website

# (54) NANOPARTICLES FOR DELIVERY OF NUCLEIC ACID CARGOS

(57) Nanoparticles suitable for delivery of a linear DNA molecule are provided. Nanoparticles suitable for delivery of mRNA are provided. Further provided are uses of the nanoparticles including the use of the nanoparticles for treating disease and the use of the nanoparticles in vaccines.

EP 4 410 316 A1

## Description

### TECHNICAL FIELD

Nanoparticles suitable for delivery of a linear DNA molecule are provided. Nanoparticles suitable for delivery of mRNA are provided. Further provided are uses of the nanoparticles including the use of the nanoparticles for treating disease and the use of the nanoparticles in vaccines.

### BACKGROUND

DNA and mRNA vaccines use nucleic acid material coding for a specific pathogen's spike protein, or a cancer antigen, to trigger an immune response.

Nucleic acid vaccines are significantly easier and more cost-effective to manufacture than conventional vaccine platforms, including attenuated or inactivated proteins, or viral vectors.

The success of mRNA COVID vaccines from BioNTech and Moderna have demonstrated the safety and efficacy of mRNA vaccines, but they come with limitations such as cold-chain requirements. DNA vaccines, including the ZyCoV-D vaccine licensed in India, are more thermo-stable than mRNA vaccines, but have their own challenges, namely the requirement of electroporation as a delivery method, which is difficult to roll out in a pandemic situation.

As an alternative, viruses as delivery agents have the advantages of high efficiency and high cell selectivity, although they have the disadvantages of toxicity, risk of insertional mutagenesis, production of inflammatory responses, high likelihood of eliciting a host immune response or limited packaging capacity.

Non-viral gene delivery systems are based on the compaction of genetic material into nanometric particles by electrostatic interaction between the negatively charged phosphate backbone of nucleic acids and cationic lipids, peptides or other polymers (Erbacher, P. et al, Gene Therapy, 1999, 6, 138-145). Known complexes for delivery include lipoplex for lipid based nucleic acid complexes, polyplex for peptide or polymer-based complexes and lipopolyplex for hybrid systems (Felgner et al., Human Gene Therapy 8, 1997, 511-512).

Non-viral lipid vector formulations which complex nucleic acid with cationic lipids suffer from problems of poor tissue penetration, non-specific charge-mediated binding to cells, and interactions with serum proteins which can lead to inflammatory responses. Using an ionizable lipid as an alternative is appealing because it offers the possibility of lower cytotoxicity and less interaction with serum components.

Known non-viral vectors are able to deliver a DNA molecule or an RNA molecule to a cell. In the context of a DNA vaccine plasmid DNA molecules suffer from many drawbacks, for example, they contain bacterial backbone, antibiotic resistant genes and bacterial contaminants which might be toxic for cells or trigger an unwanted immune response.

There exists a need for an improved delivery system for nucleic acid vaccines, and other applications.

### Description

The present invention relates to a nanoparticle suitable for delivery of a cargo, in particular suitable for use in a DNA or RNA vaccine to deliver a cargo e.g. intramuscularly. The invention provides a nanoparticle comprising: (a) a cargo; (b) a lipid component, wherein the lipid component is one or more ionizable lipids and/or one or more cationic lipids; (c) a phospholipid; (d) a steroid lipid and (e) a cationic polymer. Preferably, the nanoparticle does not comprise a targeting moiety. Preferably, the nanoparticle does not contain a targeting peptide. The nanoparticle may further comprise a PEG lipid.

The cargo comprises a nucleic acid cargo, such as DNA or RNA. Preferably, the cargo comprises a linear DNA product that has enhanced resistance to nuclease digestion (e.g. exonuclease digestion) or mRNA.

The nanoparticle may be a non-viral transfection complex.

The present inventors have developed a nanoparticle (e.g. a non-viral transfection complex) suitable for use in therapy (such as part of a vaccine). The nanoparticle of the present invention has several advantages over viral delivery systems. Firstly, the nanoparticle of the present invention is less likely to elicit an immune response, which is particularly important as repeated doses may need to be administered. In addition, the nanoparticle of the present invention can be used to deliver much larger cargos (i.e. nucleic acids), with a reduced lipid content, due to the presence of a cationic polymer.

The nanoparticles of the present invention provide further benefits in that they are cost-effective and simple to produce on a large scale.

The nanoparticles of the present invention comprise a nucleic acid cargo (e.g. a linear DNA molecule or a closed linear DNA molecule) that has an enhanced resistance to nuclease (e.g. exonuclease) digestion, which results in a prolonged in vivo life of the cargo molecule, together with the cationic polymer which helps to stably pack larger

cargoes into the nanoparticle, without disrupting the size, uniformity and efficacy of the nanoparticles (e.g. non-viral transfection complex).

Importantly, the nanoparticles of the present invention enable the efficient transfection of linear DNA molecule into cells, and thus have use in therapy as vaccines.

**Nanoparticles and non-viral transfection complexes.**

The invention provides a nanoparticle comprising: (a) a cargo; (b) a lipid component, wherein the lipid component is one or more ionizable lipids and/or one or more cationic lipids; (c) a phospholipid; (d) steroid lipid; and (e) a cationic polymer. The nanoparticle may not comprise a targeting peptide or a targeting moiety. The cargo comprises preferably a nucleic acid cargo, such as a linear DNA, or an RNA.

The lipid component may comprise an ionizable and/or a cationic lipid. Preferably, the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5 (ionizable lipids) , DOTMA, DTDTMA or DHDTMA (cationic lipids). The lipid component may be ALC-0315 and DOTMA. The lipid component may be DLin-MC3-DMA and DOTMA. The lipid component may be ALC-0315 and DLin-MC3-DMA. The nanoparticle of the invention may comprise a combination of one or ionizable lipids. The nanoparticle of the invention may comprise one or more cationic lipids. The nanoparticle may comprise one or more ionizable lipids and one or more cationic lipids.

The phospholipid may comprise DOPE, DOPC, DSPC, DPPC, DMPC or POPC. Preferably the phospholipid is DOPE. The phospholipid may be one or more of DOPE, DOPC, DSPC, DPPC, DMPC or POPC.

The nanoparticle comprises a steroid lipid. A steroid lipid may comprise cholesterol, or a derivative thereof (a cholesterol derivative), such as beta-sitosterol, fucosterol, campesterol ,stigamstanol (alkyl steroids), secosteroids (vitamin D2, D3) or pentacyclic steroids. The steroid lipid may be one or more of cholesterol, beta-sitosterol, fucosterol, campesterol ,stigamstanol (alkyl steroids), secosteroids (vitamin D2, D3) or pentacyclic steroids.

The nanoparticle of the invention comprises a cationic polymer. The cationic polymer may comprise oligolysine (linear or branched) such as K16, K17 or K30, oligohistidine (linear or branched) or oligoarginine (linear or branched) or combination of oligolysine and oligohistidine, oligohistidine and oligoarginine, oligoarginine and oligolysine, or oligolysine, oligohistidine and oligoarginine, or PEI. Preferably, the cationic polymer is oligolysine comprising 16, 17 or 30 lysine residues (K16, K17 or K30). The cationic polymer may consist of K16, K17 or K30.

The present invention provides the following embodiments:

1. The nanoparticle of the invention may comprise:

   (a) a cargo, wherein the cargo comprises a closed linear deoxyribonucleic acid (DNA) molecule;
   (b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
   (c) a phospholipid;
   (d) a steroid lipid; and
   (e) a cationic polymer.

2. The nanoparticle of the invention may comprise:

   (a) a cargo, wherein the cargo comprises a linear deoxyribonucleic acid (DNA) molecule comprising one or more nuclease-resistant nucleotides;
   (b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
   (c) a phospholipid;
   (d) a steroid lipid; and
   (e) a cationic polymer;

3. The nanoparticle of the invention may comprise:

   (a) a cargo, wherein the cargo comprises a partially closed linear deoxyribonucleic acid (DNA) molecule comprising a double-stranded DNA portion that is closed at a first end and open at a second end, wherein the

partially closed linear DNA molecule comprises one or more nuclease-resistant nucleotides in an open end region adjacent to the second end;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid;
(d) a steroid lipid; and
(e) a cationic polymer;

4. The nanoparticle of the invention may comprise:

(a) a cargo, wherein the cargo comprises mRNA;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid;
(d) a steroid lipid; and
(e) a cationic polymer;

5. The nanoparticle of the invention may comprise:

(a) a cargo, wherein the cargo comprises siRNA;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid;
(d) a steroid lipid; and
(e) a cationic polymer;

6. The nanoparticle of the invention may comprise:

(a) a cargo, wherein the cargo comprises samRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

7. The nanoparticle of the invention may comprise:

(a) a cargo, wherein the cargo comprises samRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

8. The nanoparticle of the invention may comprise:

(a) a cargo, wherein the cargo comprises samRNA
(b) a lipid component, wherein the lipid component is one or more ionizable lipids and/or one or more cationic

lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

9. The nanoparticle of the invention may comprise:

(a) a cargo, wherein the cargo comprises miRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

10. The nanoparticle of the invention may comprise:

(a) a cargo, wherein the cargo comprises snRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

11. The nanoparticle of the invention may comprise:

(a) a cargo, wherein the cargo comprises tRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

12. The nanoparticle of the invention may comprise:

(a) a cargo, wherein the cargo comprises ribosomal RNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

13. The nanoparticle of the invention may comprise:

(a) a cargo, wherein the cargo comprises antisense RNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;

(c) a phospholipid;
(d) a steroid lipid; and
(e) a cationic polymer;

14. The nanoparticle of the invention may comprise:

(a) a cargo, wherein the cargo comprises snRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

15. The nanoparticle of the invention may comprise:

(a) a cargo, wherein the cargo comprises shRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer;

16. The nanoparticle of the invention may comprise:

(a) a cargo, wherein the cargo comprises gRNA
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer.

In any of the embodiments 1 to 16 above, the nanoparticle may be a non-viral transfection complex. In any of embodiments 1 to 16, the phospholipid may comprise DOPE, DOPC, DSPC, DPPC, DMPC or POPC. In any of embodiments 1 to 16, the steroid lipid may comprise cholesterol, beta sitosterol, fucosterol, campesterol ,stigamstanol (alkyl steroids), secosteroids (vitamin D2, D3) or pentacyclic steroids. In any of embodiments 1 to 16, the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!

In any of embodiments 1 to 16, the nanoparticle may not comprise a targeting moiety. In any of embodiments 1 to 16 the nanoparticles may not comprise a targeting peptide.

In any of the embodiments 1 to 16, the phospholipid is preferably DOPE. Preferably, in embodiments 1 to 16, the steroid lipid comprises cholesterol. Preferably, the nanoparticles 1 to 16 described above comprise oligolysine, such as K16 or K30.

The nanoparticle may further comprise a PEG lipid. The PEG lipid may be a PEGylated phospholipid. The PEG lipid may be DMG-PEG

The invention further provides the following embodiments:

17. The nanoparticle of the invention may comprise:

(a) a cargo, wherein the cargo comprises a closed linear deoxyribonucleic acid (DNA) molecule;
(b) a lipid component, wherein the lipid component is one or more ionizable lipids and/or one or more cationic lipids;

(c) a phospholipid, wherein the phospholipid comprises DOPE, DOPC, DSPC, DPPC, DMPC or POPC;
(d) a steroid lipid; and
(e) a cationic polymer.

18. The nanoparticle of the invention may comprise:

(a) a cargo, wherein the cargo comprises a linear deoxyribonucleic acid (DNA) molecule comprising one or more nuclease-resistant nucleotides;
(b) a lipid component, wherein the lipid component is one or more ionizable lipids and/or one or more cationic lipids
(c) a phospholipid, wherein the phospholipid comprises DOPE, DOPC, DSPC, DPPC, DMPC or POPC;
(d) a steroid lipid; and
(e) a cationic polymer;

19.The nanoparticle may comprise:

(a) a cargo, wherein the cargo comprises a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer;

20. The nanoparticle of the invention may comprise:

(a) a cargo, wherein the cargo comprises mRNA;
(b) a lipid component, wherein the lipid component is one or more ionizable lipids and/or one or more cationic lipids
(c) a phospholipid, wherein the phospholipid comprises DOPE, DOPC, DSPC, DPPC, DMPC or POPC;
(d) a steroid lipid; and
(e) a cationic polymer;

21. The nanoparticle of the invention may comprise:

(a) a cargo, wherein the cargo comprises siRNA;
(b) a lipid component, wherein the lipid component is one or more ionizable lipids and/or one or more cationic lipids
(c) a phospholipid, wherein the phospholipid comprises DOPE, DOPC, DSPC, DPPC, DMPC or POPC;
(d) a steroid lipid; and
(e) a cationic polymer;

24. The nanoparticle of the invention may comprise:

(a) a cargo, wherein the cargo comprises samRNA;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer;

25. The nanoparticle of the invention may comprise:

(a) a cargo, wherein the cargo comprises miRNA;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer;

26. The nanoparticle of the invention may comprise:

(a) a cargo, wherein the cargo comprises snRNA;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer;

27. The nanoparticle of the invention may comprise:

(a) a cargo, wherein the cargo comprises tRNA;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer;

28. The nanoparticle of the invention may comprise:

(a) a cargo, wherein the cargo comprises ribosomal RNA;
(b) a lipid component, wherein the lipid component is one or more ionizable lipids and/or one or more cationic lipids,
(c) a phospholipid, wherein the phospholipid comprises DOPE, DOPC, DSPC, DPPC, DMPC or POPC;
(d) a steroid lipid; and
(e) a cationic polymer;

29. The nanoparticle of the invention may comprise:

(a) a cargo, wherein the cargo comprises antisense RNA;
(b) a lipid component, wherein the lipid component is one or more ionizable lipids and/or one or more cationic lipids,
(c) a phospholipid, wherein the phospholipid comprises DOPE, DOPC, DSPC, DPPC, DMPC or POPC;
(d) a steroid lipid; and
(e) a cationic polymer;

30. The nanoparticle of the invention may comprise:

(a) a cargo, wherein the cargo comprises snRNA;
(b) a lipid component, wherein the lipid component is one or more ionizable lipids and/or one or more cationic lipids,
(c) a phospholipid, wherein the phospholipid comprises DOPE, DOPC, DSPC, DPPC, DMPC or POPC;
(d) a steroid lipid; and
(e) a cationic polymer;

31. The nanoparticle of the invention may comprise:

(a) a cargo, wherein the cargo comprises shRNA;
(b) a lipid component, wherein the lipid component is one or more ionizable lipids and/or one or more cationic lipids,
(c) a phospholipid, wherein the phospholipid comprises DOPE, DOPC, DSPC, DPPC,DMPC, POPC or SM;
(d) a steroid lipid; and
(e) a cationic polymer;

32. The nanoparticle of the invention may comprise:

(a) a cargo, wherein the cargo comprises gRNA;
(b) a lipid component, wherein the lipid component is one or more ionizable lipids and/or one or more cationic

lipids,
(c) a phospholipid, wherein the phospholipid comprises DOPE, DOPC, DSPC, DPPC, DMPC or POPC;
(d) a steroid lipid; and
(e) a cationic polymer.

In any of embodiments 17 to 32, the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA. In any of embodiments 2 to 32, the steroid lipid may comprise cholesterol, beta-sitosterol, fucosterol, campesterol ,stigamstanol (alkyl steroids), secosteroids (vitamin D2, D3) or pentacyclic steroids.. In any of embodiments 1 to 16, the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

In any of embodiments 7 to 32, the nanoparticle may not comprise a targeting moiety. In any of embodiments 2 to 32 the nanoparticles may not comprise a targeting peptide.

In any of the embodiments 7 to 32 , the lipid component preferably comprises at least one ionizable lipid.

Preferably, in the embodiments 7 to 32, the nanoparticles described above comprise cholesterol. Preferably, in embodiments 7 to 32, the nanoparticles comprise an oligolysine, such as K16 or K30.

The nanoparticle may further comprise a PEG lipid. The PEG lipid may be a PEGylated phospholipid. The PEG lipid may be DMG-PEG.

The invention further provides the following embodiments:

33. The nanoparticle of the invention may comprise:

(a) a cargo, wherein the cargo comprises a closed linear deoxyribonucleic acid (DNA) molecule;
(b) a lipid component, wherein the lipid component is one or more ionizable lipids and/or one or more cationic lipids
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids(alkyl steroids), secosteroids (vitamin D2, D3) or pentacyclic steroids; and
(e) a cationic polymer.

34. The nanoparticle of the invention may comprise:

(a) a cargo, wherein the cargo comprises a linear deoxyribonucleic acid (DNA) molecule comprising one or more nuclease-resistant nucleotides;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

35. The nanoparticle of the invention may comprise:

(a) a cargo, wherein the cargo comprises a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end;
(b) a lipid component, wherein the lipid component is one or more ionizable lipids and/or one or more cationic lipids;
(c) a phospholipid;
(d) a steroid lipid, wherein the steroid lipid comprises one or more of beta-sitosterol, fucosterol, campesterol, stigamstanol (alkyl steroids), secosteroids (vitamin D2, D3) or pentacyclic steroids; and
(e) a cationic polymer;

36. The nanoparticle of the invention may comprise:

(a) a cargo, wherein the cargo comprises mRNA;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

37. The nanoparticle of the invention may comprise:

(a) a cargo, wherein the cargo comprises siRNA;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

38. The nanoparticle of the invention may comprise:

(a) a cargo, wherein the cargo comprises samRNA
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

39. The nanoparticle of the invention may comprise:

(a) a cargo, wherein the cargo comprises miRNA
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

40. The nanoparticle of the invention may comprise:

(a) a cargo, wherein the cargo comprises snRNA
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

41. The nanoparticle of the invention may comprise:

(a) a cargo, wherein the cargo comprises tRNA
(b) a lipid component, wherein the lipid component is one or more ionizable lipids and/or one or more cationic lipids;
(c) a phospholipid;
(d) a steroid lipid, wherein the steroid lipid comprises one or more of beta-sitosterol, fucosterol, campesterol, stigamstanol (alkyl steroids), secosteroids (vitamin D2, D3) or pentacyclic steroids; and
(e) a cationic polymer;

42. The nanoparticle of the invention may comprise:

(a) a cargo, wherein the cargo comprises ribosomal RNA
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

43. The nanoparticle of the invention may comprise:

(a) a cargo, wherein the cargo comprises antisense RNA
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

44. The nanoparticle of the invention may comprise:

(a) a cargo, wherein the cargo comprises snRNA
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

45. The nanoparticle of the invention may comprise:

(a) a cargo, wherein the cargo comprises shRNA
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

46. The nanoparticle of the invention may comprise:

(a) a cargo, wherein the cargo comprises gRNA
(b) a lipid component, wherein the lipid component is one or more ionizable lipids and/or one or more cationic lipids
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer;

In any of the embodiments 33 to 46 above, the nanoparticle may be a non-viral transfection complex.

In any of embodiments 33 to 46, the phospholipid may comprise DOPE, DOPC, DSPC, DPPC, DMPC or POPC. In any of embodiments 33 to 46, the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA. In any of embodiments 33 to 46, the cationic polymer may comprise an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

In any of embodiments 33 to 46, the nanoparticle may not comprise a targeting moiety. In any of embodiments

33 to 46 the nanoparticles may not comprise a targeting peptide.

In embodiments 33 to 46, the lipid preferably comprises at least one ionizable lipid. In embodiments 33 to 46, the phospholipid is preferably DOPE. In embodiments 33 to 46, the cationic polymer preferably comprises oligolysine, such as K16 or K30.

The nanoparticle may further comprise a PEG lipid. The PEG lipid may be a PEGylated phospholipid. The PEG lipid may be DMG-PEG.

The invention further provides the following embodiments:

47. The nanoparticle of the invention may comprise:

(a) a cargo, wherein the cargo comprises a closed linear deoxyribonucleic acid (DNA) molecule;
(b) a lipid component, wherein the lipid component is one or more ionizable lipids and/or one or more cationic lipids
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

48. The nanoparticle may comprise:

(a) a cargo, wherein the cargo comprises a linear deoxyribonucleic acid (DNA) molecule comprising one or more nuclease-resistant nucleotides;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

49. The nanoparticle of the invention may comprise:

(a) a cargo, wherein the cargo comprises a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

50. The nanoparticle of the invention may comprise:

(a) a cargo, wherein the cargo comprises mRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

51. The nanoparticle of the invention may comprise:

(a) a cargo, wherein the cargo comprises siRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

52. The nanoparticle of the invention may comprise:

(a) a cargo, wherein the cargo comprises samRNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

53. The nanoparticle of the invention may comprise:

(a) a cargo, wherein the cargo comprises miRNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

54. The nanoparticle of the invention may comprise:

(a) a cargo, wherein the cargo comprises snRNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

55. The nanoparticle of the invention may comprise:

(a) a cargo, wherein the cargo comprises tRNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or

oligolysine, oligohistidine and oligoarginine, or PEI;

56. The nanoparticle of the invention may comprise:

(a) a cargo, wherein the cargo comprises ribosomal RNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

57. The nanoparticle of the invention may comprise:

(a) a cargo, wherein the cargo comprises antisense RNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

58. The nanoparticle of the invention may comprise:

(a) a cargo, wherein the cargo comprises snRNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

59. The nanoparticle of the invention may comprise:

(a) a cargo, wherein the cargo comprises shRNA
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI;

60. The nanoparticle of the invention may comprise:

(a) a cargo, wherein the cargo comprises gRNA
(b) a lipid component, wherein the lipid component is one or more ionizable lipids and/or one or more cationic lipids
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an

oligolysine and an oligohistidine, an oligohistidine and an oligoarginine, an oligoarginine and an oligolysine, or oligolysine, oligohistidine and oligoarginine, or PEI.

In any of the embodiments 47 to 60 above, the nanoparticle may be a non-viral transfection complex.

In any of embodiments 47 to 60, the phospholipid may comprise DOPE, DOPC, DSPC, DPPC, DMPC or POPC. In any of embodiments 47 to 60, the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA. In any of embodiments 47 to 60, the steroid lipid may comprise cholesterol, beta-sitosterol, fucosterol, campesterol, stigamstanol (alkyl steroids), secosteroids (vitamin D2, D3) or pentacyclic steroids.

In any of embodiments 47 to 60, the nanoparticle may not comprise a targeting moiety. In any of embodiments 47 to 60 the nanoparticles may not comprise a targeting peptide.

In embodiments 47 to 60, the lipid preferably comprises at least one ionizable lipid. In embodiments 47 to 60, the phospholipid is preferably DOPE. In embodiments 47 to 60, the cationic polymer preferably comprises oligolysine, such as K16 or K30.

Preferably, the cationic polymer is a nucleic acid-binding cationic polymer (e.g. DNA-binding cationic polymer, closed linear DNA-binding cationic polymer, a partially closed linear DNA-binding cationic polymer or linear DNA-binding cationic polymer, or an RNA, such as an mRNA cationic binding polymer).

The cationic polymer may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the nucleic acid-binding cationic polymer comprises at least 16, at least 17 or at least 30 cationic monomers.

The cationic polymer may comprise a lysine, a histidine, or an arginine. The cationic polymer may comprise a lysine, a histidine, or an arginine. The cationic polymer may comprise an oligolysine (linear or branched), an oligohistidine (linear or branched) or an oligoarginine (linear or branched). For example, the cationic polymer may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the cationic polymer comprises at least 16, at least 17, or at least 30 lysine residues. More preferably still, the nucleic acid-binding polycationic component comprises at least 16 lysine residues. The polycationic polymer may consist of 16 lysine residues or 30 lysine residues.

An alternative cationic polymer is polyethylenimine (PE!), linear or branched, of MW from 500 to 10,000.

The nanoparticle may further comprise a PEG lipid. The PEG lipid may be a PEGylated phospholipid. The PEG lipid may be DMG-PEG

The invention further provides the following embodiments:

61. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DLin-MC3-DMA
(c) a phospholipid;
(d) a steroid lipid; and
(e) a cationic polymer.

62. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more

nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DLin-KC2-DMA;
(c) a phospholipid;
(d) a steroid lipid; and
(e) a cationic polymer.

63. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DLin-DMA;
(c) a phospholipid;
(d) a steroid lipid; and
(e) a cationic polymer.

64. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises TCL053;
(c) a phospholipid;
(d) a steroid lipid; and
(e) a cationic polymer.

65. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises SM-102;
(c) a phospholipid;
(d) a steroid lipid; and
(e) a cationic polymer.

66. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;

(b) a lipid component, wherein the lipid component is one or more ionizable lipids and/or one or more cationic lipids, wherein the lipid component comprises ALC-0315;
(c) a phospholipid;
(d) a steroid lipid; and
(e) a cationic polymer.

67. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises C12-200;
(c) a phospholipid;
(d) a steroid lipid; and
(e) a cationic polymer.

68. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DODMA;
(c) a phospholipid;
(d) a steroid lipid; and
(e) a cationic polymer.

69. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DODAP;
(c) a phospholipid;
(d) a steroid lipid; and
(e) a cationic polymer.

70. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids wherein the lipid component comprises Lipid A9;

(c) a phospholipid;
(d) a steroid lipid; and
(e) a cationic polymer.

71. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids wherein the lipid component comprises 9A1 P9;
(c) a phospholipid;
(d) a steroid lipid; and
(e) a cationic polymer.

72. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises , Lipid C24;
(c) a phospholipid;
(d) a steroid lipid; and
(e) a cationic polymer.

73. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises Lipid LP01;
(c) a phospholipid;
(d) a steroid lipid; and
(e) a cationic polymer.

74. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises, Lipid 5;
(c) a phospholipid;
(d) a steroid lipid; and

(e) a cationic polymer.

75. The nanoparticle of the invention may comprise:

**(a)** a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DOTMA;
(c) a phospholipid;
(d) a steroid lipid; and
(e) a cationic polymer.;

76. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DTDTMA;
(c) a phospholipid;
(d) a steroid lipid; and
(e) a cationic polymer.

77. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises DHDTMA;
(c) a phospholipid;
(d) a steroid lipid; and
(e) a cationic polymer.

In any of the embodiments 61 to 77 above, the nanoparticle may be a non-viral transfection complex. In any of embodiments 61 to 77, the phospholipid may comprise DOPE, DOPC, DSPC, DPPC, DMPC or POPC. In any of embodiments 61 to 77, the steroid lipid may comprise cholesterol, beta sitosterol, fucosterol, campesterol, stigamstanol (alkyl steroids), secosteroids (vitamin D2, D3) or pentacyclic steroids. In any of embodiments 61 to 77, the cationic polymer may comprise an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!

In any of embodiments 61 to 77, the nanoparticle may not comprise a targeting moiety. In any of embodiments 61 to 77 the nanoparticles may not comprise a targeting peptide.

In embodiments 61 to 77, the cargo preferably comprises a linear DNA (e.g. a closed linear DNA, a linear DNA that comprises one or more nuclease resistant nucleotides at a first end and is closed at a second end, or a linear DNA that comprises one or more nuclease resistant nucleotides at a first end and a second end), or an mRNA. In embodiments 61 to 77, the phospholipid is preferably DOPE. In embodiments 61 to 77, the steroid lipid comprises prefab cholesterol. In embodiments 61 to 77, the cationic polymer is preferably oligolysine, such as K16 or K30.

The nanoparticle may further comprise a PEG lipid. The PEG lipid may be a PEGylated phospholipid. The PEG lipid may be DMG-PEG.

78. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA
(c) a phospholipid, wherein the phospholipid comprises DOPE, DOPC, DSPC, DPPC, DMPC or POPC;
(d) a steroid lipid; and
(e) a cationic polymer.

79. The nanoparticle of the invention may comprise:

(a) a cargo;
(b) a lipid component, wherein the lipid component is one or more ionizable lipids and/or one or more cationic lipids, wherein the lipid component comprises DLin-KC2-DMA;
(c) a phospholipid, wherein the phospholipid comprises DOPE, DOPC, DSPC, DPPC, DMPC or POPC;
(d) a steroid lipid; and
(e) a cationic polymer.

80. The nanoparticle of the invention may comprise:

a) a cargo;
b) a lipid component, wherein the lipid component is one or more ionizable lipids and/or one or more cationic lipids, wherein the lipid component comprises DLin-DMA;
c) a phospholipid, wherein the phospholipid comprises DOPE, DOPC, DSPC, DPPC, DMPC or POPC;
d) a steroid lipid; and
e) a cationic polymer.

81. The nanoparticle of the invention may comprise:

a) a cargo;
b) a lipid component, wherein the lipid component is one or more ionizable lipids and/or one or more cationic lipids, wherein the lipid component comprises TCL053;
c) a phospholipid, wherein the phospholipid comprises DOPE, DOPC, DSPC, DPPC, DMPC or POPC;
d) a steroid lipid; and
e) a cationic polymer.

82. The nanoparticle of the invention may comprise:

a) a cargo;
b) a lipid component, wherein the lipid component is one or more ionizable lipids and/or one or more cationic lipids, wherein the lipid component comprises SM-102;
c) a phospholipid, wherein the phospholipid comprises DOPE, DOPC, DSPC, DPPC, DMPC or POPC;
d) a steroid lipid; and
e) a cationic polymer.

83. The nanoparticle of the invention may comprise:

a) a cargo;
b) a lipid component, wherein the lipid component is one or more ionizable lipids and/or one or more cationic lipids, wherein the lipid component comprises ALC-0315;
c) a phospholipid, wherein the phospholipid comprises DOPE, DOPC, DSPC, DPPC, DMPC or POPC;
d) a steroid lipid; and
e) a cationic polymer.

84. The nanoparticle of the invention may comprise:

a) a cargo;
b) a lipid component, wherein the lipid component is one or more ionizable lipids and/or one or more cationic lipids, wherein the lipid component comprises C12-200;
c) a phospholipid, wherein the phospholipid comprises DOPE, DOPC, DSPC, DPPC, DMPC or POPC;
d) a steroid lipid; and
e) a cationic polymer.

85. The nanoparticle of the invention may comprise:

a) a cargo;
b) a lipid component, wherein the lipid component is one or more ionizable lipids and/or one or more cationic lipids, wherein the lipid component comprises DODMA;
c) a phospholipid, wherein the phospholipid comprises DOPE, DOPC, DSPC, DPPC, DMPC or POPC;
d) a steroid lipid; and
e) a cationic polymer.

86. The nanoparticle of the invention may comprise:

a) a cargo;
b) a lipid component, wherein the lipid component is one or more ionizable lipids and/or one or more cationic lipids, wherein the lipid component comprises DODAP;
c) a phospholipid, wherein the phospholipid comprises DOPE, DOPC, DSPC, DPPC, DMPC or POPC;
d) a steroid lipid; and
e) a cationic polymer.

87. The nanoparticle of the invention may comprise:

a) a cargo;
b) a lipid component, wherein the lipid component is one or more ionizable lipids and/or one or more cationic lipids wherein the lipid component comprises Lipid A9;
c) a phospholipid, wherein the phospholipid comprises DOPE, DOPC, DSPC, DPPC, DMPC or POPC;
d) a steroid lipid; and
e) a cationic polymer.

88. The nanoparticle of the invention may comprise:

a) a cargo;
b) a lipid component, wherein the lipid component is one or more ionizable lipids and/or one or more cationic lipids wherein the lpid component comprises 9A1 P9;
c) a phospholipid, wherein the phospholipid comprises DOPE, DOPC, DSPC, DPPC, DMPC or POPC;
d) a steroid lipid; and
e) a cationic polymer.

89. The nanoparticle of the invention may comprise:

a) a cargo;
b) a lipid component, wherein the lipid component is one or more ionizable lipids and/or one or more cationic lipids, wherein the lipid component comprises , Lipid C24;
c) a phospholipid, wherein the phospholipid comprises DOPE, DOPC, DSPC, DPPC, DMPC or POPC;
d) a steroid lipid; and
e) a cationic polymer.

90. The nanoparticle of the invention may comprise:

a) a cargo;
b) a lipid component, wherein the lipid component is one or more ionizable lipids and/or one or more cationic lipids, wherein the lipid component comprises Lipid LP01;
c) a phospholipid, wherein the phospholipid comprises DOPE, DOPC, DSPC, DPPC, DMPC or POPC;

d) a steroid lipid; and
e) a cationic polymer.

91. The nanoparticle of the invention may comprise:

a) a cargo;
b) a lipid component, wherein the lipid component is one or more ionizable lipids and/or one or more cationic lipids, wherein the lipid component comprises, Lipid 5;
c) a phospholipid, wherein the phospholipid comprises DOPE, DOPC, DSPC, DPPC, DMPC or POPC;
d) a steroid lipid; and
e) a cationic polymer.

92. The nanoparticle of the invention may comprise:

**a)** a cargo;
b) a lipid component, wherein the lipid component is one or more ionizable lipids and/or one or more cationic lipids, wherein the lipid component comprises DOTMA;
c) a phospholipid, wherein the phospholipid comprises DOPE, DOPC, DSPC, DPPC, DMPC or POPC;
d) a steroid lipid; and
e) a cationic polymer.

93. The nanoparticle of the invention may comprise:

a) a cargo;
b) a lipid component, wherein the lipid component is one or more ionizable lipids and/or one or more cationic lipids, wherein the lipid component comprises DTDTMA;
c) a phospholipid, wherein the phospholipid comprises DOPE, DOPC, DSPC, DPPC, DMPC or POPC;
d) a steroid lipid; and
e) a cationic polymer.

94. The nanoparticle of the invention may comprise:

a) a cargo;
b) a lipid component, wherein the lipid component is one or more ionizable lipids and/or one or more cationic lipids, wherein the lipid component comprises DHDTMA;
c) a phospholipid, wherein the phospholipid is one or more of DOPE, DOPC, DSPC, DPPC, DMPC or POPC;
d) a steroid lipid; and
e) a cationic polymer.

In any of the embodiments 78 to 94, the nanoparticle may be a non-viral transfection complex. In any of embodiments 78 to 94, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 1 to 16, the steroid lipid may comprise cholesterol, beta sitosterol, fucosterol, campesterol, stigamstanol (alkyl steroids), secosteroids (vitamin D2, D3) or pentacyclic steroids. In any of embodiments 78 to 94, the cationic polymer may comprise an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

In any of embodiments 78 to 94, the nanoparticle may not comprise a targeting moiety. In any of embodiments 78 to 94 the nanoparticle may not comprise a targeting peptide.

In any of embodiments 78 to 94, the cargo comprises preferably a linear DNA or mRNA. In any of embodiments 78 to 94, the steroid lipid comprises preferably cholesterol. In any of embodiments 78 to 94, the cationic polymer is preferably oligolysine.

The nanoparticle may further comprise a PEG lipid. The PEG lipid may be a PEGylated phospholipid. The PEG lipid may be DMG-PEG.

The invention further provides the following embodiments:

95. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

96. The nanoparticle of the invention may comprise:

(a) a cargo;
(b) a lipid component, wherein the lipid component is one or more ionizable lipids and/or one or more cationic lipids, wherein the lipid component comprises DLin-KC2-DMA;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

97. The nanoparticle of the invention may comprise:

(a) a cargo;
(b) a lipid component, wherein the lipid component is one or more ionizable lipids and/or one or more cationic lipids, wherein the lipid component comprises DLin-DMA;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

98. The nanoparticle of the invention may comprise:

(a) a cargo;
(b) a lipid component, wherein the lipid component is one or more ionizable lipids and/or one or more cationic lipids, wherein the lipid component comprises TCL053;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

99. The nanoparticle of the invention may comprise:

(a) a cargo;
(b) a lipid component, wherein the lipid component is one or more ionizable lipids and/or one or more cationic lipids, wherein the lipid component comprises SM-102;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

100. The nanoparticle of the invention may comprise:

(a) a cargo;
(b) a lipid component, wherein the lipid component is one or more ionizable lipids and/or one or more cationic lipids, wherein the lipid component comprises ALC-0315;
(c) a phospholipid;

(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

101. The nanoparticle of the invention may comprise:

(a) a cargo;
(b) a lipid component, wherein the lipid component is one or more ionizable lipids and/or one or more cationic lipids, wherein the lipid component comprises C12-200;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

102. The nanoparticle of the invention may comprise:

(a) a cargo;
(b) a lipid component, wherein the lipid component is one or more ionizable lipids and/or one or more cationic lipids, wherein the lipid component comprises DODMA;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

103. The nanoparticle of the invention may comprise:

(a) a cargo;
(b) a lipid component, wherein the lipid component is one or more ionizable lipids and/or one or more cationic lipids, wherein the lipid component comprises DODAP;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

104. The nanoparticle of the invention may comprise:

(a) a cargo;
(b) a lipid component, wherein the lipid component is one or more ionizable lipids and/or one or more cationic lipids wherein the lipid component comprises Lipid A9;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

105. The nanoparticle of the invention may comprise:

(a) a cargo;
(b) a lipid component, wherein the lipid component is one or more ionizable lipids and/or one or more cationic lipids wherein the lipid component comprises 9A1 P9;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

106. The nanoparticle of the invention may comprise:

(a) a cargo;
(b) a lipid component, wherein the lipid component is one or more ionizable lipids and/or one or more cationic

lipids, wherein the lipid component comprises , Lipid C24;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

107. The nanoparticle of the invention may comprise:

(a) a cargo;
(b) a lipid component, wherein the lipid component is one or more ionizable lipids and/or one or more cationic lipids, wherein the lipid component comprises Lipid LP01;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

108. The nanoparticle of the invention may comprise:

(a) a cargo;
(b) a lipid component, wherein the lipid component is one or more ionizable lipids and/or one or more cationic lipids, wherein the lipid component comprises, Lipid 5;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

109. The nanoparticle of the invention may comprise:

**(a)** a cargo;
(b) a lipid component, wherein the lipid component is one or more ionizable lipids and/or one or more cationic lipids, wherein the lipid component comprises DOTMA;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

110. The nanoparticle of the invention may comprise:

(a) a cargo;
(b) a lipid component, wherein the lipid component is one or more ionizable lipids and/or one or more cationic lipids, wherein the lipid component comprises DTDTMA;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

111. The nanoparticle of the invention may comprise:

(a) a cargo;
(b) a lipid component, wherein the lipid component is one or more ionizable lipids and/or one or more cationic lipids, wherein the lipid component comprises DHDTMA;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

In any of the embodiments 95 to 111 above, the nanoparticle may be a non-viral transfection complex.
In any of embodiments 95 to 111, the phospholipid may comprise DOPE, DOPC, DSPC, DPPC, DMPC or

POPC. In any of embodiments 95 to 111, cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 95 to 111, the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!

In any of embodiments 95 to 111, the nanoparticle may not comprise a targeting moiety. In any of embodiments 95 to 111, the nanoparticles may not comprise a targeting peptide.

In any of embodiments 95 to 111, the cargo comprises preferably a linear DNA or mRNA. In any of embodiments 95 to 111, the phospholipid is preferably DOPE. In any of embodiments 95 to 111, the cationic polymer is oligolysine, such as K16 or K30.

The nanoparticle may further comprise a PEG lipid. The PEG lipid may be a PEGylated phospholipid. The PEG lipid may be DMG-PEG

The invention further provides the following embodiments:

112. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

113. The nanoparticle of the invention may comprise:

(a) a cargo;
(b) a lipid component, wherein the lipid component is one or more ionizable lipids and/or one or more cationic lipids, wherein the lipid component comprises DLin-KC2-DMA;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

114. The nanoparticle of the invention may comprise:

(a) a cargo;
(b) a lipid component, wherein the lipid component is one or more ionizable lipids and/or one or more cationic lipids, wherein the lipid component comprises DLin-DMA;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

115. The nanoparticle of the invention may comprise:

(a) a cargo;
(b) a lipid component, wherein the lipid component is one or more ionizable lipids and/or one or more cationic

lipids, wherein the lipid component comprises TCL053;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

116. The nanoparticle of the invention may comprise:

(a) a cargo;
(b) a lipid component, wherein the lipid component is one or more ionizable lipids and/or one or more cationic lipids, wherein the lipid component comprises SM-102;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

117. The nanoparticle of the invention may comprise:

(a) a cargo;
(b) a lipid component, wherein the lipid component is one or more ionizable lipids and/or one or more cationic lipids, wherein the lipid component comprises ALC-0315;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!.

118. The nanoparticle may comprise:

(a) a cargo;
(b) a lipid component, wherein the lipid component is one or more ionizable lipids and/or one or more cationic lipids, wherein the lipid component comprises C12-200;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

119. The nanoparticle of the invention may comprise:

(a) a cargo;
(b) a lipid component, wherein the lipid component is one or more ionizable lipids and/or one or more cationic lipids, wherein the lipid component comprises DODMA;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

120. The nanoparticle of the invention may comprise:

(a) a cargo;
(b) a lipid component, wherein the lipid component is one or more ionizable lipids and/or one or more cationic lipids, wherein the lipid component comprises DODAP;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

121. The nanoparticle of the invention may comprise:

(a) a cargo;
(b) a lipid component, wherein the lipid component is one or more ionizable lipids and/or one or more cationic lipids wherein the lipid component comprises Lipid A9;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

122. The nanoparticle of the invention may comprise:

(a) a cargo;
(b) a lipid component, wherein the lipid component is one or more ionizable lipids and/or one or more cationic lipids wherein the lipid component comprises 9A1 P9;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

123. The nanoparticle of the invention may comprise:

(a) a cargo;
(b) a lipid component, wherein the lipid component is one or more ionizable lipids and/or one or more cationic lipids, wherein the lipid component comprises Lipid C24;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

124. The nanoparticle of the invention may comprise:

(a) a cargo;
(b) a lipid component, wherein the lipid component is one or more ionizable lipids and/or one or more cationic lipids, wherein the lipid component comprises Lipid LP01;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

125. The nanoparticle of the invention may comprise:

(a) a cargo;
(b) a lipid component, wherein the lipid component is one or more ionizable lipids and/or one or more cationic lipids, wherein the lipid component comprises, Lipid 5;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

126. The nanoparticle of the invention may comprise:

**(a)** a cargo;
(b) a lipid component, wherein the lipid component is one or more ionizable lipids and/or one or more cationic lipids, wherein the lipid component comprises DOTMA;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

127. The nanoparticle of the invention may comprise:

(a) a cargo;
(b) a lipid component, wherein the lipid component is one or more ionizable lipids and/or one or more cationic lipids, wherein the lipid component comprises DTDTMA;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

128. The nanoparticle of the invention may comprise:

(a) a cargo;
(b) a lipid component, wherein the lipid component is one or more ionizable lipids and/or one or more cationic lipids, wherein the lipid component comprises DHDTMA;
(c) a phospholipid;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

In any of the embodiments 112 to 128 above, the nanoparticle may be a non-viral transfection complex.

In any of embodiments 112 to 128, the phospholipid may comprise DOPE, DOPC, DSPC, DPPC, DMPC or POPC. In any of embodiments 112 to 128, the steroid lipid may comprise cholesterol, beta sitosterol, fucosterol, campesterol, stigamstanol (alkyl steroids), secosteroids (vitamin D2, D3) or pentacyclic steroids. In any of embodiments 112 to 128, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA.

In any of embodiments 112 to 128, the nanoparticle may not comprise a targeting moiety. In any of embodiments 112 to 128 the nanoparticles may not comprise a targeting peptide.

In embodiments 112 to 128, the phospholipid is preferably DOPE. In embodiments 112 to 128, the steroid is preferably cholesterol. In embodiments 112 to 128, the cargo comprises preferably a linear DNA or mRNA.

The nanoparticle may further comprise a PEG lipid. The PEG lipid may be a PEGylated phospholipid. The PEG lipid may be DMG-PEG

The invention further provides the following embodiments:

129. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid, wherein the phospholipid is phosphatidylethanolamine;
(d) a steroid lipid; and
(e) a cationic polymer.

130. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid, wherein the phospholipid is 1,2-dioleoyl-sn-glycero-3-phosphoetha-nolamine (DOPE);
(d) a steroid lipid; and
(e) a cationic polymer.

131. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid, wherein the phospholipid is phosphatidyl chline;
(d) a steroid lipid; and
(e) a cationic polymer.

132. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;

(b) a lipid component, wherein the lipid component is one or more ionizable lipids and/or one or more cationic lipids;
(c) a phospholipid, wherein the phospholipid is 1,2-dioleoyl- sn-glycero-3-phosphoethanoltrimethylamine (DOPC);
(d) a steroid lipid; and
(e) a cationic polymer.

133. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid, wherein the phospholipid is DSPC;
(d) a steroid lipid; and
(e) a cationic polymer.

134. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid, wherein the phospholipid is DPPC;
(d) a steroid lipid; and
(e) a cationic polymer.

135. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid, wherein the phospholipid is DMPC;
(d) a steroid lipid; and
(e) a cationic polymer.

136. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic

lipids;
(c) a phospholipid, wherein the phospholipid is POPC;
(d) a steroid lipid; and
(e) a cationic polymer.

137. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid, wherein the phospholipid is SM;
(d) a steroid lipid; and
(e) a cationic polymer.

In any of the embodiments 129 to 137 above, the nanoparticle may be a non-viral transfection complex.
In any of embodiments 129 to 137 the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA. In any of embodiments 129 to 137, the steroid lipid may comprise cholesterol, beta sitosterol, fucosterol, campesterol, stigamstanol (alkyl steroids), secosteroids (vitamin D2, D3) or pentacyclic steroids. In any of embodiments 129 to 137, the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.
In any of embodiments 129 to 137, the nanoparticle may not comprise a targeting moiety. In any of embodiments 129 to 137 the nanoparticles may not comprise a targeting peptide.
In embodiments 129 to 137, the lipid preferably comprises at least one ionizable lipid. In embodiments 129 to 137, the steroid lipid comprises preferably cholesterol. In embodiments 129 to 137, the cationic polymer is preferably oligolysine, such as K16 or K30.
The nanoparticle may further comprise a PEG lipid. The PEG lipid may be a PEGylated phospholipid. The PEG lipid may be DMG-PEG.

138. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid, wherein the phospholipid is phosphatidylethanolamine;
(d) a steroid lipid; and
(e) a cationic polymer.

139. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid, wherein the phospholipid is 1,2-dioleoyl-sn-glycero-3-phosphoetha-nolamine (DOPE);
(d) a steroid lipid; and
(e) a cationic polymer.

140. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid, wherein the phospholipid is phosphatidyl chline;
(d) a steroid lipid; and
(e) a cationic polymer.

141. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid, wherein the phospholipid is 1 ,2-dioleoyl- sn-glycero-3-phosphoethanoltrimethylamine (DOPC);
(d) a steroid lipid; and
(e) a cationic polymer.

142. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid, wherein the phospholipid is DSPC;
(d) a steroid lipid; and
(e) a cationic polymer.

143. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid, wherein the phospholipid is DPPC;
(d) a steroid lipid; and
(e) a cationic polymer.

144. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid, wherein the phospholipid is DMPC;
(d) a steroid lipid; and
(e) a cationic polymer.

145. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid, wherein the phospholipid is POPC;
(d) a steroid lipid; and
(e) a cationic polymer.

146. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid, wherein the phospholipid is SM;
(d) a steroid lipid; and
(e) a cationic polymer.

In any of the embodiments 138 to 146above, the nanoparticle may be a non-viral transfection complex.

In any of embodiments 138 to 146, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 138 to 146, the steroid lipid may comprise cholesterol, beta sitosterol, fucosterol, campesterol, stigamstanol (alkyl steroids), secosteroids (vitamin D2, D3) or pentacyclic steroids. In any of embodiments 138 to 146, the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!

In any of embodiments 138 to 146, the nanoparticle may not comprise a targeting moiety. In any of embodiments 138 to 146the nanoparticles may not comprise a targeting peptide.

In embodiments 138 to 146, the cargo comprises preferably a linear DNA or mRNA. In embodiments 138 to 146, the steroid lipid comprises preferably cholesterol. In embodiments 138 to 146, the cationic polymer is preferably oligolysine, such as K16 or K30.

The nanoparticle may further comprise a PEG lipid. The PEG lipid may be a PEGylated phospholipid. The PEG lipid may be DMG-PEG

147. The nanoparticle of the invention may comprise:

(a) a cargo;
(b) a lipid component, wherein the lipid component is one or more ionizable lipids and/or one or more cationic lipids;
(c) a phospholipid, wherein the phospholipid is phosphatidylethanolamine;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

148. The nanoparticle of the invention may comprise:

(a) a cargo;
(b) a lipid component, wherein the lipid component is one or more ionizable lipids and/or one or more cationic lipids;
(c) a phospholipid, wherein the phospholipid is 1,2-dioleoyl-sn-glycero-3-phosphoetha-nolamine (DOPE);
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and

(e) a cationic polymer.

149. The nanoparticle of the invention may comprise:

(a) a cargo;
(b) a lipid component, wherein the lipid component is one or more ionizable lipids and/or one or more cationic lipids;
(c) a phospholipid, wherein the phospholipid is phosphatidyl choline;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

150. The nanoparticle of the invention may comprise:

(a) a cargo;
(b) a lipid component, wherein the lipid component is one or more ionizable lipids and/or one or more cationic lipids;
(c) a phospholipid, wherein the phospholipid is 1,2-dioleoyl- sn-glycero-3-phosphoethanoltrimethylamine (DOPC);
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

151. The nanoparticle of the invention may comprise:

(a) a cargo;
(b) a lipid component, wherein the lipid component is one or more ionizable lipids and/or one or more cationic lipids;
(c) a phospholipid, wherein the phospholipid is DSPC;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

152. The nanoparticle of the invention may comprise:

(a) a cargo;
(b) a lipid component, wherein the lipid component is one or more ionizable lipids and/or one or more cationic lipids;
(c) a phospholipid, wherein the phospholipid is DPPC;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

153. The nanoparticle of the invention may comprise:

(a) a cargo;
(b) a lipid component, wherein the lipid component is one or more ionizable lipids and/or one or more cationic lipids;
(c) a phospholipid, wherein the phospholipid is DMPC;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

154. The nanoparticle of the invention may comprise:

(a) a cargo;
(b) a lipid component, wherein the lipid component is one or more ionizable lipids and/or one or more cationic lipids;

(c) a phospholipid, wherein the phospholipid is POPC;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

155. The nanoparticle of the invention may comprise:

(a) a cargo;
(b) a lipid component, wherein the lipid component is one or more ionizable lipids and/or one or more cationic lipids;
(c) a phospholipid, wherein the phospholipid is SM;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer.

In any of the embodiments 147 to 155, the nanoparticle may be a non-viral transfection complex.
In any of embodiments 147 to 155, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 147 to 155, the lipid component may be DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA. In any of embodiments 1 to 16, the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.
In any of embodiments 147 to 155, the nanoparticle may not comprise a targeting moiety. In any of embodiments 147 to 155, the nanoparticles may not comprise a targeting peptide.
In embodiments 147 to 155, the cargo comprises preferably a linear DNA or mRNA. In embodiments 147 to 155, the lipid preferably comprises at least one ionizable lipid. In embodiments 147 to 155, the cationic polymer is preferably oligolysine, such as K16 or K30.
The nanoparticle may further comprise a PEG lipid. The PEG lipid may be a PEGylated phospholipid. The PEG lipid may be DMG-PEG.

156. The nanoparticle of the invention may comprise:

(a) a cargo;
(b) a lipid component, wherein the lipid component is one or more ionizable lipids and/or one or more cationic lipids;
(c) a phospholipid, wherein the phospholipid is phosphatidylethanolamine;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

157. The nanoparticle of the invention may comprise:

(a) a cargo;
(b) a lipid component, wherein the lipid component is one or more ionizable lipids and/or one or more cationic lipids;
(c) a phospholipid, wherein the phospholipid is 1,2-dioleoyl-sn-glycero-3-phosphoetha-nolamine (DOPE);
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

158. The nanoparticle of the invention may comprise:

(a) a cargo;
(b) a lipid component, wherein the lipid component is one or more ionizable lipids and/or one or more cationic lipids;
(c) a phospholipid, wherein the phospholipid is phosphatidyl choline;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI..

159. The nanoparticle of the invention may comprise:

(a) a cargo;
(b) a lipid component, wherein the lipid component is one or more ionizable lipids and/or one or more cationic lipids;
(c) a phospholipid, wherein the phospholipid is 1,2-dioleoyl- sn-glycero-3-phosphoethanoltrimethylamine (DOPC);
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI..

160. The nanoparticle of the invention may comprise:

(a) a cargo;
(b) a lipid component, wherein the lipid component is one or more ionizable lipids and/or one or more cationic lipids;
(c) a phospholipid, wherein the phospholipid is DSPC;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI..

161. The nanoparticle of the invention may comprise:

(a) a cargo;
(b) a lipid component, wherein the lipid component is one or more ionizable lipids and/or one or more cationic lipids;
(c) a phospholipid, wherein the phospholipid is DPPC;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

162. The nanoparticle of the invention may comprise:

(a) a cargo;
(b) a lipid component, wherein the lipid component is one or more ionizable lipids and/or one or more cationic lipids;
(c) a phospholipid, wherein the phospholipid is DMPC;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an

oligolysine and an oligohistidine, an oligohistidine and an oligoarginine, an oligoarginine and an oligolysine, or oligolysine, oligohistidine and oligoarginine, or PEI.

163. The nanoparticle of the invention may comprise:

(a) a cargo;
(b) a lipid component, wherein the lipid component is one or more ionizable lipids and/or one or more cationic lipids;
(c) a phospholipid, wherein the phospholipid is POPC;
(d) a steroid lipid; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

In any of the embodiments 156 to 163, the nanoparticle may be a non-viral transfection complex. In any of embodiments 156 to 163, the cargo is a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 156 to 163, the steroid lipid may comprise cholesterol, beta sitosterol, fucosterol, campesterol, stigamstanol (alkyl steroids), secosteroids (vitamin D2, D3) or pentacyclic steroids. In any of embodiments 156 to 163, the lipid component may comprise DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA.
In any of embodiments 156 to 163, the nanoparticle may not comprise a targeting moiety. In any of embodiments 156 to 163 the nanoparticles may not comprise a targeting peptide.
In embodiments 156 to 163 the cargo comprises preferably a linear DNA or mRNA. In embodiments 156 to 163, the lipid preferably comprises at least one ionizable lipid. In embodiments 156 to 163, the steroid lipid comprises preferably cholesterol.
The nanoparticle may further comprise a PEG lipid. The PEG lipid may be a PEGylated phospholipid. The PEG lipid may be DMG-PEG.

164. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid, wherein the steroid lipid comprises one or more of beta-sitosterol; and
(e) a cationic polymer.

165. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;

(d) a steroid lipid, wherein the steroid lipid comprises one or more of fucosterol; and
(e) a cationic polymer.

166. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid, wherein the steroid lipid comprises one or more of campesterol; and
(e) a cationic polymer.

167. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid, wherein the steroid lipid comprises one or more of stigamstanol; and
(e) a cationic polymer.

168. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids, wherein the lipid component comprises SM-102;
(c) a phospholipid;
(d) a steroid lipid, wherein the steroid lipid comprises one or more of cholesterol; and
(e) a cationic polymer.

In any of the embodiments 164 to 168, the nanoparticle may be a non-viral transfection complex.

In any of embodiments 164 to 168, the phospholipid may comprise DOPE, DOPC, DSPC, DPPC, DMPC or POPC. In any of embodiments 164 to 168, the steroid lipid may comprise cholesterol, beta sitosterol, fucosterol, campesterol, stigamstanol (alkyl steroids), secosteroids (vitamin D2, D3) or pentacyclic steroids. In any of embodiments 164 to 168, the cationic polymer may comprise an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!

In any of embodiments 164 to 168, the nanoparticle may not comprise a targeting moiety. In any of embodiments 164 to 168 the nanoparticles may not comprise a targeting peptide.

In embodiments 164 to 168 the cargo comprises preferably a linear DNA or mRNA. In embodiments 164 to 168, the lipid preferably comprises at least one ionizable lipid. In embodiments 164 to 168, the steroid lipid comprises preferably cholesterol.

The nanoparticle may further comprise a PEG lipid. The PEG lipid may be a PEGylated phospholipid. The PEG lipid may be DMG-PEG

169. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid;
(d) a steroid lipid, wherein the steroid lipid comprises one or more of beta-sitosterol; and
(e) a cationic polymer.

170. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5, DOTMA, DTDTMA or DHDTMA;
(c) a phospholipid;
(d) a steroid lipid, wherein the steroid lipid comprises one or more of fucosterol; and
(e) a cationic polymer.

171. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid, wherein the steroid lipid comprises one or more of campesterol; and
(e) a cationic polymer.

172. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid, wherein the steroid lipid comprises one or more of stigamstanol; and
(e) a cationic polymer.

173. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid, wherein the steroid lipid comprises one or more of cholesterol; and
(e) a cationic polymer.

In any of the embodiments 169 to 173, the nanoparticle may be a non-viral transfection complex. In any of embodiments 169 to 173, the phospholipid may comprise DOPE, DOPC, DSPC, DPPC, DMPC or POPC. In any of embodiments 169 to 173, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 169 to 173, the cationic polymer may comprise an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!

In any of embodiments 169 to 173, the nanoparticle may not comprise a targeting moiety. In any of embodiments 169 to 173the nanoparticles may not comprise a targeting peptide.

In embodiments 169 to 173, the cargo comprises preferably a linear DNA or mRNA. In embodiments 169 to 173, the phospholipid is preferably DOPE. In embodiments 169 to 173, the cationic polymer is preferably oligolysine, such as K16 or K30.

The nanoparticle may further comprise a PEG lipid. The PEG lipid may be a PEGylated phospholipid. The PEG lipid may be DMG-PEG

174. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid, wherein the steroid lipid comprises one or more of beta-sitosterol; and
(e) a cationic polymer.

175. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid, wherein the steroid lipid comprises one or more of fucosterol; and
(e) a cationic polymer.

176. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid, wherein the steroid lipid comprises one or more of campesterol; and
(e) a cationic polymer.

177. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid, wherein the steroid lipid comprises one or more of stigamstanol; and
(e) a cationic polymer.

178. The nanoparticle of the invention may comprise:

(a) a cargo;

(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid, wherein the steroid lipid comprises one or more of cholesterol; and
(e) a cationic polymer.

In any of the embodiments 174 to 178 above, the nanoparticle may be a non-viral transfection complex.

In any of embodiments 174 to 178, the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA. In any of embodiments 174 to 178, the cargo is a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 174 to 178, the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PE!

In any of embodiments 174 to 178, the nanoparticle may not comprise a targeting moiety. In any of embodiments 174 to 178, the nanoparticles may not comprise a targeting peptide.

In embodiments 174 to 178, the cargo comprises preferably a linear DNA or mRNA. In embodiments 174 to 178, the lipid preferably comprises at least one ionizable lipid. In embodiments 174 to 178, the cationic polymer is preferably oligolysine, such as K16 or K30.

The nanoparticle may further comprise a PEG lipid. The PEG lipid may be a PEGylated phospholipid. The PEG lipid may be DMG-PEG

179. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of beta-sitosterol; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

180. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of fucosterol; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

181. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of campesterol; and
(e) a cationic polymer, wherein the cationic polymer comprises an oligolysine (linear or branched) such as K16,

K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

182. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of stigamstanol; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

183. The nanoparticle may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of cholesterol; and
(e) a cationic polymer, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

In any of the embodiments 179 to 183 above, the nanoparticle may be a non-viral transfection complex.

In any of embodiments 179 to 183, the phospholipid may comprise DOPE, DOPC, DSPC, DPPC, DMPC or POPC. In any of embodiments 179 to 183, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 179 to 183, the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA.

In any of embodiments 179 to 183, the nanoparticle may not comprise a targeting moiety. In any of embodiments 179 to 183, the nanoparticles may not comprise a targeting peptide.

In embodiments 179 to 183, the cargo comprises preferably a linear DNA or mRNA. In embodiments 179 to 183, the lipid preferably comprises at least one ionizable lipid. In embodiments 179 to 183, the phospholipid is preferably DOPE.

The nanoparticle may further comprise a PEG lipid. The PEG lipid may be a PEGylated phospholipid. The PEG lipid may be DMG-PEG.

184. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and

(e) a cationic polymer wherein the cationic polymer comprises an oligolysine (linear or branched) such as K16, K17 or K30.

185. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligohistidine (linear or branched).

186. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises an oligoarginine (linear or branched).

187. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises oligolysine and oligohistidine.

188. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and
(e) a cationic polymer wherein the cationic polymer comprises oligohistidine and oligoarginine.

189.The nanoparticle may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises oligoarginine and oligolysine.

190. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises or oligolysine, oligohistidine and oligoarginine.

191. The nanoparticle of the invention may comprise:

(a) a cargo, wherein the cargo comprises a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA;
(b) a lipid component, wherein the lipid component is one or more ionizable lipids and/or one or more cationic lipids;
(c) a phospholipid;
(d) a steroid lipid; and
(e) a cationic polymer, wherein the cationic polymer comprises PEI.

In any of the embodiments 184 to 191above, the nanoparticle may be a non-viral transfection complex.

In any of embodiments 184 to 191, the phospholipid may comprise DOPE, DOPC, DSPC, DPPC, DMPC or POPC. In any of embodiments 184 to 191, the steroid lipid may comprise cholesterol, beta sitosterol, fucosterol, sterl 3 or stigamstanol. In any of embodiments 184 to 191, the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA.

In any of embodiments 184 to 191, the nanoparticle may not comprise a targeting moiety. In any of embodiments 184 to 191, the nanoparticles may not comprise a targeting peptide.

In embodiments 184 to 191, the lipid component preferably comprises at least one ionizable lipid. In embodiments 184 to 191, the phospholipid is preferably DOPE. In embodiments 184 to 191, the steroid lipid comprises cholesterol.

The nanoparticle may further comprise a PEG lipid. The PEG lipid may be a PEGylated phospholipid. The PEG lipid may be DMG-PEG.

192. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer wherein the cationic polymer comprises an oligolysine (linear or branched) such as K16, K17 or K30.

193. The nanoparticle may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer, wherein the cationic polymer comprises an oligohistidine (linear or branched).

194. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer, wherein the cationic polymer comprises an oligoarginine (linear or branched).

195. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer, wherein the cationic polymer comprises oligolysine and oligohistidine.

196. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer wherein the cationic polymer comprises oligohistidine and oligoarginine.

197. The nanoparticle of the invention may comprise:

(a) a cargo;
(b) a lipid component, wherein the lipid component is one or more ionizable lipids and/or one or more cationic

lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer, wherein the cationic polymer comprises oligoarginine and oligolysine.

198. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer, wherein the cationic polymer comprises or oligolysine, oligohistidine and oligoarginine.

199. The nanoparticle of the invention may comprise:

(a) a cargo;
(b) a lipid component, wherein the lipid component is one or more ionizable lipids and/or one or more cationic lipids, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA;
(c) a phospholipid;
(d) a steroid lipid; and
(e) a cationic polymer, wherein the cationic polymer comprises PEI.

In any of the embodiments 192 to 199, the nanoparticle may be a non-viral transfection complex.

In any of embodiments 192 to 199, the phospholipid may comprise DOPE, DOPC, DSPC, DPPC, DMPC or POPC. In any of embodiments 192 to 199, the steroid lipid may comprise cholesterol, beta sitosterol, fucosterol, campesterol, stigamstanol (alkyl steroids), secosteroids (vitamin D2, D3) or pentacyclic steroids. In any of embodiments 192 to 199, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA.

In any of embodiments 192 to 199, the nanoparticle may not comprise a targeting moiety. In any of embodiments 192 to 199 the nanoparticles may not comprise a targeting peptide.

In embodiments 192 to 199, the cargo comprises preferably a linear DNA or mRNA. In embodiments 192 to 199, the phospholipid is preferably DOPE. In embodiments 192 to 199, the steroid lipid comprises cholesterol.

The nanoparticle may further comprise a PEG lipid. The PEG lipid may be a PEGylated phospholipid. The PEG lipid may be DMG-PEG.

200. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer wherein the cationic polymer comprises an oligolysine (linear or branched) such as K16, K17 or K30.

201. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer, wherein the cationic polymer comprises an oligohistidine (linear or branched).

202. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer, wherein the cationic polymer comprises an oligoarginine (linear or branched).

203. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer, wherein the cationic polymer comprises oligolysine and oligohistidine.

204. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer wherein the cationic polymer comprises oligohistidine and oligoarginine.

205. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer, wherein the cationic polymer comprises oligoarginine and oligolysine.

206. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;
(d) a steroid lipid; and
(e) a cationic polymer, wherein the cationic polymer comprises or oligolysine, oligohistidine and oligoarginine.

207. The nanoparticle of the invention may comprise:

(a) a cargo;
(b) a lipid component, wherein the lipid component is one or more ionizable lipids and/or one or more cationic lipids;
(c) a phospholipid, wherein the phospholipid comprises DOPE, DOPC, DSPC, DPPC, DMPC or POPC;

(d) a steroid lipid; and
(e) a cationic polymer, wherein the cationic polymer comprises PEI.

In any of the embodiments 200 to 207, the nanoparticle may be a non-viral transfection complex.
In any of embodiments 200 to 207, the cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA. In any of embodiments 200 to 207, the steroid lipid may comprise cholesterol, beta sitosterol, fucosterol, campesterol, stigamstanol (alkyl steroids), secosteroids (vitamin D2, D3) or pentacyclic steroids. In any of embodiments 200 to 207, the lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA.
In any of embodiments 200 to 207, the nanoparticle may not comprise a targeting moiety. In any of embodiments 200 to 207, the nanoparticles may not comprise a targeting peptide.
In embodiments 200 to 207, the cargo comprises preferably a linear DNA or mRNA. In embodiments 200 to 207, the lipid component preferably comprises at least one ionizable lipid. In embodiments 200 to 207, the steroid lipid comprises cholesterol.
The nanoparticle may further comprise a PEG lipid. The PEG lipid may be a PEGylated phospholipid. The PEG lipid may be DMG-PEG.

208. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer wherein the cationic polymer comprises an oligolysine (linear or branched) such as K16, K17 or K30.

209. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer, wherein the cationic polymer comprises an oligohistidine (linear or branched).

210. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer, wherein the cationic polymer comprises an oligoarginine (linear or branched).

211. The nanoparticle of the invention may comprise:

(a) a cargo;
(b) a lipid component, wherein the lipid component is one or more ionizable lipids and/or one or more cationic lipids;

(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer, wherein the cationic polymer comprises oligolysine and oligohistidine.

212. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer wherein the cationic polymer comprises oligohistidine and oligoarginine.

213. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer, wherein the cationic polymer comprises oligoarginine and oligolysine.

214. The nanoparticle of the invention may comprise:

(a) a cargo;
(b) a lipid component, wherein the lipid component is one or more ionizable lipids and/or one or more cationic lipids;
(c) a phospholipid;
(d) a steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer, wherein the cationic polymer comprises or oligolysine, oligohistidine and oligoarginine.

215. The nanoparticle of the invention may comprise:

(a) a cargo;
(b) a lipid component, wherein the lipid component is one or more ionizable lipids and/or one or more cationic lipids;
(c) a phospholipid;
(d) steroid lipid, wherein the steroid lipid comprises one or more of cholesterol, beta-sitosterol, fucosterol, campesterol, stigamstanol (alkyl steroids), secosteroids (vitamin D2, D3) or pentacyclic steroids; and
(e) a cationic polymer, wherein the cationic polymer comprises PEI.

In any of the embodiments 208 to 215, the nanoparticle may be a non-viral transfection complex. In any of embodiments 208 to 215, the phospholipid may comprise DOPE, DOPC, DSPC, DPPC, DMPC or POPC. In any of embodiments 208 to 215, lipid component may comprise DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA. In any of embodiments 208 to 215, cargo may comprise a closed linear deoxyribonucleic acid (DNA) molecule, a linear deoxyribonucleic acid (DNA) molecule comprising one or more nuclease-resistant nucleotides, a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end, mRNA, siRNA, samRNA, miRNA, gRNA, shRNA, antisense RNA, ribosomal RNA, tRNA or snRNA.
In any of embodiments 208 to 215, the nanoparticle may not comprise a targeting moiety. In any of embodiments 208 to 215, the nanoparticles may not comprise a targeting peptide.
In embodiments 208 to 215, the cargo comprises preferably linear DNA or mRNA. In embodiments 208 to 215,

the lipid preferably comprises at least one ionizable lipid. In embodiments 208 to 215, the phospholipid is DOPE.

The nanoparticle may further comprise a PEG lipid. The PEG lipid may be a PEGylated phospholipid. The PEG lipid may be DMG-PEG.

Specific nanoparticles of the invention may include:

A nanoparticle comprising: a closed linear DNA; ALC-0315; DOPE; Cholesterol; DMG-PEG; and K16 (oligolysine consisting of 16 lysine residues), wherein the nanoparticle does not comprise a targeting peptide.

A nanoparticle comprising: a closed linear DNA; DLin-MC3-DMA; DOPE; cholesterol; DMG-PEG; and K16 (oligolysine consisting of 16 lysine residues) wherein the nanoparticle does not comprise a targeting peptide.

A nanoparticle comprising: a closed linear DNA; DOTMA; DOPE; cholesterol; DMG-PEG; and K16 (oligolysine consisting of 16 lysine residues) wherein the nanoparticle does not comprise a targeting peptide.

A nanoparticle comprising: a closed linear DNA; ALC-1315 and DOTMA; DOPE; cholesterol; DMG-PEG; and K16 (oligolysine consisting of 16 lysine residues) wherein the nanoparticle does not comprise a targeting peptide.

A nanoparticle comprising: an mRNA; ALC-0315; DOPE; cholesterol; DMG-PEG; and K16 (oligolysine consisting of 16 lysine residues) wherein the nanoparticle does not comprise a targeting peptide.

A nanoparticle comprising: an mRNA; DLin-MC3-DMA; DOPE; cholesterol; DMG-PEG; and K16 (oligolysine consisting of 16 lysine residues) wherein the nanoparticle does not comprise a targeting peptide.

A nanoparticle comprising: an mRNA; DOTMA; DOPE; cholesterol; DMG-PEG; and K16 (oligolysine consisting of 16 lysine residues) wherein the nanoparticle does not comprise a targeting peptide.

A nanoparticle comprising: an mRNA; ALC-1315 and DOTMA; DOPE; cholesterol; DMG-PEG; and K16 (oligolysine consisting of 16 lysine residues) wherein the nanoparticle does not comprise a targeting peptide.

A nanoparticle comprising: a closed linear DNA; ALC-0315; DOPE; Cholesterol; DMG-PEG; and K30 (oligolysine consisting of 30 lysine residues) wherein the nanoparticle does not comprise a targeting peptide.

A nanoparticle comprising: a closed linear DNA; DLin-MC3-DMA; DOPE; cholesterol; DMG-PEG; and K30 (oligolysine consisting of 30 lysine residues) wherein the nanoparticle does not comprise a targeting peptide.

A nanoparticle comprising: a closed linear DNA; DOTMA; DOPE; cholesterol; DMG-PEG; and K16 (oligolysine consisting of 16 lysine residues) wherein the nanoparticle does not comprise a targeting peptide.

A nanoparticle comprising: a closed linear DNA; ALC-1315 and DOTMA; DOPE; cholesterol; DMG-PEG; and K30 (oligolysine consisting of 30 lysine residues) wherein the nanoparticle does not comprise a targeting peptide.

A nanoparticle comprising: an mRNA; ALC-0315; DOPE; cholesterol; DMG-PEG; and K30 (oligolysine consisting of 30 lysine residues) wherein the nanoparticle does not comprise a targeting peptide.

A nanoparticle comprising: an mRNA; DLin-MC3-DMA; DOPE; cholesterol; DMG-PEG; and K30 (oligolysine consisting of 30 lysine residues) wherein the nanoparticle does not comprise a targeting peptide.

A nanoparticle comprising: an mRNA; DOTMA; DOPE; cholesterol; DMG-PEG; and K30 (oligolysine consisting of 30 lysine residues) wherein the nanoparticle does not comprise a targeting peptide.

A nanoparticle comprising: an mRNA; ALC-1315 and DOTMA; DOPE; cholesterol; DMG-PEG; and K30 (oligolysine consisting of 30 lysine residues) wherein the nanoparticle does not comprise a targeting peptide.

The term "targeting peptide" or "targeting moiety" refers to a peptide sequence or other moiety that has the ability to target, bind and/or interact with a specific cell type. For example, a brain cell targeting sequence may target, bind and/or interact with a receptor on a brain cell, or a biologically active molecule present on the surface of brain cells. The term "specific" refers to the ability to preferentially target, bind and/or interact with a given target, for example, a receptor on a brain, over other cell types. The nanoparticles of the invention may not comprise such a targeting peptide or a targeting moiety.

In the nanoparticle of the invention, including embodiments 1 to 215, the cargo may be a nucleic acid. The cargo may be an RNA molecule or a DNA molecule.

The nucleic acid may be a DNA molecule. The DNA molecule may be a linear DNA molecule or a DNA molecule comprising a linear portion. The nucleic acid may be single-stranded, double-stranded, or partially single-stranded and partially double-stranded.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid (e.g. the closed linear DNA molecule) comprises at least 500 nucleotides.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least

8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid (e.g. the closed linear DNA molecule) comprises at least 500 base pairs.

The linear DNA molecule may be a chromosome. The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The linear double-stranded DNA molecule may comprise a first adaptor molecule at a first end and a second adaptor molecule at a second end. The first adaptor molecule and the second adaptor may comprise one or more protected nucleotides (i.e. nucleotides resistant to nuclease (e.g. exonuclease) digestion). The presence of protected nucleotides may confer resistance to nuclease (e.g. exonuclease) digestion. The linear double-stranded DNA molecule may be a DNA molecule having a double-stranded portion comprising a double stranded linear adaptor comprising protected nucleotides (i.e. nucleotides resistant to nuclease (e.g. exonuclease) digestion) at a first end and a double stranded linear adaptor comprising protected nucleotides (i.e. nucleotides resistant to nuclease (e.g. exonuclease) digestion) at a second end.

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The linear DNA molecule may be a partially closed linear DNA molecule. The partially closed linear DNA molecule may comprise a double-stranded DNA portion that is closed at a first end and open at a second end. The partially closed linear DNA molecule may comprise a double-stranded DNA portion that is closed at a first end by a single-stranded portion (i.e. it may comprise a first hairpin at the first end) and open at a second end. The partially closed linear DNA molecule may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the partially closed linear DNA molecule. This is to say that the open-end region adjacent to the second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The partially closed linear DNA molecule may comprise a hairpin-loop at the 5' end or the 3' end. The partially closed linear DNA molecule may comprise a first adaptor molecule at a first end and a second adaptor molecule at a second end. The first adaptor molecule may comprise a hairpin and a second adaptor may comprise one or more protected nucleotides (i.e. nucleotides resistant to nuclease (e.g. exonuclease) digestion). The hairpin may confer resistance to nuclease (e.g. exonuclease) digestion. The presence of protected nucleotides may confer resistance to nuclease (e.g. exonuclease) digestion. The partially closed linear DNA molecule may be a DNA molecule having a double-stranded portion closed at a first end by ligation of a first adaptor (e.g. hairpin adaptor) to the first end and comprising at a second end a double stranded linear adaptor comprising protected nucleotides (i.e. nucleotides resistant to nuclease (e.g. exonuclease) digestion).

The RNA molecule may be a messenger RNA (mRNA), a transfer RNA, a ribosomal RNA, a small interfering RNA (siRNA), an antisense RNA (an antisense oligonucleotide), a small nuclear RNA (snRNA), a double-stranded RNA, a microRNA (miRNA), short hairpin RNA (shRNA), guide RNA (gRNA), self-amplifying RNA (samRNA), or circular RNA.

The RNA molecule may comprise at least 5, at least 10, at least 15, at least 20, at least 25, at least 30, at least 35, at least 40, at least 45, at least 50, at least 75, at least 100, at least 150, at least 200 nucleotides, at least 500 nucleotides, at least 1,000 nucleotides, at least 2,000 nucleotides, at least 5,000 nucleotides, at least 10,000 nucleotides, at least 15,000 nucleotides or at least 16,000 nucleotides. The RNA molecule may comprise 5-20,000, 6-19,000, 7-18,000, 8-17,000, 9-16,000, 10-15,000, 10-13,000, 15-10,000, 20-5,000, 20-1,000, 20-500, or 25-300 nucleotides. The RNA molecule may comprise one or more protected nucleotides (i.e. nucleotides resistant to nuclease (e.g. exonuclease) digestion) (e.g. phosphorothioated nucleotides).

The RNA molecule may be an mRNA molecule comprising at least 5, at least 10, at least 15, at least 20, at least 25, at least 30, at least 35, at least 40, at least 45, at least 50, at least 75, at least 100, at least 150, or at least 200

nucleotides. The mRNA molecule may comprise 5-20,000, 6-19,000, 7-18,000, 8-17,000, 9-16,000, 10-15,000, 10-13,000, 15-10,000, 20-5,000, 20-1,000, 20-500, or 25-300 nucleotides. The mRNA molecule may comprise one or more protected nucleotides (i.e. nucleotides resistant to nuclease (e.g. exonuclease) digestion) (e.g. phosphorothioated nucleotides).

The RNA molecule may be a self-amplifying mRNA (samRNA) molecule comprising at least 3,000, at least 4,000, at least 5,000, at least 6,000, at least 7,000, at least 8,000, at least 9,000, at least 10,000, at least 11,000, at least 12,000, at least 13,000, at least 14,000, at least 15,000, at least 16,000, at least 17,000, at least 18,000, at least 19,000, or at least 20,000 nucleotides. The samRNA molecule may comprise 3,000-22,000, 5,000-21,000, 7,000-20,000, or 8,000-17,000 nucleotides. The samRNA molecule may comprise one or more protected nucleotides (i.e. nucleotides resistant to nuclease (e.g. exonuclease) digestion) (e.g. phosphorothioated nucleotides).

The siRNA may comprise a double-stranded portion of at least 17 base pairs, at least 18 base pairs or preferably at least 19 base pairs. The siRNA may comprise a double stranded portion of 17-30 base pairs, 18-27 base pairs, 19-24 base pairs or preferably 19-21 base pairs. The siRNA may comprise one or more protected nucleotides (i.e. nucleotides resistant to nuclease (e.g. exonuclease) digestion) (e.g. phosphorothioated nucleotides).

The miRNA may comprise at least 5, at least 10, at least 15, at least 20, at least 25, at least 30, at least 35, at least 40, at least 45, at least 50, at least 75, at least 100 nucleotides. The miRNA molecule may comprise 10-200, 12-150, 15-125, 17-100, 18-75, 20-75, 20-50, or 20-30 nucleotides.

The miRNA may comprise a double-stranded portion of at least 15 base pairs, at least 17 base pairs or preferably at least 20 base pairs. The miRNA may comprise a double stranded portion of 15-30 base pairs, 17-27 base pairs, 20-25 base pairs or preferably 21-23 base pairs. The miRNA may comprise one or more protected nucleotides (i.e. nucleotides resistant to nuclease (e.g. exonuclease) digestion) (e.g. phosphorothioated nucleotides).

The antisense RNA may comprise at least 18, at least 19, at least 20, at least 21, at least 22, or at least 23 nucleotides. The antisense RNA may comprise 18-24 nucleotides or preferably 19-23 nucleotides. The antisense RNA may comprise one or more protected nucleotides (i.e. nucleotides resistant to nuclease (e.g. exonuclease) digestion) (e.g. phosphorothioated nucleotides).

In the nanoparticle of the invention, including embodiments 1 to 215, the term "phospholipid" refers to a lipid comprising a fatty acid chain and a phosphate group. Phospholipids are typically neutral molecules in that they do not have an overall charge, unlike a cationic lipid, which is positively charged. Phospholipids are typically zwitterionic molecules comprising both positive and negative charged components, but no overall charge. For example, the phospholipid may be DOPE (1,2-dioleoyl-sn-glycero-3-phosphoetha-nolamine), DOPC (1,2-dioleoyl- sn-glycero-3-phosphoethanoltrimethylamine) DSPC (1,2-distearoyl-*sn*-glycero-3-phosphocholine), DPPC (Dipalmitoylphosphatidylcholine), DMPC (1,2-dimyristoyl-*sn*-glycero-3-phosphocholine), or POPC (1-Palmitoyl-2-oleoylphosphatidylcholine).

In the nanoparticle of the invention, including embodiments 1 to 215, the steroid lipid may be cholesterol or a derivative thereof (i.e. a cholesterol derivative).

The lipid component (one or more ionizable and/or one or more cationic lipids) together with the phospholipid and the steroid lipid (and optionally a PEG lipid) may form a liposome.

The liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, at least 25%, at least 30% at least 35%, at least 40%, at least 45%, at least 50%, or at least 55% a cholesterol or derivative thereof (as defined by molar amount of a cholesterol or derivative thereof). That is to say that the liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, or at least 25% a cholesterol or derivative thereof and at least 99%, at least 98%, at least 97%, at least 96% at least 95%, at least 94%, at least 93%, at least 92%, at least 91%, at least 90%, at least 89%, at least 88%, at least 87%, at least 86%, at least 85%, at least 84%, at least 83%, at least 82%, at least 81%, at least 80% at least 79%, at least 78%, at least 77%, at least 76%, at least 75%, at least 70%, at least 65%, at least 60%, at least 55%, at least 50%, or at least 45% of other lipids in the liposome (as defined by molar ratio). The liposome may comprise 10% cholesterol or derivative thereof. The liposome may comprise 20% cholesterol or derivative thereof. The liposome may comprise 30% cholesterol or derivative thereof. The liposome may comprise 40% cholesterol or derivative thereof.

In the nanoparticle of the invention, including embodiments 1 to 215, the cholesterol derivative may be cholesterol, beta-sitosterol, fucosterol, campesterol, stigamstanol (alkyl steroids), secosteroids (vitamin D2, D3) or pentacyclic steroids. The structures of fucosterol, campesterol and stigamstanol are shown below (from Nano Lett. 2020, 20, 6, 4543-4549):

A

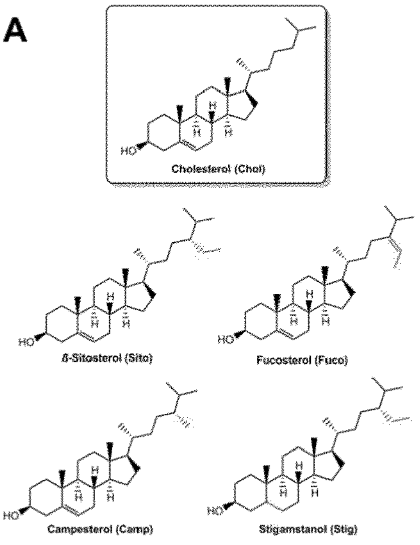

Cholesterol (Chol)

ß-Sitosterol (Sito)

Fucosterol (Fuco)

Campesterol (Camp)

Stigamstanol (Stig)

In the nanoparticle of the invention, including embodiments 1 to 215, the cationic polymer may be a nucleic-acid binding cationic polymer. A cationic component of the cationic polymer may be used to establish a desired charge (i.e. and negative/positive ratio) of the nanoparticle. A specific charge (i.e. a nitrogen/phosphate ratio) may be required to facilitate or enhance cell transfection.

The nucleic acid-binding cationic polymer may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the nucleic acid-binding cationic polymer comprises at least 16, at least 17 or at least 30 cationic monomers. The nucleic acid-binding cationic polymer may comprise less than 10, or less than 9 cationic monomers. The nucleic acid-binding cationic polymer may comprise 8 cationic monomers.

The cationic polymer may comprise a lysine, a histidine, or an arginine. The nucleic acid-binding cationic polymer may comprise an oligolysine (linear or branched), an oligohistidine (linear or branched) or an oligoarginine (linear or branched). For example, the nucleic acid-binding cationic polymer may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the nucleic acid-binding cationic polymer comprises at least 16, at least 17, or at least 30 lysine residues. More preferably still, the nucleic acid-binding cationic polymer comprises at least 17 lysine residues. The nucleic acid-binding cationic polymer may comprise less than 10, or less than 9 lysine residues. The nucleic acid-binding cationic polymer may comprise 8 lysine residues.

The cationic polymer may be linear or branched. The nucleic acid-binding cationic polymer may be linear or branched. For example, the nucleic acid-binding cationic polymer may comprise at least 16, at least 17, or at least 30 lysine residues in a linear chain. Alternatively, the nucleic acid-binding cationic polymer may comprise at least 16, at least 17, or at least 30 lysine residues in a branched chain. The nucleic acid may be a nucleic acid-binding cationic polymer. The nucleic acid-binding cationic polymer may be linear or branched. For example, the nucleic acid-binding cationic polymer (e.g. the DNA-binding cationic polymer) may comprise at least 16, at least 17, or at least 30 lysine residues in a linear chain. Alternatively, the nucleic acid-binding cationic polymer (e.g. the DNA-binding cationic polymer) may comprise at least 16, at least 17, or at least 30 lysine residues in a branched chain.

The lipid component (one or more ionizable and/or one or more cationic lipids) together with the phospholipid and the steroid lipid (and optionally a PEG lipid) may form a liposome.

The lipid component comprises a cationic lipid or an ionizable lipid (or a combination thereof).

In the nanoparticle of the invention, including embodiments 1 to 215, the liposome comprises at least one lipid component. The liposome may comprise at least one cationic lipid. The liposome may comprise at least one ionizable lipid. The liposome may comprise at least one ionizable lipid, at least one steroid lipid and at least one phospholipid. The liposome may comprise at least one PEG lipid. The liposome may comprise at least one cationic lipid, at least one steroid lipid and at least one phospholipid. The liposome may comprise at least one ionizable lipid, at least one steroid

lipid, at least one phospholipid and at least one PEG lipid. The liposome may comprise at least one cationic lipid, at least one steroid lipid, at least one phospholipid and at least one PEGylated lipid. The liposome may comprise one or more of the ionizable lipids described in Table 1.

The ionizable lipid may be any one or more of those listed in the table below:

| | |
|---|---|
| DLin-MC3-DMA | 4-(dimethylamino)-butanoic acid, (10Z,13Z)-1-(9Z,12Z)-9,12-octadecadien-1-yl-10,13-nonadecadien-1-yl ester |
| DLin-KC2-DMA | N,N-dimethyl-2,2-di-(9Z,12Z)-9,12-octadecadien-1-yl-1,3-dioxolane-4-ethanamine |
| DLin-DMA | 1,2-Dilinoleyloxy-N,N-dimethyl-3-aminopropane |
| TCL053 | 2-(((4-(dimethylamino)butanoyl)oxy)methyl)-2-((((Z)-tetradec-9-enoyl)oxy)methyl)propane-1 , 3-diyl (9Z,9'Z)-bis(tetradec-9-enoate) |
| SM-102 | 8-[(2-hydroxyethyl)[6-oxo-6-(undecyloxy)hexyl]amino]-octanoic acid, 1-octylnonyl ester |
| ALC-0315 | 2-hexyl-decanoic acid, 1, 1'-[[(4-hydroxybutyl)imino]di-6,1 -hexanediyl] ester |
| C12-200 | 1,1'-[[2-[4-[2-[[2-[bis(2-hydroxydodecyl)amino]ethyl](2-hydroxydodecyl)amino]ethyl]-1-piperazinyl]ethyl]imino]bis-2-dodecanol |
| DODMA | N,N-dimethyl-2,3-bis[(9Z)-9- octadecen-1-yloxy]-1-propanamine |
| DODAP | 9Z-octadecenoic acid, 1,1'-[1-[(dimethylamino)methyl]-1,2-ethanediyl] ester |
| Lipid A9 | bis(2-butyloctyl) 10-(N-(3-(dimethylamino)propyl)nonanamido)nonadecanedioate |
| 9A1P9 | 2-(dioctylamino)ethyl nonyl hydrogen phosphate |
| Lipid C24 | bis(2-octyldodecyl) 3,3'-((4-(4-methylpiperazin-1 -yl)butyl)azanediyl)dipropionate |
| Lipid LP01 | 9Z,12Z-octadecadienoic acid, 3-[4,4-bis(octyloxy)-1-oxobutoxy]-2-[[[[3-(diethylamino)propoxy] carbonyl]oxy]methyl]propyl ester |
| Lipid 5 | 8-[(2-hydroxyethyl)[8-(nonyloxy)-8-oxooctyl]amino]-octanoic acid, 1 -octylnonyl ester |

The liposome may comprise one or more of the following cationic lipids: DTDTMA (ditetradecyl trimethyl ammonium), DOTMA (2,3-dioleyloxypropy1-1-trimentyl ammonium), or DHDTMA (dihexadecyl trimethyl ammonium). In addition to the cation, the cationic lipids may comprise a counter anion, for example, an inorganic counter ion, especially a pharmaceutically acceptable anion such as chloride or bromide. Preferably, the cationic lipid is DOTMA.

The nanoparticle of the invention comprises a phospholipid. The term "phospholipid" refers to a lipid comprising a fatty acid chin and a phosphate group. Phospholipids are typically neutral molecules in that they do not have an overall charge, unlike a cationic lipid, which is positively charged. Phospholipids are typically zwitterionic molecules comprising both positive and negative charged components, but no overall charge. For example, the phospholipid may be DOPE (1,2-dioleoyl-sn-glycero-3-phosphoetha-nolamine), DOPC (1,2-dioleoyl- sn-glycero-3-phosphoethanoltrimethylamine) DSPC (1,2-distearoyl-*sn*-glycero-3-phosphocholine), DPPC (Dipalmitoylphosphatidylcholine), DMPC (1,2-dimyristoyl-*sn*-glycero-3-phosphocholine), or POPC (1-Palmitoyl-2-oleoylphosphatidylcholine). Preferably, the phospholipid is DOPE.

The nanoparticles of the invention may comprise a PEGylated lipid.

In the nanoparticle of the invention, including embodiments 1 to 215, the PEG lipid may be provided by the phospholipid comprising a PEG (polyethylene glycol) moiety. The PEG moiety may have a molecular weight of from about 100 to about 10,000, optionally the PEG moiety has a molecular weight of from about 250 to about 7,500, optionally the PEG moiety has a molecular weight of from about 500 to about 5,000, optionally the PEG moiety has a molecular weight of from about 750 to about 4,000, optionally the PEG moiety has a molecular weight of from about 1,000 to about 3,000, optionally the PEG moiety has a molecular weight of approximately 2,000.

The lipid may be a PEGylated lipid e.g. DMG-PEG.

The lipid component (one or more ionizable and/or one or more cationic lipids) together with the phospholipid and the steroid lipid (and optionally a PEG lipid) may form a liposome.

In the nanoparticle of the invention, including embodiments 1 to 215, the steroid lipid may be cholesterol or a derivative thereof (i.e. a cholesterol derivative). The nanoparticle of the invention may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, at least 25%, at least 30% at least 35%, at least 40%, at least

45%, at least 50%, or at least 55% cholesterol or derivative thereof (as defined by molar amount of cholesterol or derivative thereof). That is to say that the nanoparticle may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, or at least 25% cholesterol and at least 99%, at least 98%, at least 97%, at least 96% at least 95%, at least 94%, at least 93%, at least 92%, at least 91%, at least 90%, at least 89%, at least 88%, at least 87%, at least 86%, at least 85%, at least 84%, at least 83%, at least 82%, at least 81%, at least 80% at least 79%, at least 78%, at least 77%, at least 76%, at least 75%, at least 70%, at least 65%, at least 60%, at least 55%, at least 50%, or at least 45% of other lipids in the liposome (as defined by molar ratio). The cholesterol derivative may be beta-sitosterol, fucosterol, campesterol or stigamstenol.

The molar ratio of the lipid component comprising a ionizable lipid and/or a cationic lipid to a phospholipid in the nanoparticle (or non-viral transfection complex) may be 1:1, 1:2, 1:3, 1:4, 1:5, 2:1, 3:1, 4:1, 5:1 ,6:1, 7:1, 8:1, 9:1 or 10:1. Preferably, the molar ratio of at least one cationic lipid to at least one phospholipid in the nanoparticle (or non-viral transfection complex) is 1:1 or 2:1. For example, the molar ratio of DOTMA to DOPE in the nanoparticle (or non-viral transfection complex) may be 1:1. That is to say that the molar amount of DOTMA and DOPE in the nanoparticle is the same. The molar ratio of DOTMA to DOPE in the nanoparticle (or non-viral transfection complex) may be 2:1. That is to say that the molar amount of DOTMA is twice the molar amount of DOPE. The molar ratio of ALC-0315 to DOPE may be 1:1, 2:1, 3:1, 4:1, 5:1 ,6:1, 7:1, 8:1, 9:1 or 10:1.

The nanoparticle may comprise an ionizable lipid and a cationic lipid. The nanoparticle of the invention may comprise an ionizable lipid, a phospholipid and a cationic lipid. The nanoparticle may further comprise cholesterol. The nanoparticle of the invention may comprise an ionizable lipid, a phospholipid and cholesterol. The nanoparticle of the invention may comprise a cationic lipid, a phospholipid and cholesterol. The nanoparticle of the invention may comprise an ionizable lipid, a phospholipid, cholesterol and a PEG lipid. The nanoparticle of the invention may comprise a cationic lipid, a phospholipid, cholesterol and a PEG lipid. The nanoparticle of the invention may comprise an ionizable lipid, a phospholipid, cholesterol and a cationic polymer. For example, the nanoparticle may comprise ALC-0315, DMG-PEG, cholesterol and DOPE and peptide K16. The nanoparticle of the invention may comprise DLin-MC3-DMA, DMG-PEG, cholesterol and DOPE and peptide K30. The nanoparticle of the invention may comprise ALC-0315, DMG-PEG, cholesterol, DOPE and DOTMA and PEI. The nanoparticle of the invention may comprise DLin-MC3-DMA, DOTMA, DMG-PEG, cholesterol and DOPE and peptide K16.

The mass ratio of cationic polymer to DNA molecule described herein in the nanoparticle may be from 0.1:1 to 9:1 (cationic polymer: nucleic acid cargo). The mass ratio may be from 1:1 to 6:1. The mass ratio may be from 2:1 to 4:1. For example, the mass ratio of cationic polymer to DNA or RNA molecule described herein may be about 0.1:1, about 0.5:1, about 1:1, about 1.5:1, about 2:1, about 2.5:1, about 3:1, about 3.5:1, about 4:1, about 4.5:1, about 5:1, about 5.5:1, about 6:1, about 6.5:1, about 7:1, about 7.5:1, about 8:1, about 8.5:1 or about 9:1 [cationic polymer: nucleic acid cargo].

The molar ratio of cationic polymer to DNA molecule described herein in the nanoparticle may be at least 50:1, at least 100:1, at least 150:1, at least 200:1 , at least 250:1 at least 300:1, at least 350:1, at least 400:1, at least 450:1. at least 500:1, at least 550:1 at least 600:1, at least 650:1, at least 700:1, at least 750:1 at least 800:1, at least 850:1, at least 900:1, at least 950:1 at least 1000:1, at least 1050:1, at least 1100:1, at least 1150:1 at least 1200:1, at least 1250:1, or at least 1300:1. The molar ratio may be from 100:1 to 1500:1, from 200:1 to 1200:1, from 300:1 to 1100:1, from 400:1 to 1000:1, from 500:1 to 900:1, from 600:1 to 800:1 or about 700:1.

The cationic polymer may be an oligopeptide. The oligopeptide may comprise at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40 amino acids. The amount of positively charged amino acids in the cationic polymer may be at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31.

The molar ratio of the cationic polymer to the DNA or RNA molecule in the nanoparticle may be at least 350:1 or at least 650:1 and the N/P ratio (charge ratio (i.e. Nitrogen/Phosphate molar ratio) of the nanoparticle may be about 4. The molar ratio of the cationic polymer to the nucleic acid molecule may be from 100:1 to 650:1 or from 200:1 to 400:1 and the N/P ratio of the nanoparticle may be about 4.

The molar ratio of the cationic polymer to the DNA molecule in the nanoparticle may be at least 500:1 or at least 900:1 and the N/P ratio of the nanoparticle may be about 6. The molar ratio of the cationic polymer to the nucleic acid molecule may be from 200:1 to 1100:1 or from 350:1 to 700:1 and the N/P ratio of the nanoparticle may be about 6.

The molar ratio of the cationic polymer to the DNA molecule in the nanoparticle may be at least 750:1 or at least 1400:1 and the N/P ratio of the nanoparticle may be about 8.

The molar ratio of the cationic polymer to the DNA molecule in the nanoparticle may be from 550:1 to 1400:1,

or from 650:1 to 1100:1 and the N/P ratio of the nanoparticle is 9.

The cationic polymer may comprise at least 30 (e.g. 31) positively charged amino acids. In such cases, the peptide : DNA or RNA cargo molar ratio may be between 30:1 and 1000:1, 75:1 to 750:1 or between 100:1 and 500:1.

The cationic polymer may comprise at least 15 (e.g. 16) positively charged amino acids. In such cases, the peptide : DNA or RNA cargo(e.g. nucleic acid) molar ratio may be between 50:1 to 2000:1, 75:1 to 1000:1 or between 200:1 and 1400:1.

The specific ratio of cationic polymer to DNA or RNA cargo allows for an effective and stable formulation of the nanoparticle.

The molar ratio of DNA molecule: lipid: cationic polymer in the nanoparticle may be 1 (DNA molecule) to between 1000-6000 (lipid) to between 30 to 2000 (peptide).

The lipid component (one or more ionizable and/or one or more cationic lipids) together with the phospholipid and the steroid lipid (and optionally a PEG lipid) may form a liposome.

The mass ratio of nucleic acid (e.g., DNA or mRNA) to liposome is from 1:5 to 1:15, or about 1:11.

The nanoparticle (e.g. the non-viral transfection complex) may have a particle size of less than 300 nm, for example less than 200 nm or less than 100 nm or about 80nm . In a population of particles there will be some variation in particle size but the above criteria will be taken as met if at least 70%, at least 80% or at least 90% of the particles are of less than 300 nm, for example less than 200nm or less than 100 nm. Preferably, in a population of particles, at least 80% of the particles are less than 300 nm, for example less than 200nm or less than 100 nm. Preferably, the nanoparticle, is a self-assembled nanoparticle. In a population of self-assembled nanoparticles (e.g. non-viral transfection complexes), the size of the particles may be lower than the size of particles which were produced by methods other than self-assembly methods (e.g. methods in which the lipid component is conjugated to a cationic polymer before encapsulating of a cargo). For example, the size of the self-assembled nanoparticles may be lower by at least 5%, at least 10%, at least 15%, at least 20%, at least 25%, at least 30%, at least 35% or at least 40% than the size of particles which were produced by methods other than self-assembly methods. Preferably, the self-assembly of the nanoparticle is performed using a microfluidics device.

In a population or a library of nanoparticles described herein, each nanoparticle may have substantially the same size as at least 9, at least 99, at least 999, at least 9,999, at least 99,999 other nanoparticles in the library.

Thus, in a population or a library of nanoparticles (e.g. non-viral transfection complexes) of the present invention, the nanoparticles may be monodisperse or substantially monodisperse. The nanoparticles (e.g. the non-viral transfection complex) may have a polydispersity index (PDI) of less than 0.4, less than 0.3, less than 0.2, or less than 0.15. The nanoparticles (e.g. the non-viral transfection complex) may have a polydispersity index similar or equal to the polydispersity index of empty liposomes (control).

In a population or library of self-assembled nanoparticles (e.g. non-viral transfection complexes), a polydispersity index may be lower than the polydispersity index of a population of nanoparticles which were produced by methods other than self-assembly methods (e.g. methods in which the lipid component is conjugated to a cationic polymer before encapsulating of a cargo). The polydispersity index of a population or library of self-assembled nanoparticles may be lower by at least 1%, at least 5%, at least 10%, at least 15%, at least 20%, at least 25%, at least 30%, at least 35%, or at least 40% than the polydispersity index of a population or a library of nanoparticles which were produced by methods other than self-assembly methods.

The polydispersity index is a measure of the heterogeneity of a sample based on size. Polydispersity can occur due to size distribution in a sample or agglomeration or aggregation of the sample during isolation or analysis. The skilled person would know different ways of determining the polydispersity index. For example, the polydispersity index can be obtained from instruments that use dynamic light scattering (DLS) or determined from electron micrographs. In general, the polydispersity index values of less than 0.3 are more common to monodisperse or substantially monodisperse samples, while values of above 0.7 are common to a broad size (e.g. polydisperse) distribution of particles.

The inventors of the present application have discovered that the generation of monodisperse or substantially monodisperse nanoparticles of the present invention is facilitated by a specific ratio of charges between a cationic polymer, a lipid component and a cargo.

Thus, the invention provides a library comprising two or more nanoparticles described herein. A library may comprise at least 3, at least 4, at least 5, at least 6, at least 7, at least 8, at least 9, at least 10, at least 20, at least 30, at least 40, at least 50, at least 60, at least 70, at least 80, at least 90, at least 100, at least 1000, at least 10,000, at least 100,000 nanoparticles described herein. The polydispersity index (PDI) of the nanoparticles in the library may be less than about 0.3, or less than about 0.2, for example between 0.3 and 0.1, between 0.25 and 0.15, or between 0.22 and 0.13. The polydispersity index (PDI) of the nanoparticles in the library may be 0.22, 0.19, 0.17, 0.13.

The nanoparticle (e.g. the non-viral transfection complex) may have a charge ratio (i.e. Nitrogen/Phosphate (N/P) molar ratio) of 2.0-13.0, 3.0-12.0, 4.0-11.0 or 4.0-12.0. The nanoparticle (e.g. the non-viral transfection complex) may have a charge ratio (i.e. N/P ratio) of about 4.0, about 4.2, about 4.5, about 4.7, about 5.0, about 5.2, about 5.5, about 5.7, about 6.0, about 6.5, about 7.5, about 8.5, about 9.0, about 9.5 or about 10.5. Preferably, the nanoparticle

has a charge ratio (i.e. N/P ratio) of 7.0-11.0, 4.0-9.0, or 3.0-8.0.

Preferably, the nanoparticle has a charge ratio of about 4.0, or about 5.0, or about 6.0. or about 9.0.

For example, if the nucleic acid-binding component of the nanoparticle is a nucleic acid-binding cationic polymer comprising 16 lysine residues, the nanoparticle may have a charge ratio (i.e. N/P ratio) of 3.0-12.0 or 4.0-12.0, preferably 7.0-11.0. For example, if the nucleic acid-binding component of the nanoparticle is a nucleic acid-binding cationic polymer comprising 16 lysine residues, the nanoparticle may have a charge ratio (i.e. N/P ratio) of about 4.0, about 5.0, about 6.0 or about 9.0.

For example, if the cationic polymer comprises 30 lysine residues, the nanoparticle may have a charge ratio (i.e. N/P ratio) of 3.0-8.0 or 4.0-8.0. For example, if the cationic polymer comprises 30 lysine residues, the nanoparticle may have a charge ratio (i.e. N/P ratio) of about 4.2, about 4.5, about 4.7, about 5.2, about 5.5, about 5.7 about 6.5, or about 7.5 Preferably, when the cationic polymer comprises 30 lysine residues, the N/P ratio may be about 4.0, about 5.0, about 6.0 or about 9.0.

The nanoparticle (e.g. the non-viral transfection complex) may have a charge ratio (i.e. Nitrogen/Phosphate (N/P) molar ratio) of 3.5-11.0, 4.0-11.0, 5.0-11.0, 9.0-11.0, 7.0-13.0, 7.2-13.0, 7.5-13.0, or 8.0-13.0.

The charge ratio (N/P ratio) is calculated from the molar amount of each free amine group(s) (N) in the components of the nanoparticle to the phosphate groups (P) in the components of the nanoparticle. For example, the free amine group(s) may come from the cationic polymer and the lipid component and the phosphate group(s) may come from the phosphate groups in the nucleic acid molecule (e.g. DNA molecule) (P). The charge ratio is typically driven by the mass of the cationic polymer. An N/P ratio of 1, for example, consists of 1 amine group to 1 phosphate and is conventionally expressed as N/P = 1. Similarly, a ratio N/P = 5 refers to the ratio between 5 amine groups to 1 phosphate group.

For example, for nanoparticles consisting of lipid, peptide and mRNA components, the N/P ratio is calculated as followed:

$$\frac{N}{P}ratio = \frac{(Moles_{lip} \times N_{lip)} + (Moles_{pep} \times N_{pep})}{(Moles_{mRNA} \times P_{mRNA})}$$

For three-component nanoparticles, the mass of each component to formulate can be calculated from the desired mass of nucleic acid to be encapsulated and the desired charge ratio of Peptide/mRNA and Lipid/mRNA.

First, P must be calculated:

$$Moles_{mRNA} = \left(\frac{Mass_{mRNA}}{MW_{mRNA}}\right)$$

$$P = Moles_{mRNA} \times Number\ of\ bases_{mRNA}$$

The number of moles required of each lipid and peptide in the final formulation can then be calculated as followed:

$$Moles_{pep} = \frac{\left(Charge\ ratio\,\frac{Peptide}{mRNA} \times P\right)}{N_{pep}}$$

$$Moles_{lip} = \frac{\left(Charge\ ratio\,\frac{Lipid}{mRNA} \times P\right)}{N_{lip}}$$

Where $N_{pep}$ is equal to the number of positively charged amino acids in the peptide sequence (Lysine, Histidine and Arginine), and $N_{lip}$ is equal to the number of free amine groups in the cationic lipid component.

Therefore, the mass of peptide or lipid to formulate in the final nanoparticle formulation can be calculated as follows:

$$Mass = Moles\ \times Molecular\ Weight$$

Finally, the N/P ratio of the final nanoparticle equation can be calculated using the formulation above.

The term "about" as used herein for numerical parameters refers to a value within 10% of the underlying parameter (i.e. plus or minus 10%). For example, a charge ratio of "about 4.5" can include charge (N/P) ratios between 4.1 - 5.0, including charge ratios 4.1 and 5.0.

The nanoparticle (e.g. the non-viral delivery complex) may deliver the cargo, such as a nucleic acid cargo, to a cell with a transfection efficiency of at least 5%, at least 10%, at least 15%, at least 20%, at least 25%, at least 30%, at least 35%, at least 40%, at least 50%, at least 52%, at least 55%, at least 60%, at least 65%, at least 70%, at least 75%, at least 80%, at least 85%, at least 90%, at least 95%, or at least 98%. Preferably, the transfection efficiency is at least 15%. Preferably, the transfection efficiency of a nanoparticle comprising closed linear DNA as a cargo is better than a nanoparticle comprising a plasmid DNA.

The nanoparticle of the present invention facilitates delivery of a DNA or RNA cargo to a cell. The improved delivery is determined, for example, by determining the transfection efficiency (i.e. the percentage of cells transfected from cells non-transfected). The nanoparticle (e.g. the non-viral delivery complex) comprising a cationic polymer may deliver the DNA or RNA cargo to a cell a transfection efficiency of at least 5%, 10%, 15%, 20%, 25%, 30%, 35%, 40%, 45% or 50%. The nanoparticle (e.g. the non-viral delivery complex) comprising a cationic polymer may deliver the DNA or RNA cargo to a cell with a transfection efficiency which is higher than the transfection efficiency of a nanoparticle without the cationic polymer (i.e. a nanoparticle comprising a lipid component, a phospholipid, a steroid lipid and a cargo). Preferably, the transfection efficiency of a nanoparticle comprising a comprising a cationic polymer, such as a cationic oligopeptide, is higher than the transfection efficiency of a nanoparticle without the cationic peptide.

The skilled person is aware of different ways to determine the transfection efficiency. For example, the transfection efficiency may be determined by measuring or detecting the level of expression of genes encoded on the nucleic acid. For example, the nucleic acid may encode green fluorescent protein (GFP), which, once expressed, may be detected to determine the transfection efficiency.

The nanoparticle is preferably a self-assembled nanoparticle. The nanoparticle may be a nanoparticle which is produced by a process in which pre-existing components (e.g. a lipid component, a cargo and a cationic polymer) form an organized structure as a consequence of specific, local interactions among the components themselves, without external direction.

The DNA or RNA cargo, the lipid component, the steroid lipid, the phospholipid and the cationic polymer may reversibly interact to form a self-assembled nanoparticle. The DNA or RNA cargo, the lipid component, the steroid lipid, the phospholipid and the cationic polymer may reversibly interact in the self-assembled nanoparticle through intermolecular forces. The DNA cargo, the lipid component, the steroid lipid, the phospholipid and the cationic polymer may reversibly interact in the self-assembled nanoparticle through non-covalent interactions. The DNA or RNA cargo, the lipid component, the steroid lipid, the phospholipid and the cationic polymer may reversibly interact in the self-assembled nanoparticle through hydrogen bonds, van der Waals, hydrophobic, and/or electrostatic interactions. The DNA or RNA cargo, the lipid component and the cationic polymer may not be conjugated or linked by forces other than inter-molecular forces in the self-assembled nanoparticle.

The cargo suitable for use in the nanoparticle (e.g. non-viral transfection complex) described herein is a nucleic acid. Preferably, the cargo comprises a closed linear DNA molecule or a linear DNA molecule. The closed linear DNA molecule and/or the linear DNA molecule may have an enhanced resistance to nuclease (e.g. exonuclease) digestion.

The nucleic acid may be a DNA molecule. The nucleic acid may be a closed linear DNA molecule (e.g. a covalently-closed linear DNA molecule) or a linear DNA molecule (e.g. linear double-stranded DNA molecule). The DNA molecule may be a linear DNA molecule or a partially linear DNA molecule (i.e. the DNA molecule may comprise a linear portion). The nucleic acid may be single-stranded, double-stranded, or partially single-stranded and partially double-stranded.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid (e.g. the closed linear DNA molecule) comprises at least 500 nucleotides.

The nucleic acid (e.g. the closed linear DNA molecule) may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid (e.g. the closed linear DNA molecule) comprises at least 500 base pairs.

The linear DNA molecule may be a closed linear DNA molecule. The closed linear DNA molecule may comprise a double-stranded DNA portion that is closed at a first end by a first single-stranded portion (i.e. it may comprise a first

hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The linear DNA molecule may be a portion of a chromosome or a gene. The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The linear double-stranded DNA molecule may comprise a first adaptor molecule at a first end and a second adaptor molecule at a second end. The first adaptor molecule and the second adaptor may comprise one or more protected nucleotides (i.e. nucleotides resistant to nuclease (e.g. exonuclease) digestion). The presence of protected nucleotides may confer resistance to nuclease (e.g. exonuclease) digestion. The linear double-stranded DNA molecule may be a DNA molecule having a double-stranded portion comprising a double stranded linear adaptor comprising protected nucleotides (i.e. nucleotides resistant to nuclease (e.g. exonuclease) digestion) at a first end and a double stranded linear adaptor comprising protected nucleotides (i.e. nucleotides resistant to nuclease (e.g. exonuclease) digestion) at a second end.

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The linear DNA molecule be a partially closed linear DNA molecule. The partially closed linear DNA molecule may comprise a double-stranded DNA portion that is closed at a first end and open at a second end. The partially closed linear DNA molecule may comprise a double-stranded DNA portion that is closed at a first end by a single-stranded portion (i.e. it may comprise a first hairpin at the first end) and open at a second end. The partially closed linear DNA molecule may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the partially closed linear DNA molecule. This is to say that the open-end region adjacent to the second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The open-end region adjacent to the second end may comprise a sense strand and an antisense strand. The open-end region adjacent to the second end may comprise one or more nuclease-resistant nucleotides in the sense strand or the antisense strand. The open-end region adjacent to the second end may comprise one or more nuclease-resistant nucleotides in both the sense and antisense strand. The open-end region adjacent to the second end may comprise at least two, at least three, at least four, or at least five nuclease-resistant nucleotides in the sense and/or antisense strand(s). Preferably, the open-end region adjacent to the second end comprises five nuclease-resistant nucleotides in the sense and/or antisense strands.

The partially closed linear DNA molecule may comprise a hairpin-loop at the 5' end or the 3' end. The partially closed linear DNA molecule may comprise a first adaptor molecule at a first end and a second adaptor molecule at a second end. The first adaptor molecule may comprise a hairpin and a second adaptor may comprise one or more protected nucleotides (i.e. nucleotides resistant to nuclease (e.g. exonuclease) digestion). The hairpin may confer resistance to nuclease (e.g., exonuclease) digestion. The presence of protected nucleotides may confer resistance to nuclease (e.g. exonuclease) digestion. The partially closed linear DNA molecule may be a DNA molecule having a double-stranded portion closed at a first end by ligation of a first adaptor (e.g. hairpin adaptor) to the first end and

comprising at a second end a double stranded linear adaptor comprising protected nucleotides (i.e. nucleotides resistant to nuclease (e.g. exonuclease) digestion).

The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 5' of the sense strand of the cassette. The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 5' of the antisense strand of the cassette. The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 3' of the sense strand of the cassette. The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 3' of the antisense strand of the cassette.

The partially closed linear DNA molecule may comprise (i) a cassette, wherein the cassette comprises a sense strand and an antisense strand; (ii) one or more nuclease-resistant nucleotides in an open-end region of the partially closed linear DNA molecule, wherein the open-end region is 5' of the sense strand of the cassette; and (iii) one or more nuclease-resistant nucleotides in an open-end region of the partially closed linear DNA molecule, wherein the open-end region is 3' of the antisense strand of the cassette.

The partially closed linear DNA molecule may comprise (i) a cassette, wherein the cassette comprises a sense strand and an antisense strand; (ii) one or more nuclease-resistant nucleotides in an open-end region of the partially closed linear DNA molecule, wherein the open-end region is 3' of the sense strand of the cassette; and (iii) one or more nuclease-resistant nucleotides in an open-end region of the partially closed linear DNA molecule, wherein the open-end region is 5' of the antisense strand of the cassette.

The closed linear DNA molecule has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open ended cassettes have been demonstrated to be inefficient with respect to gene expression when introduced into host tissue. This has been attributed to cassette instability due to the action of exonucleases in the extracellular space.

As shown in Figures 8, and 10 and Example 5, the nanoparticles described herein are very stable over prolonged periods. The nanoparticle may show no variation or very small variation in the PDI value and/or size of the nanoparticle for at least 20 weeks, at least 30 weeks or at least 40 weeks when stored under 4°C. The nanoparticle may show no variation or very small variation in the transfection efficiency for at least 20 weeks, at least 30 weeks or at least 40 weeks when stored under 4°C.

Sequestering DNA ends inside closed structures also has other advantages. The DNA ends are prevented from integrating with genomic DNA and so closed linear DNA molecules offer improved safety. In addition, the closed linear structure reduces concatamerisation of DNA molecules inside host cells and thus expression levels of the gene product can be regulated in a more sensitive manner.

The linear DNA molecule (e.g. the linear double-stranded DNA molecule, the closed linear DNA or the partially closed linear DNA molecule) may comprise a cassette. Thus, the linear DNA molecule (e.g. the linear double-stranded DNA molecule, the closed linear DNA or the partially closed linear DNA molecule) may comprise a sense strand and an antisense strand, wherein the linear DNA molecule comprises a single cassette and one or more protected (e.g. phosphorothioated) nucleotides at internal positions in each strand (optionally wherein the linear DNA molecule comprises one or more protected (e.g. phosphorothioated) nucleotides at internal positions in each strand outside of the cassette).

The term "single cassette" as used herein in the context of a linear DNA molecule is intended to encompass a linear DNA molecule that does not comprise or consist of a plurality of cassettes. That is to say that the linear DNA molecule comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The linear DNA molecule (e.g. the linear double-stranded DNA molecule, the closed linear DNA or the partially closed linear DNA molecule) may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The

cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, polyC, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded linear DNA molecule may comprise a spacer region. The spacer region may comprise at least 10, at least 20, at least 30, at least 40, at least 50, at least 60, at least 70, at least 80, at least 90, at least 100, at least 125, at least 150, at least 175, or at least 200 base pairs.

The closed linear DNA molecule may comprise a spacer region. The spacer region may be at least 10, at least 20, at least 30, at least 40, at least 50, at least 60, at least 70, at least 80, at least 90, at least 100, at least 125, at least 150, at least 175, or at least 200 base pairs.

The partially closed linear DNA molecule may comprise a spacer region. The spacer region may comprise at least 10, at least 20, at least 30, at least 40, at least 50, at least 60, at least 70, at least 80, at least 90, at least 100, at least 125, at least 150, at least 175, or at least 200 base pairs.

The double-stranded linear DNA molecule may comprise an inverted terminal repeat sequence.

The closed linear DNA molecule may comprise an inverted terminal repeat sequence.

The partially closed linear DNA molecule may comprise an inverted terminal repeat sequence.

The double-stranded linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the double-stranded linear DNA molecule comprises at least 500 base pairs.

The closed linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the closed linear DNA molecule comprises at least 500 base pairs.

The partially closed linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the partially closed linear DNA molecule comprises at least 500 base pairs.

The closed linear DNA molecule may comprise one or more protected nucleotides (i.e. nucleotides resistant to nuclease (e.g. exonuclease) digestion). The closed linear DNA molecule may comprise one or more of protected nucleotides (i.e. nucleotides resistant to nuclease (e.g. exonuclease) in each strand. For example, the closed linear DNA molecule may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) in each strand.

The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The protected nucleotides may be located at the 3’ and/or 5’ end and/or 3’ and/or 5’ end region of the linear double-stranded DNA molecule. Preferably, the protected nucleotides are located at the 3’ and 5’ end and at the 3’ and 5’ region of the linear double-stranded DNA molecule. The 3’ region comprises at least 20 3’-end nucleotides of the linear DNA molecule. The 3’ region comprises less than 30 3’-end nucleotides of the linear DNA molecule. The 5’ region comprises no more than 30 5’-end nucleotides of the linear DNA molecule. That is to say that the nucleotides which are the last and/or the first nucleotides in the linear DNA molecule may be protected

nucleotides. In other words, the linear DNA molecule may comprise a "cap" of protected nucleotides, which protects the ends of the linear DNA molecule from nuclease (e.g. exonuclease) digestion.

The partially closed linear DNA molecule may comprise one or more protected nucleotides (i.e. nucleotides resistant to nuclease (e.g. exonuclease) digestion). The partially closed linear DNA molecule may comprise one or more of protected nucleotides (i.e. nucleotides resistant to nuclease (e.g. exonuclease) in each strand. For example, the partially closed linear DNA molecule may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) in each strand.

The protected nucleotides (i.e. nucleotides resistant to nuclease (e.g. exonuclease) digestion) may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The protected nucleotides (i.e. nucleotides resistant to nuclease (e.g. exonuclease) digestion) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The linear double-stranded DNA molecule may comprise one or more protected nucleotides (e.g. phosphorothioated nucleotides) at internal positions in each strand (i.e. the sense and antisense strands).

The internal positions may be any positions in the linear double-stranded DNA molecule other than the last nucleotide on the 3'-end and the 5'-end of the sense and antisense strands. The internal positions may be located at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule. The internal positions may be located at least 7 nucleotides away from the 3'-end and/or the 5'-end of each strand of the linear double-stranded DNA molecule. Preferably, the internal positions are located at least 10 nucleotides away from the 3'-end and/or the 5'-end of each strand of the linear double-stranded DNA molecule. In the linear double-stranded DNA molecule, the internal positions may be located at least 7 nucleotides away from the 3'-end and the 5'-end of the sense strand. Preferably, in the linear double-stranded DNA molecule, the internal positions are located at least 10 nucleotides away from the 3'-end and the 5'-end of the sense strand. In the linear double-stranded DNA molecule, the internal positions may be located at least 7 nucleotides away from the 3'-end and the 5'-end of the antisense strand. Preferably, in the linear double-stranded DNA molecule, the internal positions may be located at least 10 nucleotides away from the 3'-end and the 5'-end of the antisense strand. In the linear double-stranded DNA molecule, the internal positions may be located at least 7 nucleotides away from the 3'-end of each strand. Preferably, in the linear double-stranded DNA molecule, the internal positions may be located at least 10 nucleotides away from the 3'-end of each strand. In the linear double-stranded DNA molecule, the internal positions may be located at least 7 nucleotides away from the 5'-end of each strand. Preferably, in the linear double-stranded DNA molecule, the internal positions may be located at least 10 nucleotides away from the 5'-end of each strand.

The linear double-stranded DNA molecule may comprise a cassette. The location of the protected (e.g. phosphorothioated) nucleotides in the linear double-stranded DNA molecule may be such that the cassette is protected from nuclease (e.g. exonuclease III) digestion. Thus, the one or more phosphorothioated nucleotides at the internal positions in each strand may be selected from:

(a) a 5'-end nucleotide of the cassette;
(b) a 3'-end nucleotide of the cassette; and
(c) one or more nucleotides outside of the cassette

The term "*5'-end nucleotide of the cassette*" as used herein is intended to encompass the 5'-end nucleotide of each strand of the cassette. Thus, in the linear double-stranded DNA molecule, the cassette typically comprises a 5'-end nucleotide of the sense strand and a 5'-end nucleotide of the antisense strand.

The term "*3'-end nucleotide of the cassette*" as used herein is intended to encompass the 3'-end nucleotide of each strand of the cassette. Thus, in the linear double-stranded DNA molecule, the cassette typically comprises a 3'-end nucleotide of the sense strand and a 3'-end nucleotide of the antisense strand.

The term "*outside of the cassette*" as used herein is intended to encompass any nucleotides which are not part of the cassette. This includes any nucleotides that are comprised in the linear double-stranded DNA molecule and which also do not form part of the cassette. The term "*N nucleotides outside of the cassette*" or "*N nucleotides away from the cassette*" is intended to describe nucleotides that are located N nucleotides from the end of the cassette towards the end of the linear double-stranded DNA molecule. For example, the term "*2 nucleotides outside of the cassette*" in the context of internal positions of nucleotides is meant to describe a nucleotide that is outside of the cassette and that is

two nucleotides away from the last nucleotide of the cassette. For example, in the sequence: 5'-AAAAAACATAAAA (SEQ ID NO: 3), wherein the cassette starts from nucleotide "T" (in the 5' to 3' direction), the term "*2 nucleotides outside of the cassette*" refers to the "C" nucleotide. Thus, the term "*at least 2 nucleotides outside of the cassette*" or *"at least 2 nucleotides away from the cassette"* is meant to describe nucleotides that are outside of the cassette and that are at least two nucleotides away from the last nucleotide of the cassette. In the example above, nucleotides "*at least 2 nucleotides away from the cassette*" would be any nucleotides selected from 5'-AAAAAAC. Similarly, the term "*at least 2 nucleotides away from the 5'-end of the cassette*" is meant to describe nucleotides that are outside of the cassette and that are at least two nucleotides away from the last nucleotide at the 5'-end of the cassette. In the example above, the last nucleotide at the 5'-end of the cassette is "T", and nucleotides "*at least 2 nucleotides away from the 5'-end of the cassette*" would be any nucleotides selected from 5'-AAAAAAC.

The internal positions may not be located between the second and penultimate nucleotide of the cassette. The internal positions may be any position in the linear double-stranded DNA molecule other than the last nucleotide on the 3'-end and the 5'-end of the sense and antisense strands and other than nucleotides located between the second and penultimate nucleotide of the cassette. The internal positions may be located outside of the cassette and at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule and/or at least 2, 3, 4, 5, 6, 7, 8, 9, 10, 12, 14, 16, 18, 20, 30, 40, 50, 75, or 100 nucleotides away (i.e. outside of the cassette) from the 3'-end and/or the 5'-end of each strand of the cassette. Preferably, the internal positions is located outside of the cassette and at least 6, at least 8, or at least 10 nucleotides away from the 3'-end and/or the 5'-end of each strand of the linear double-stranded DNA molecule and/or at least 6, at least 8, or at least 10 nucleotides away from the 3'-end and/or the 5'-end of each strand of the cassette (i.e. at least 6, at least 8, or at least 10 nucleotides outside of the cassette). Preferably, the cassette does not comprise a phosphorothioated nucleotide at either one of the positions 1-2, 1-3, 1-4, 1-5, 1-6, 1-7, 1-8, 1-9, 1-10, 1-12, 1-14, 1-16, 1-18, or 1-20 of the sense and/or the antisense strand away from the 3'-end and/or the 5'-end of the cassette (i.e. outside of the cassette). Preferably, the internal positions in each strand are located at least 6 nucleotides away from the end of the linear double-stranded DNA molecule and are not located between the second and penultimate nucleotide of the cassette. Preferably, the internal positions in each strand are located at least 10 nucleotides away from the end of the linear double-stranded DNA molecule and are not located between the second and penultimate nucleotide of the cassette. Preferably, the internal positions in each strand are located at least 6 nucleotides away from the end of the linear double-stranded DNA molecule and at least 6 nucleotides away from the end of the cassette (i.e. at least 6 nucleotides outside of the cassette). Preferably, the internal positions in each strand are located at least 10 nucleotides away from the end of the linear double-stranded DNA molecule and at least 10 nucleotides away from the end of the cassette (i.e. at least 10 nucleotides outside of the cassette). The linear double-stranded DNA molecule may comprise a first phosphorothioated nucleotide at an internal position which is a position other than the last nucleotide on the 3'-end and the 5'-end of the sense and antisense strands as long as this position is located at least 2, 3, 4, 5, 6, 7, 8, 9, 10, 12, 14, 16, 18, 20, 30, 40, or 50 nucleotides outside of the cassette. For example, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are not located between the second and penultimate nucleotide of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located outside of the cassette. The linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 2, 3, 4, 5, 6, 7, 8, 9, 10, 12, 14, 16, 18, 20, 30, 40, or 50 nucleotides outside of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 6, 8, or 10 nucleotides outside of the cassette.

The linear double-stranded DNA molecule may comprise at least 2, 3, 4, 5, 6, 7, 8, 9 or 10 protected (e.g. phosphorothioated) nucleotides at internal positions in each strand. Preferably, the linear double-stranded DNA molecule comprises at least 2 protected (e.g. phosphorothioated) nucleotides at internal positions in each strand.

The location of phosphorothioated nucleotides in the sense strand of the linear double-stranded DNA molecule may be such that the one or more phosphorothioated nucleotides are located upstream of the cassette (i.e. towards the 5'-end of the sense strand of the DNA molecule).

In the sense strand of the linear double-stranded DNA molecule:

(a) one of the at least two phosphorothioated nucleotides may be the 5'-end nucleotide of the cassette; and/or
(b) one of the at least two phosphorothioated nucleotides may be in a first region of the sense strand, wherein the first region of the sense strand is 5' of the cassette.

The location of phosphorothioated nucleotides in the sense strand of the linear double-stranded DNA molecule may be such that the one or more phosphorothioated nucleotides are located downstream of the cassette (i.e. towards the 3'-end of the sense strand of the DNA molecule).

In the sense strand of the linear double-stranded DNA molecule:

(a) one of the at least two phosphorothioated nucleotides may be the 3'-end nucleotide of the cassette; and/or
(b) one of the at least two phosphorothioated nucleotides may be in a second region of the sense strand, wherein the second region of the sense strand is 3' of the cassette.

The location of phosphorothioated nucleotides in the antisense strand of the linear double-stranded DNA molecule may be such that the one or more phosphorothioated nucleotides are located upstream of the cassette (i.e. towards the 5'-end of the antisense strand of the DNA molecule).

In the antisense strand of the linear double-stranded DNA molecule:

(a) one of the at least two phosphorothioated nucleotides may be the 5'-end nucleotide of the cassette; and/or
(b) one of the at least two phosphorothioated nucleotides may be in a first region of the antisense strand, wherein the first region of the antisense strand is 5' of the cassette.

The location of phosphorothioated nucleotides in the antisense strand of the linear double-stranded DNA molecule may be such that the one or more phosphorothioated nucleotides are located downstream of the cassette (i.e. towards the 3'-end of the antisense strand of the DNA molecule).

In the antisense strand of the linear double-stranded DNA molecule:

(a) one of the at least two phosphorothioated nucleotides may be the 3'-end nucleotide of the cassette; and/or
(b) one of the at least two phosphorothioated nucleotides may be in a second region of the antisense strand, wherein the second region of the antisense strand is 3' of the cassette.

The location of phosphorothioated nucleotides in both the sense and the antisense strand of the linear double-stranded DNA molecule may be such that at least one phosphorothioated nucleotide is located downstream of the cassette and at least one phosphorothioated nucleotide is located upstream of the cassette in each strand.

In the linear double-stranded DNA molecule:

(a) in the sense strand one of the at least two phosphorothioated nucleotides may be the 5'-end nucleotide of the cassette, and/or one of the at least two phosphorothioated nucleotides may be in a first region of the sense strand, wherein the first region of the sense strand is 5' of the cassette;
(b) in the sense strand one of the at least two phosphorothioated nucleotides may be the 3'-end nucleotide of the cassette, and/or one of the at least two phosphorothioated nucleotides may be in a second region of the sense strand, wherein the second region of the sense strand is 3' of the cassette;
(c) in the antisense strand one of the at least two phosphorothioated nucleotides may be the 5'-end nucleotide of the cassette, and/or one of the at least two phosphorothioated nucleotides may be in a first region of the antisense strand, wherein the first region of the antisense strand is 5' of the cassette; and
(d) in the antisense strand one of the at least two phosphorothioated nucleotides may be the 3'-end nucleotide of the cassette, and/or one of the at least two phosphorothioated nucleotides may be in a second region of the antisense strand, wherein the second region of the antisense strand is 3' of the cassette.

The term "*first region of the sense strand*" as used herein is intended to encompass the part of the sense strand of the linear double-stranded DNA molecule that is between the 5'-end of the linear double-stranded DNA molecule and the first 5' nucleotide of the cassette in the sense strand. For example, in the sequence of the sense strand: 5'-AAAAAACATAAAA-3' (SEQ ID NO: 3), wherein the cassette starts from nucleotide "T" in the 5'-3' direction, the term "*first region of the sense strand*" refers to the 5'-AAAAAACA-3' region.

The term "*first region of the antisense strand*" is intended to encompass the part of the antisense strand of the linear double-stranded DNA molecule that is between the 5'-end of the linear double-stranded DNA molecule and the first 5' nucleotide of the cassette in the antisense strand. For example, in the sequence of the antisense strand: 5'-AAAAAACATAAAA-3' (SEQ ID NO: 3), wherein the cassette starts from nucleotide "T" in the 5'-3' direction, the term "*first region of the antisense strand*" refers to the 5'-AAAAAACA-3' region.

The term "*second region of the sense strand*" is intended to encompass the part of the sense strand of the linear double-stranded DNA molecule that is between the 3'-end of the linear double-stranded DNA molecule and the first 3' nucleotide of the cassette in the sense strand. For example, in the sequence of the sense strand: 5'-AAAAAACATAAAA-3' (SEQ ID NO: 3), wherein the cassette starts from nucleotide "T" in the 3'-5' direction, the term "*second region of the sense strand*" refers to the 5'-AAAA-3' region.

The term "*second region of the antisense strand*" is intended to encompass the part of the antisense strand of the linear double-stranded DNA molecule that is between the 3'-end of the linear double-stranded DNA molecule and the first 3' nucleotide of the cassette in the antisense strand. For example, in the sequence of the antisense strand: 5'-AAAAAACATAAAA-3' (SEQ ID NO: 3), wherein the cassette starts from nucleotide "T" in the 3'-5' direction, the term "*second region of the antisense strand*" refers to the 5'-AAAA-3' region.

The linear double-stranded DNA molecule may comprise a plurality of the phosphorothioated nucleotides upstream (i.e. towards the 5'-end of the sense strand of the DNA molecule) of the cassette. For example, the linear double-stranded DNA molecule may comprise at least 2, 3, 4, 5, 6, 7, 8, 9 or 10 phosphorothioated nucleotides upstream of the cassette. Preferably, at least 2 phosphorothioated nucleotides upstream of the cassette. Thus, the location of phosphorothioated nucleotides in the sense strand of the linear double-stranded DNA molecule may be such that at least two phosphorothioated nucleotides are located upstream of the cassette.

In the sense strand of the linear double-stranded DNA molecule:

(a) one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or

(b) the at least two phosphorothioated nucleotides may be in a first region of the sense strand, wherein the first region of the sense strand is 5' of the cassette.

The linear double-stranded DNA molecule may comprise a plurality of the phosphorothioated nucleotides downstream (i.e. towards the 3-end of the DNA molecule) of the cassette. For example, the linear double-stranded DNA molecule may comprise at least 2, 3, 4, 5, 6, 7, 8, 9 or 10 phosphorothioated nucleotides downstream of the cassette, preferably at least 2 phosphorothioated nucleotides downstream of the cassette. Thus, the location of phosphorothioated nucleotides in the sense strand of the linear double-stranded DNA molecule may be such that at least two phosphorothioated nucleotides are located downstream of the cassette.

In the sense strand of the linear double-stranded DNA molecule:

(a) one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or

(b) the at least two phosphorothioated nucleotides may be in a first region of the sense strand, wherein the first region of the sense strand is 3' of the cassette.

The location of phosphorothioated nucleotides in the antisense strand of the linear double-stranded DNA molecule may be such that at least two phosphorothioated nucleotides are located upstream of the cassette (i.e. towards the 5'-end of the antisense strand of the DNA molecule).

In the antisense strand of the linear double-stranded DNA molecule:

(a) one of the at least two phosphorothioated nucleotides is the 5'-end nucleotide of the cassette and one of the at least two phosphorothioated nucleotides is in a first region of the antisense strand, wherein the first region of the antisense strand is 5' of the cassette; or

(b) the at least two phosphorothioated nucleotides are in a first region of the antisense strand, wherein the first region of the antisense strand is 5' of the cassette.

The location of phosphorothioated nucleotides in the antisense strand of the linear double-stranded DNA molecule may be such that at least two phosphorothioated nucleotides are located downstream of the cassette (i.e. towards the 3'-end of the antisense strand of the DNA molecule).

In the antisense strand of the linear double-stranded DNA molecule:

(a) one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or

(b) the least two phosphorothioated nucleotides may be in a second region of the antisense strand, wherein the second region of the antisense strand is 3' of the cassette.

Each strand of the linear double-stranded DNA molecule may comprise a plurality of phosphorothioated nucleotides. For example, the linear double-stranded DNA molecule may comprise at least 2, 3, 4, 5, 6, 7, 8, 9 or 10 phosphorothioated nucleotides upstream and downstream of the cassette. The location of phosphorothioated nucleotides in both the sense and the antisense strand of the linear double-stranded DNA molecule may be such that the at least two phosphorothioated nucleotides are located downstream of the cassette and at least two phosphorothioated nucleotides are located upstream of the cassette in each strand.

In the linear double-stranded DNA molecule:

(a) in the sense strand:

i. one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or

ii. the at least two phosphorothioated nucleotides may be in a first region of the sense strand, wherein the first region of the sense strand is 5' of the cassette;

(b) in the sense strand:

i. one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or

ii. the at least two phosphorothioated nucleotides may be in a second region of the sense strand, wherein the second region of the sense strand is 3' of the cassette;

(c) in the antisense strand:

i. one of the at least two phosphorothioated nucleotides may be the 5'-end nucleotide of the cassette and one of the at least two phosphorothioated nucleotides may be in a first region of the antisense strand, wherein the first region of the antisense strand is 5' of the cassette; or

ii. the at least two phosphorothioated nucleotides may be in a first region of the antisense strand, wherein the first region of the antisense strand is 5' of the cassette; and

(d) in the antisense strand:

i. one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or

ii. the at least two phosphorothioated nucleotides may be in a second region of the antisense strand, wherein the second region of the antisense strand is 3' of the cassette.

The amount of phosphorothioated nucleotides at internal positions in each strand may depend on the length of the linear double-stranded DNA product. Thus, the linear double-stranded DNA product may comprise phosphorothioated nucleotides at a ratio of the phosphorothioated nucleotides to total nucleotides that is at least 0.0001, at least 0.0025, at least 0.01 at least 0.025, at least 0.05, at least 0.075, at least 0.10, at least 0.12 at least 0.15, at least 0.25, at least 0.35, at least 0.5, or at least 0.75. The linear double-stranded DNA product may comprise phosphorothioated nucleotides at a ratio of the phosphorothioated nucleotides to total nucleotides that is less than 1, less than 0.9, less than, 0.8, less than 0.65, less than 0.5, less than 0.4, less than 0.3, less than 0.2, less than 0.1, less than 0.075, or less than 0.05. The linear double-stranded DNA product may comprise phosphorothioated nucleotides at a ratio of the phosphorothioated nucleotides to total nucleotides that is between 0.0001-1, between 0.0025-0.75, between 0.025-0.65, between 0.025-0.15, or between 0.25-0.50. Preferably, a ratio of the phosphorothioated nucleotides to total nucleotides is between 0.025-0.15. The linear double-stranded DNA product may comprise phosphorothioated nucleotides at a ratio of the phosphorothioated nucleotides to total nucleotides that is between 0.005-0.3, between 0.0075-0.2, between 0.01-0.15, between 0.01-0.10, between 0.02-0.08, between 0.03-0.07, between 0.04-0.06 or between 0.05-0.075. Preferably, a ratio of the phosphorothioated nucleotides to total nucleotides is between 0.01-0.10. Preferably, a ratio of the phosphorothioated nucleotides to total nucleotides is about 0.02, about 0.025, about 0.05, about 0.075, about 0.08, about 0.10, about 0.12, about 0.15, or about 0.25. More preferably still, a ratio of the phosphorothioated nucleotides to total nucleotides is about 0.025 or about. 0.05. As shown in the Examples, a ratio of the phosphorothioated nucleotides to total nucleotides of about 0.025 is sufficient to provide enhanced resistance to exonuclease digestion. As shown in the Examples, a ratio of the phosphorothioated nucleotides to total nucleotides of about 0.05 or about 0.075 is particularly

effective in providing enhanced resistance to exonuclease digestion (as shown by levels of expression; see Examples 7 and 8). Moreover, as shown in the Examples, the ratio of about 0.05 is particularly suitable for in vivo expression of the gene of interest (see Examples 10).

The amount of phosphorothioated nucleotides at internal positions in each strand can be different. For example, if the linear double-stranded DNA product comprises the total of 1000 nucleotides and the ratio of the phosphorothioated nucleotides to total nucleotides is 0.1, the linear double-stranded DNA product comprises 100 phosphorothioated nucleotides, out of which 75 might be located in the sense strand and 25 in the antisense strand of the DNA product. For example, 100% of the phosphorothioated nucleotides in the linear double-stranded DNA product may be located in the sense strand. Alternatively, 100% of the phosphorothioated nucleotides in the linear double-stranded DNA product may be located in the antisense strand. The sense strand may comprise at least 90%, 80%, 70%, 60%, 50%, 40%, 30%, 20%, or 10% of the total number of the phosphorothioated nucleotides in the linear double-stranded DNA product. The antisense strand may comprise at least 90%, 80%, 70%, 60%, 50%, 40%, 30%, 20%, or 10% of the total number of the phosphorothioated nucleotides in the linear double-stranded DNA product. The sense and antisense strands may comprise an equal number of the phosphorothioated nucleotides. Preferably, each of the sense and antisense strands comprises 50% of the total number of the phosphorothioated nucleotides in the linear double-stranded DNA product.

The linear double-stranded DNA molecule or the closed linear DNA molecule (e.g. the covalently closed linear DNA molecule) may be resistant to nuclease digestion or may have improved or enhanced resistance to nuclease digestion. The linear double-stranded DNA molecule or the closed linear DNA molecule may be resistant to exonuclease digestion or have improved or enhanced resistance to exonuclease digestion. The linear double-stranded DNA molecule or the closed linear DNA molecule may be resistant, or have improved or enhanced resistance, to digestion by exonucleases that cleave the 3'-end nucleotides (e.g. exonuclease III) and/or exonucleases that cleave the 5'-end nucleotides (e.g. exonuclease VIII). The terms "improved" or "enhanced" in the context of resistance to enzyme digestion refer to higher resistance to enzyme digestion when compared to a DNA molecule not produced by the methods described herein. For example, for the linear double-stranded DNA molecule, when compared to a molecule that does not comprise protected nucleotides, such as phosphorothioated nucleotides.

The linear DNA molecule (e.g. the linear double-stranded DNA molecule) or the closed linear DNA molecule (e.g. the covalently closed linear DNA molecule) may comprise a functional portion.

The functional portion may be at the 3' and/or the 5' end (or end region) of the polynucleotide chain. The functional portion may be attached or bound to the 5' overhang, the 3' overhang and/or the blunt end of the linear DNA molecule (e.g. the linear double-stranded DNA molecule) or the closed linear DNA molecule. The functional portion may be attached or bound to the 5' overhang, the 3' overhang and/or the blunt end of linear DNA molecule (e.g. the linear double-stranded DNA molecule) or the closed linear DNA molecule by covalent linkage or non-covalent linkage, or by nucleic acid hybridisation. The functional portion may be attached or bound to linear DNA molecule (e.g. the linear double-stranded DNA molecule) or the closed linear DNA molecule directly or indirectly (e.g. via a linker molecule). The functional portion may be attached or bound by being bound to the linear DNA molecule (e.g. the linear double-stranded DNA molecule) or the closed linear DNA molecule and/or by being bound or annealed to linker molecules that are bound to the linear DNA molecule (e.g. the linear double-stranded DNA molecule) or the closed linear DNA molecule. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol).

The DNA molecule may comprise two functional portions; a first functional portion at the 3'-end of the polynucleotide chain, and a second functional portion at the 5'-end of the polypeptide chain. The first functional portion may be attached or bound to the 5' overhang, the 3' overhang and/or the blunt end of the 3'-end of the DNA molecule. The second functional portion may be attached or bound to the 5' overhang, the 3' overhang and/or the blunt end of the 5'-end of the DNA molecule. The two functional portions may be the same or different. For example, the first functional portion may be a barcode to facilitate detection and/or sequencing of the DNA molecule, the second functional portion may be a nuclear targeting sequence.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the DNA product described herein and/or that is capable of binding to a further molecule or target. The binding molecule may be a protein, a polypeptide, or a peptide. The binding molecule may be an antibody, such as a

monoclonal antibody or a polyclonal antibody. The binding molecule may be an antibody fragment.

The functional portion may facilitate detection of the DNA molecule by binding to capture molecules (e.g. capture antibodies bound by protein-protein interactions). The functional portion may bind to a cell target, for example, a cell receptor.

The functional portion may be a label. Thus, the sense strand of the DNA molecule may comprise a label at the 5'-end or the 3'-end for detection. Alternatively or additionally, the antisense strand of the DNA molecule may comprise a label at the 5'-end or the 3'-end for detection. The 'label' can be any chemical entity which enable the detection of the double-stranded nucleic acid molecule via, physical, chemical and/or biological means. The label may be a chromophore, a fluorophore and/or a radioactive molecule.

To facilitate detection and/or quantification of the DNA molecule, the functional portion may comprise a fluorophore, a radioactive compound or a barcode.

A signal corresponding to the presence, absence and/or level of the DNA molecule may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the 3' overhang, the 5' overhang or the blunt end of the DNA molecule. The binding moiety is capable of binding to 3' and/or 5' end of the DNA molecule. The signal may be measured by determining the presence, absence and/or level of the barcoded portion of the barcode (e.g. by sequencing or PCR). The barcoded portion may comprise at least 2, 3, 4, 5, 6, 7, 8, 9, or 10 nucleotides. The barcode may comprise at least 2 binding moieties (e.g. a first binding moiety and a second binding moiety). For example, the first binding moiety linked to the first barcoded portion may bind to the 3' end of the DNA molecule and the second binding moiety linked to the second barcoded portion may bind to the 5' end of the DNA molecule. The 3' and 5' ends may comprise a 3' overhang, a 5' overhang or a blunt end.

A signal corresponding to the presence, absence and/or level of the DNA molecule may be measured using a fluorophore (i.e. a fluorescently-labelled molecule), which is attached or bound to the 3' overhang, the 5' overhang or the blunt end of the linear molecule. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina® (e.g. the HiSeq™, MiSeq™ and/or Genome Analyzer™ sequencing systems), Oxford Nanopore™ Technologies (e.g. the MinION sequencing system), Ion Torrent™ (e.g. the Ion PGM™ and/or Ion Proton™ sequencing systems), Pacific Biosciences (e.g. the PACBIO RS II sequencing system); Life Technologies™ (e.g. a SOLiD sequencing system), Roche (e.g. the 454 GS FLX+ and/or GS Junior sequencing systems), or any other sequencing platform of interest.

### 1. Pharmaceutical compositions

The invention provides a pharmaceutical composition comprising a nanoparticle (e.g. a non-viral transfection complex) described herein. The invention provides a pharmaceutical composition comprising a nanoparticle (e.g. a non-viral transfection complex) described herein and a pharmaceutically suitable carrier or excipient.

The invention provides a pharmaceutical composition comprising a nanoparticle (e.g. a non-viral transfection complex), which comprises: a nanoparticle comprising:

(a) a cargo;
(b) a lipid component, wherein the lipid component is one or more ionizable lipids and/or one or more cationic lipids;
(c) a phospholipid;
(d) a steroid lipid; and
(e) a cationic polymer.

The invention provides a pharmaceutical composition comprising:

i. a nanoparticle (e.g. a non-viral transfection complex), which comprises:

(a) a cargo;
(b) a lipid component, wherein the lipid component is one or more ionizable lipids and/or one or more cationic lipids;
(c) a phospholipid;
(d) a steroid lipid; and

(e) a cationic polymer; and

ii. a pharmaceutically suitable carrier.

The pharmaceutical composition may comprise:

i. a nanoparticle (e.g. a non-viral transfection complex), of any one of embodiments 1 to 215; and
ii. a pharmaceutically suitable carrier.

The pharmaceutical composition may comprise:

i. a nanoparticle (e.g. the non-viral transfection complex), which comprises:

(a) a closed linear DNA molecule;
(b) a lipid component, wherein the lipid component is one or more ionizable lipids and/or one or more cationic lipids;
(c) a phospholipid;
(d) a steroid lipid; and
(e) a cationic polymer.; and

ii. a pharmaceutically suitable carrier.

The pharmaceutical composition may comprise:

a nanoparticle (e.g. the non-viral transfection complex), which comprises:

(a) a linear DNA molecule comprising one or more nuclease-resistant nucleotides (e.g. phosphorothioated nucleotides;
(b) a lipid component, wherein the lipid component is one or more ionizable lipids and/or one or more cationic lipids;
(c) a phospholipid;
(d) a steroid lipid; and
(e) a cationic polymer.; and

a pharmaceutically suitable carrier.

The pharmaceutical composition may comprise:

a nanoparticle (e.g. the non-viral transfection complex), which comprises:

(a) a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides (e.g. phosphorothioated nucleotides) in an open end region adjacent to the second end;
(b) a lipid component, wherein the lipid component is one or more ionizable lipids and/or one or more cationic lipids;
(c) a phospholipid;
(d) a steroid lipid; and
(e) a cationic polymer.; and

a pharmaceutically suitable carrier.

The pharmaceutical composition may comprise:

a nanoparticle (e.g. the non-viral transfection complex), which comprises:

(a) an mRNA molecule;
(b) a lipid component, wherein the lipid component is one or more ionizable lipids and/or one or more cationic

lipids;
(c) a phospholipid;
(d) a steroid lipid; and
(e) a cationic polymer.; and

a pharmaceutically suitable carrier.

In all embodiments described herein, the presence of a pharmaceutically suitable carrier is optional. The nanoparticle of the invention may function as a pharmaceutically suitable carrier.

In all embodiments described herein the lipid component may be one or more of DLin-MC3-DMA, DLin-KC2-DMA, DLin-DMA, TCL053, SM-102, ALC-0315, C12-200, DODMA, DODAP, Lipid A9, 9A1 P9, Lipid C24, Lipid LP01, Lipid 5 (ionizable lipids) , DOTMA, DTDTMA or DHDTMA (cationic lipids).

The phospholipid may comprise DOPE, DOPC, DSPC, DPPC, DMPC or POPC. Preferably the phospholipid is DOPE.

A steroid lipid may be one or more of cholesterol, or a derivative thereof (a cholesterol derivative), such as beta-sitosterol, fucosterol, campesterol, stigamstanol (alkyl steroids), secosteroids (vitamin D2, D3) or pentacyclic steroids.

The cationic polymer may comprise oligolysine (linear or branched) such as K16, K17 or K30, oligohistidine (linear or branched) or oligoarginine (linear or branched) or combination of oligolysine and oligohistidine, oligohistidine and oligoarginine, oligoarginine and oligolysine, or oligolysine, oligohistidine and oligoarginine, or PEI. Preferably, the cationic polymer is oligolysine comprising 16, 17 or 30 lysine residues (K16, K17 or K30).

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally, transdermally, intra-articularly, by IV or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

The pharmaceutical composition may be administered by injection (e.g. intravenous or intramuscular injection). The pharmaceutical composition may be formulated as an emulsion consisting of a sterile, pyrogen-free preparation or a lipid complex preparation.

For example, the pharmaceutical composition may be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

The pharmaceutical composition may be administered by intra-articular injection (i.e. into the joint). Such methods are routinely used in the art, for example, for the administration of hydrocortisone into the joint.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

In another mode of administration, the pharmaceutical composition can be administered in situ, via a catheter or pump. A catheter or pump can, for example, direct the composition into the selected tissue to receive the pharmaceutical composition.

The parenteral carrier system (e.g. intravenous carrier system or intramuscular carrier system) may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers

to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

## 2. Uses and applications

### General therapeutic and diagnostic uses

The invention provides the nanoparticle described herein for use in therapy.

The invention also provides the pharmaceutical composition described herein for use in therapy.

The nanoparticle may comprise a nucleic acid cargo described herein which encodes a sequence of a therapeutic protein, a part of a vaccine, or an element of a genetic engineering mechanism, which is used to treat a disease or infection in a subject.

The nanoparticle described herein, or the pharmaceutical composition described herein may be used in the treatment of a disease or disorder. The disease or disorder may be an illness or disease caused by a pathogen, or a cancer, or a viral infection (for example, coronavirus (e.g. COVID-19), herpes simplex virus type 2, HIV, influenza, and/or measles).

Preferably, the nanoparticle described herein, or the pharmaceutical composition described herein may be s used to treat a pathogen or cancer/tumour by way of a cancer antigen or spike protein, coat of a pathogen.

The nanoparticle described herein, or the pharmaceutical composition described herein may be used as a medicament. The invention provides the nanoparticle described herein, or the pharmaceutical composition described herein for use in the manufacture of a medicament for the prophylaxis of a condition caused in a subject by a pathogen or cancer by stimulating the immune response.

The invention provides a method for treatment of a disease or disorder caused in a subject by a pathogen or a cancer, the method comprising administering the nanoparticle described herein, or the pharmaceutical composition described herein to the subject. Preferably, the amount of the nanoparticle, or the pharmaceutical composition administered to the subject is a therapeutically active amount. Thus, the invention also provides the nanoparticle described herein, or the pharmaceutical composition described herein may be used in cancer therapy.

A subject treated with the nanoparticle described herein, or the pharmaceutical composition described herein may receive the nanoparticle described herein, or the pharmaceutical composition described herein with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the nanoparticle described herein, or the pharmaceutical composition described herein into the subject's body as described in more detail above (see "Pharmaceutical compositions"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the nanoparticle described herein, or the pharmaceutical composition described herein that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides use of the nanoparticle described herein, or the pharmaceutical composition described herein in a method of diagnosing a disease and/or a disorder.

The invention provides the use of the nanoparticle described herein, or the pharmaceutical composition described herein in the "in vitro" diagnosis of a disease.

The invention also provides the nanoparticle described herein, or the pharmaceutical composition described herein for use in a method of diagnosis "in vivo" of disease.

The nanoparticle described herein, or the pharmaceutical composition described herein may be used to diagnose a disease or disorder. The disease or disorder may be a viral infection (for example, coronavirus (e.g. COVID-19), herpes simplex virus type 2, HIV, influenza, and/or measles), or a cancer, for example, breast cancer, bowel cancer, ALL, AML, bone cancer, bladder cancer, cervical cancer, CLL, CML, gastric cancer, lung cancer, melanoma, ovarian cancer, pancreatic cancer, prostate cancer, renal cell cancer, skin cancer or testicular cancer).

The diagnostic and treatment methods described herein may be in vitro methods or in vivo methods.

The method of diagnosis may rely on the detection and/or quantification of the nanoparticle described herein, or the pharmaceutical composition described herein.

To facilitate detection and/or quantification of the nanoparticle described herein, the nanoparticle may be attached or bound to a functional portion. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the nanoparticle For example, the signal may be measured by flow cytometry and/or fluorescence-activated cell sorting of the nanoparticle attached to a fluorescent probe.

The nanoparticle may be detected by being bound to a capture moiety, for example in a lateral flow assay. In this example, the functional portion is a protein, for example, an antibody specific for the capture moiety. The capture of the antibody attached to the nanoparticle may produce a visual signal (e.g. a band of a different colour).

The invention also provides a method that combines diagnosis of a disease with a treatment of the disease.

Cell therapy

The nanoparticle described herein, or the pharmaceutical composition described herein may be used in cell therapy.

The invention provides the use of the nanoparticle described herein, or the pharmaceutical composition described herein in the production of cell therapy. The invention provides a method of cell therapy, comprising contacting the nanoparticle described herein, or the pharmaceutical composition described herein with a cell. Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

Vaccines

The products of the invention are particularly suitable for use in vaccine production. The nanoparticle described herein, the cationic polymer described herein, or the pharmaceutical composition described herein may be used to produce a vaccine, preferably an mRNA-based vaccine. For example, vaccines such as the BioNTech and Moderna mRNA vaccines against COVID-19.

The nanoparticle described herein or the pharmaceutical composition described herein may be used as a vaccine.

The nanoparticle described herein, the cationic polymer described herein, or the pharmaceutical composition described herein may be used in the therapeutic or prophylactic immunisation.

The nanoparticle described herein of the pharmaceutical composition described herein may be used in the therapeutic treatment of a cancer, such as a neoantigen vaccine.

CRISPR delivery

The nanoparticles and cationic polymers are particularly suitable for use in delivery of CRISPR machinery to cells, for example in in vivo therapy.

The nanoparticle may comprise a cargo, which is a nucleic acid cargo (e.g. the nucleic acid cargo described herein). The nucleic acid cargo may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease

of the CRISPR system (e.g. Cas9, Cpf1, or MAD7). The repair template (or editing template) may introduce at least one mutation (e.g. an insertion, deletion, and/or substitution) in the desired DNA region (i.e. target molecule). The repair template (or editing template) may comprise at least 10, at least 20, at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, 10000, at least 11000, at least 12000, at least 13000, at least 14000, or at least 15000 base pairs.

Thus, the invention provides the nanoparticle for use in CRISPR system delivery to a cell. The invention provides a method of delivering the CRISPR system to a cell, comprising contacting the nanoparticle with a cell.

The cell may be an animal cell, preferably mammal cell, such as human cell.

The invention also provides a cell obtainable by the methods described herein. The cell is particularly suitable for use in cell therapy and/or in vivo therapy.

Uses of the products described herein may be *in vivo* or *in vitro* uses.

## 3. Methods for producing nanoparticles

The invention provides a method for producing (or forming) a nanoparticle described herein, wherein the method comprises:

(a) contacting a lipid component, a phospholipid, a steroid lipid with a linear DNA molecule which is resistant to nuclease (e.g. exonuclease) digestion and a cationic polymer; and
(b) forming the nanoparticle.

The step of contacting a lipid component, a phospholipid, a steroid lipid with a linear DNA molecule and a cationic polymer may be performed in a single step or multiple step.

The invention provides a method for producing (or forming) a nanoparticle described herein, wherein the method comprises:

(a) contacting a lipid component, a phospholipid, a steroid lipid with a closed linear DNA molecule and a cationic polymer to form a single contiguous aqueous volume; and
(b) forming the nanoparticle.

The invention also provides a method for producing (or forming) a nanoparticle described herein, wherein the method comprises:

(a) contacting a lipid component, a phospholipid, a steroid lipid with a linear DNA molecule comprising one or more protected nucleotides and a cationic polymer to form a single contiguous aqueous volume; and
(b) forming the nanoparticle.

The invention also provides a method for producing (or forming) a nanoparticle described herein, wherein the method comprises:

(a) contacting a lipid component, a phospholipid, a steroid lipid with a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end; and a cationic polymer to form a single contiguous aqueous volume; and
(a) forming the nanoparticle.

The invention provides a method for producing (or forming) a nanoparticle described herein, wherein the method comprises:

(a) contacting a lipid component, a phospholipid, a steroid lipid with a closed linear DNA molecule and a cationic polymer; and
(b) forming the nanoparticle.

The invention also provides a method for producing (or forming) a nanoparticle described herein, wherein the method comprises:

(a) contacting a lipid component, a phospholipid, a steroid lipid with a linear DNA molecule comprising one or more protected nucleotides and a cationic polymer; and
(b) forming the nanoparticle.

The invention provides a method for producing (or forming) a nanoparticle described herein, wherein the method comprises:

(a) contacting a lipid component, a phospholipid, a steroid lipid with a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end and a cationic polymer; and
(b) forming the nanoparticle.

The lipid component (one or more ionizable and/or one or more cationic lipids) together with the phospholipid and the steroid lipid (and optionally a PEG lipid) may form a liposome.
The method may comprise the steps:

(a) contacting a liposome with a closed linear DNA molecule and a cationic polymer; and
(b) forming the nanoparticle.

The method may comprise the steps:

(a) contacting a liposome with a linear DNA molecule comprising one or more protected nucleotides and a cationic polymer; and
(b) forming the nanoparticle.

The method may comprise the steps:

(a) contacting a liposome with a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end; and a cationic polymer; and
(b) forming the nanoparticle.

The method may further comprise a step of forming a liposome. The step of forming a liposome may be performed before the step of contacting a liposome with a closed linear DNA molecule (or a linear DNA molecule comprising one or more protected nucleotides or a partially closed linear DNA molecule) and a cationic polymer.
The step of forming the nanoparticle may be performed by shaking, pipetting or using a microfluidics device. Preferably, the nanoparticle is formed using a microfluidics device. Using a microfluidics system may decrease the size of nanoparticles. For example, the size can be decreased from 118nm to 90nm as compared to nanoparticles formed using pipetting. In addition, using a microfluidics system may decrease the average PDI values. For example, the average PDI values may be decreased from 0.19 to 0.15 as compared to nanoparticles formed using pipetting. The smaller size of the nanoparticle and lower PDI value is preferred in the context of cell delivery. Thus, the invention provides a nanoparticle described herein obtainable using a microfluidics device.
The step of forming the nanoparticle may rely on self-assembly. That is to say that the nanoparticles of the present invention do not require conjugation of a cationic polymer to a lipid component before encapsulating the DNA or RNA cargo.
In aqueous solutions, lipids can self-assemble into liposomes. This is driven by hydrophilic interactions between polar headgroups, and van der Waals interactions between hydrocarbon chains. The lipids are organised so that the headgroups are exposed to the aqueous interface, while the hydrophobic tails are shielded from the aqueous milieu, forming a bilayer. Thus, the lipid component comprising an ionizable and/or a cationic lipid together with the phospholipid and the steroid lipid (and optionally a PEG lipid) may form a liposome.
The method may further comprise a step of diluting the nanoparticle to a desired nucleic acid concentration. The step of diluting the nanoparticle to a desired nucleic acid concentration after the step of forming the nanoparticle. For example, if the nucleic acid is a closed linear DNA molecule, the desired nucleic acid concentration may be 0.01 ng/µL - 10,000 ng/µL, 0.1 ng/µL - 1000 ng/µL or 1 ng/µL - 100 ng/µL.

### 4. Cell transfection

The invention provides a cell transfection composition comprising the nanoparticle described herein.

The invention provides a method for transfecting a cell comprising:

(a) contacting a cell with the nanoparticle described herein; and
(b) transfecting the nanoparticle to the cell.

The cell may be an animal cell, such as mammal cell (e.g. a human cell), a fungal cell, a cell of a micro-organism (e.g. a prokaryotic cell or a eukaryotic cell), or a plant cell. The cell may be a human cell. The cell may be a C2C12 cell or a HEK293 cell.

The cell transfection composition may or may not comprise an agent selected from a photosensitizing agent and/or a radical initiator e.g. a photoinitiator. Preferably, this agent improves the function of the cargo (e.g. the nucleic acid cargo described herein) at a target site and/or protects the cargo (e.g. the nucleic acid cargo described herein) from metabolism and/or degradation.

The invention further provides a cell obtainable by the methods of the invention. Thus, the cell may comprise the nanoparticle of the invention.

The invention further provides a cell transfected with the nanoparticle described herein.

The step of contacting the cell with the nanoparticle may be performed in vivo. For example, the nanoparticle may be administered to an organism (e.g. a subject) in need thereof. The organism (e.g. the subject) may be an animal, such as mammal (e.g. a human), a fungus, a micro-organism, or a plant.

### Brief Description of the Drawings

Figure 1A shows size and 1B shows PDI of hand mixed DOTMA, ALC-0315 or Dlin-MC3-DMA hpDNA lipid-peptide nanoparticles prepared with a lipid molar ratio of either nanoparticles denoted 102 1.5%( 59% cationic/ionizable lipid, 10% Cholesterol, 29.5% DOPE and 1.5% DMG-PEG) or denoted 406 1% (50.57% cationic/ionizable lipid, 40% Cholesterol, 8.43% DOPE and 1% DMG-PEG) at a hpDNA: lipid mass ratio of 1:11 or 1:15, with peptide K16 or K30, and at an N/P ratio of 6 (wherein hpDNA is closed linear DNA). hpDNA LNPs controls were prepared by microfluidic mixing at an N/P ratio of 6. *n* = 3. Error bars represent standard deviation.

Figure 2 shows biophysical size traces of representative hpDNA lipid-peptide nanoparticles prepared with peptide K30, at a hpDNA:mass ratio of 1:15 and an N/P ratio of 6 (A-C), or hpDNA LNPs (D-F). A) DOTMA 406 1%, B) ALC 406 1%, C) Dlin-MC3-DMA 102 1.5%, D) DOTMA LNP, E) ALC-0315 LNP, F) Dlin-MC3-DMA LNP. *n* = 3.

Figure 3 shows the rransfection efficiency in HEK293T cells of hand mixed DOTMA, ALC-0315 or Dlin-MC3-DMA hpDNA lipid-peptide nanoparticles prepared with a lipid molar ratio of either 102 1.5% or 406 1%, at a hpDNA: lipid mass ratio of 1:11 (A) or 1:15 (B), with peptide K16 or K30, and at an N/P ratio of 6. hpDNA LNPs controls were prepared by microfluidic mixing at an N/P ratio of 6. *n* = 3. Error bars represent standard deviation.

Figure 4A shows size and 4B shows PDI of DOTMA 406 1% (DOTMA), ALC-0315 406 1% (ALC) or Dlin-MC3-DMA 102 1.5% (MC3) hpDNA lipid-peptide nanoparticles prepared at a hpDNA: lipid mass ratio of 1:15, with peptide K16 and at an N/P ratio of 6. with either hand mixing or microfluidics methods. hpDNA LNPs controls were prepared by microfluidic mixing at an N/P ratio of 6. *n* = 3. Error bars represent standard deviation.

Figure 5 shows the transfection efficiency in HEK293T cells 48-hours post-transfection with DOTMA 406 1% (DOTMA), ALC-0315 406 1% (ALC) or Dlin-MC3-DMA 102 1.5% (MC3) hpDNA lipid-peptide nanoparticles prepared at a hpDNA: lipid mass ratio of 1:15, with peptide K16 and at an N/P ratio of 6. with either hand mixing or microfluidics methods. hpDNA LNPs controls were prepared by microfluidic mixing at an N/P ratio of 6. L2K positive control was prepared at a mass ratio of 1:3 hpDNA:L2K. *n* = 3. Error bars represent standard deviation.

Figure 6 shows fluorescent microscopy images of HEK293T cells 48-hours post transfection with DOTMA 406 1% (DOTMA), ALC-0315 406 1% (ALC) or Dlin-MC3-DMA 102 1.5% (MC3) hpDNA lipid-peptide nanoparticles prepared at a hpDNA: lipid mass ratio of 1:15, with peptide K16 and at an N/P ratio of 6. with either hand mixing or microfluidics methods. hpDNA LNPs controls were prepared by microfluidic mixing at an N/P ratio of 6. L2K positive control was prepared at a mass ratio of 1:3 hpDNA:L2K. *n* = 3. Error bars represent standard deviation.

Figure 7 shows the biophysical characteristics over 42 days of mRNA lipid-peptide nanoparticles stored at either +4°C (A) or -80°C (B) in 15% trehalose. Nanoparticles were prepared with DOTMA 406 1 % at an mRNA: lipid mass ratio of 1:15, with peptide K16 and at an N/P ratio of either 5 or 9 by microfluidics mixing. hpDNA LNPs controls were prepared by microfluidic mixing at an N/P ratio of 6. *n* = 3. Figure 8 shows biophysical traces of the formulations shown in Figure 7, at Day 1 and Day 42 at +4°C.

Figure 8 shows biophysical size traces at 1 and 42 days post-preparation of mRNA lipid-peptide nanoparticles stored at either +4°C in 15% trehalose. Nanoparticles were prepared with DOTMA 406 1% at an mRNA: lipid mass ratio of 1:15, with peptide K16 and at an N/P ratio of either 5 or 9 by microfluidics mixing. hpDNA LNPs controls were prepared by microfluidic mixing at an N/P ratio of 6. *n* = 3.

Figure 9 shows biophysical size traces at 1 and 42 days post-preparation of mRNA lipid-peptide nanoparticles stored at either -80°C in 15% trehalose. Nanoparticles were prepared with DOTMA 406 1% at an mRNA: lipid mass ratio of 1:15, with peptide K16 and at an N/P ratio of either 5 or 9 by microfluidics mixing. hpDNA LNPs controls were prepared by microfluidic mixing at an N/P ratio of 6. *n* = 3.

Figure 10 shows the transfection efficiency of mRNA lipid-peptide nanoparticles stored at either +4°C (A) or -80°C (B) in 15% trehalose at 5 time points over a 42 day period. Nanoparticles were prepared with DOTMA 406 1 % at an mRNA: lipid mass ratio of 1:15, with peptide K16 and at an N/P ratio of either 5 or 9 by microfluidics mixing. hpDNA LNPs controls were prepared by microfluidic mixing at an N/P ratio of 6. *n* = 3.

Figure 11 shows the biophysical characteristics (A) and transfection efficiency of HEK293T cells (B) of mRNA LNPs or, mRNA lipid-peptide nanoparticles (NP) containing peptide K16 and either DOTMA, ALC-0315 or both at a ratio of 10:1 DOTMA:ALC-0315, at an mRNA:peptide ratio of 1:11 and an N/P ratio of 6. The molar ratios of lipids were as described in Table 2. *n* = 3.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

Table 1. sequences in the application

| SEQ ID NO: | name | sequence |
|---|---|---|
| 1 | K16 | KKKKKKKKKKKKKKKK |
| 2 | K30 | KKKKKKKKKKKKKKKKKKKKKKKKKKKKKK |
| 3 | Example DNA sequence | AAAAAACATAAAA |

The invention is further disclosed in the following clauses:

1. A nanoparticle comprising:

(a) a cargo;
(b) a lipid component, wherein the lipid component is one or more ionizable lipids and/or one or more cationic lipids;
(c) a phospholipid;
(d) steroid lipid; and
(e) a cationic polymer.

2. The nanoparticle of clause 1, wherein the nanoparticle does not comprise a targeting moiety or a targeting peptide.

3. The nanoparticle of clause 1 or clause 2, wherein the nanoparticle further comprises a PEG lipid.

4. The nanoparticle of any one of clause 2 to 3, wherein the nanoparticle is a non-viral transfection complex.

5. The nanoparticle of any one of clauses 1 to 4, wherein the lipid component comprises DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA.

6. The nanoparticle of any one of clauses 1 to 4, wherein the lipid component is one or more of DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA.

7. The nanoparticle of any one of clauses 1 to 6, wherein the phospholipid comprises DOPE, DOPC, DSPC, DPPC, DMPC or POPC.

8. The nanoparticle of any one of clauses 1 to 6, wherein the phospholipid is one or more of DOPE, DOPC, DSPC, DPPC, DMPC or POPC.

9.The nanoparticle of any one of clauses 1 to 8, wherein the steroid lipid comprises cholesterol, beta-sitosterol, fucosterol, campesterol, stigamstanol (alkyl steroids), secosteroids (vitamin D2, D3) or pentacyclic steroids.

10. The nanoparticle of any one of clauses 1 to 8, wherein the steroid lipid is one or more of cholesterol, beta-sitosterol, fucosterol, campesterol, stigamstanol (alkyl steroids), secosteroids (vitamin D2, D3) or pentacyclic steroids.

11. The nanoparticle of any one of clauses 1 to 10, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

12. The nanoparticle of any one of clauses 1 to 10, wherein the cationic polymer consists of an oligolysine (linear or branched) such as K16, K17 or K30, an oligohistidine (linear or branched) or an oligoarginine (linear or branched) or PEI.

13. The nanoparticle of any one of clauses 1 to 12, wherein the wherein the Nitrogen/Phosphate (N/P) ratio of the nanoparticle is 3.0-12.0 or 4.0-11.0, optionally wherein:

the nucleic acid-binding cationic component comprises 16 lysine residues and the N/P ratio of the nanoparticle is about 4.0, about 5.0, about 6.0 or about 9.0; or
the nucleic acid-binding cationic component comprises 30 lysine residues and the N/P ratio of the nanoparticle is about 4.0, about 5.0, about 6.0 or about 9.0.

14. The nanoparticle of any one of clauses 1 to 13, wherein:

a molar ratio of the polycationic peptide to the nucleic acid in the nanoparticle is at least 100:1 or at least 650:1 and the N/P ratio of the nanoparticle is about 4;
a molar ratio of the polycationic peptide to the nucleic acid in the nanoparticle is at least 200:1 or at least 1100:1 and the N/P ratio of the nanoparticle is about 6; or
a molar ratio of the polycationic peptide to the nucleic acid in the nanoparticle is at least 750:1 or at least 1400:1 and the N/P ratio of the nanoparticle is about 8.

15. The nanoparticle of any one of clauses 1-14, wherein the Nitrogen/Phosphate (N/P) ratio of the nanoparticle has a charge ratio (i.e. N/P ratio) of 7.0-11.0, 4.0-9.0, or 3.0-8.0.

16. The nanoparticle of any one of clauses 1 to 12, wherein the cationic polymer comprises at least 30 (e.g. 31) positively charged amino acids and the cationic polymer : DNA or RNA cargo (e.g. nucleic acid) molar ratio may be between 30:1 and 1000:1, 75:1 to 750:1 or between 100:1 and 500:1.

17. The nanoparticle of any one of clauses 1 to 12, wherein the cationic polymer comprises at least 15 (e.g. 16) positively charged amino acids, and the cationic polymer : DNA or RNA cargo (e.g. nucleic acid) molar ratio may be between 50:1 to 2000:1, 75:1 to 1000:1 or between 200:1 and 1400:1.

18. The nanoparticle of clauses 16 or 17, wherein the cationic polymer: DNA or RNA mass ratio is 0.1:1 to 9:1.

## EXAMPLES

### Example 1: Liposome formulation

DOTMA, Cholesterol, DOPE, DMG-PEG and ALC-0315 were purchased from Avanti Polar Lipids, Alabaster, Alabama, USA. DLin-MC3-DMA was purchased from Cayman Chemical, Ann Arbor, Michigan, USA.

Liposomes were made using NanoAssemblr Ignite, a microfluidcs device (Precison Nanosystems, Vancouver Canada). The cationic or ionizable lipid, phospholipid, cholesterol, and PEG lipid were mixed together at various molar ratios in ethanol and injected into the cartridge at a total flow rate of 12mL/ min and a flow rate ratio of 3:1 aqueous phase: lipids in ethanol. They were then dialysed overnight in 10k Slide-A-Lyzer (Thermo Fisher) cassettes to remove any residual ethanol. Prior to use in experiments, liposomes were stored at 4°C.

### Example 2: Biophysical characteristics and of cationic and ionizable hpDNA lipid-peptide nanoparticles

Peptides K16 and K30 were synthesised via solid-phase peptide synthetic chemistry. hpDNA contained an eGFP reporter gene. hpDNA lipid-peptide nanoparticles were formed via electrostatic interaction by vigorous pipette mixing of liposomes, peptide, and hpDNA, in that order. The cationic or ionizable lipid was either DOTMA, ALC-0315 or DLin-MC3-DMA. The molar ratio of lipids was either 59% cationic/ionizable lipid, 10% Cholesterol, 29.5% DOPE and 1.5% DMG-PEG (denoted 102 1.5%), or 50.57% cationic/ionizable lipid, 40% Cholesterol, 8.43% DOPE and 1% DMG-PEG (denoted 406 1%). The mass ratio of hpDNA:liposome was either 1:11 or 1:15 and either cationic peptide K16 or K30 was added to give a total nitrogen/phosphate ratio (N/P ratio) of 6. The final hpDNA concentration was 80μg/mL. Prior to use in experiments, hpDNA lipid-peptide nanoparticles were stored at 4°C.

hpDNA nanoparticles lacking peptide were prepared as controls for each cationic or ionizable lipid at ratios comparable to a conventional mRNA LNP. These controls were termed "LNP" for clarity. The molar ratio of lipids was 50% cationic/ionizable, 38.5% Cholesterol, 10% DOPE and 1.5% DMG-PEG and the N/P ratio was 6. hpDNA LNPs were prepared as per conventional mRNA LNP microfluidics methods. Briefly, lipids in ethanol were mixed with hpDNA in 10mM NaAC (pH 4) for ionizable lipids, or water for DOTMA using the NanoAssemblr Ignite at a total flow rate of 12mL/ min and a flow rate ratio of 3:1 aqueous phase: lipids in ethanol. The final hpDNA concentration was 80μg/mL. They were then dialysed overnight in 10k Slide-A-Lyzer (Thermo Fisher) cassettes to remove any residual ethanol and NaAC. Prior to use in experiments, hpDNA LNPs were stored at 4°C.

The size in nm (diameter) and polydispersity index (PDI) of the hpDNA-lipid-peptide nanoparticles or LNPs was measured by Dynamic Light Scattering (DLS) using the Zetasizer Ultra (Malvern). Samples containing 1.6 μg hpDNA were diluted in 1ml nuclease free water for analysis. The results show that the hpDNA lipid-peptide nanoparticles have favourable biophysical characteristics.

Figure 1 shows the size and PDI of lipid-peptide hpDNA nanoparticles prepared by hand mixing with three different cationic or ionizable lipids at two different molar ratios of lipid (102 1.5% or 406 1%). Nanoparticles were prepared with two non-targeting peptides (K16 and K30) at two different mass ratios of hpDNA to lipid (1:11 and 1:15), whilst the N/P ratio was always 6. For ionizable formulations (ALC-315 and DLin-MC3-DMA), the range of sizes was between 54 and 182nm and the PDI was always below 0.22, indicating a uniform population. Nanoparticles prepared with the cationic lipid DOTMA were of comparable size to the ionizable formulations, between 75 and 130nm, but the PDI was on average higher, between 0.22 and 0.35. The hpDNA LNPs prepared with a standard microfluidics method were in general smaller that the hpDNA lipid-peptide nanoparticles prepared with the same cationic or ionizable lipid, but the PDI was generally comparable despite the superior mixing expected by using microfluidics. The exception to this was DOTMA lipid-peptide hpDNA nanoparticles where the PDI was higher than the DOTMA LNP, although the uniformity of lipid-peptide hpDNA nanoparticles is expected to improve by using a microfluidics mixing method.

Figure 2 shows the biophysical characteristics of a representative hpDNA lipid-peptide nanoparticle, as well as a hpDNA LNP, for three cationic or ionizable lipids. A-C show hpDNA lipid-peptide nanoparticles prepared with DOTMA, ALC-0315 or DLin-MC3-DMA 406 1%, respectively, with peptide K30 at a 1:15 hpDNA: lipid mass ratio. D-F show hpDNA LNPs prepared with DOTMA, ALC-0315 or DLin-MC3-DMA, respectively.

### Example 3: HEK293T transfection - eGFP

HEK293T cells, an immortalised human embryonic kidney cell line, were cultured in Dulbecco's Modified Eagle's Medium (DMEM, Gibco) containing 10% fetal bovine serum (FBS, Gibco) and 1% pen/strep (Gibco), termed full growth media.

Transfections were performed in a 96-well plate at a density of 16,000 (HEK293T) cells per well, seeded 24 h

previously. hpDNA lipid-peptide nanoparticles and LNPs, prepared as above with differing cationic/ionizable lipids and lipid molar ratios, were diluted in OptiMEM reduced serum media (Gibco) to a hpDNA concentration of 1.5 μg/mL and delivered to cells at a dose of 300 ng per well. Controls included cells with no transfection complexes added (OptiMEM only). All conditions were performed in triplicate. Cells were incubated at 37°C for 4 hours, before replacing media with growth media. 48 h post-transfection, the cells were rinsed in PBS, before incubation in 0.05% Trypsin EDTA (Gibco) to detach the cells. Cells were then resuspended in full growth media for analysis by flow cytometry using the Aligent Novocyte flow cytometer.

Figure 3 shows the transfection efficiency of HEK293T cells, transfected with hpDNA lipid-peptide nanoparticles at a hpDNA: lipid mass ratio of 1:11 (Figure 3A) or 1:15 (Figure 3B) and an N/P ratio of 6, 48 hours post-transfection. For DOTMA lipid-peptide nanoparticles, the transfection efficiency at 1:11 mass ratio was lower than the DOTMA hpDNA LNP, however increasing the lipid to a 1:15 mass ratio demonstrated comparable transfection efficiency to the DOTMA hpDNA LNP control. There was no difference in transfection efficiency between the 102 and 406 molar lipid ratios at either 1:11 or 1:15. For ALC and MC3 lipid-peptide nanoparticles, the transfection efficiency exceeded that of the ALC or MC3 hpDNA LNP regardless of lipid molar ratio, or hpDNA: lipid mass ratio. In general, ALC at the 406 molar ratio demonstrated greater transfection efficiency than the 102 molar ratio, but for MC3 the 102 molar ratio was superior to the 406 molar ratio.

Therefore, hpDNA lipid-peptide nanoparticles with all three cationic/ionizable lipids achieved a transfection efficiency comparable or greater to that of an hpDNA LNP with an equivalent N/P ratio made using a conventional microfluidics method. To achieve this, DOTMA nanoparticles required a 1:15 hpDNA: lipid mass ratio, however, ALC and MC3 lipid-peptide nanoparticles demonstrated superior transfection efficiency to the hpDNA LNP at both lipid molar ratios and both hpDNA: lipid mass ratios tested. This was despite the lower overall lipid mass of a lipid-peptide nanoparticle compared to an LNP. The hpDNA: lipid mass ratios of the LNPs are shown in the table below.

*Table 2: The hpDNA: lipid mass ratio required to achieve an N/P ratio of 6 for 4 cationic or ionizable LNPs, where the lipid component consists of cationic/ionizable lipid, Cholesterol, DOPE and DMG-PEG at the molar ratio of 50: 38.5:10:1.5.*

| **Cationic/ionizable lipid in LNP** | **Mass ratio hpDNA: lipid** |
|---|---|
| DOTMA | 1:21.7 |
| ALC-0315 | 1:23.4 |
| DLin-MC3-DMA | 1:21.2 |

**Example 4: hpDNA lipid-peptide nanoparticles can be prepared by either hand-mixing or microfluidic methods**

hpDNA lipid-peptide nanoparticles were prepared by either a hand-mixing method (above) or a microfluidic method using the NanoAssemblr Ignite. For hpDNA lipid-peptide nanoparticles prepared using the NanoAssemblr Ignite, liposomes and peptide in water were mixed by vigorous pipetting and vortexing, before mixing this lipid/peptide solution with hpDNA in water on the NanoAssemblr Ignite at a FRR of 3:1 and a TFR of 12 mL/min. Liposomes were either DOTMA or ALC-0315/Cholesterol/DOPE/DMG-PEG at a molar ratio of 50.57:40:8.43:1 (406 1%), or DLin-MC3-DMA /Cholesterol/DOPE/DMG-PEG at a molar ratio of 59:10:29.5:1.5 (102 1.5%). The hpDNA: lipid mass ratio was 1:15 and peptide K16 was added to give an N/P ratio of 6.

Figure 4 shows the biophysical characteristics of DOTMA, ALC-0313 or DLin-MC3-DMA hpDNA lipid-peptide nanoparticles or LNPs prepared by either hand mixing or NanoAssemblr microfluidics methods. Preparing lipid-peptide nanoparticles with microfluidics produced smaller nanoparticles than hand mixing and these particles were of comparable size to the hpDNA LNP controls. For the ionizable lipid-peptide nanoparticles, the PDI was similar between hand mixing and microfluidics methods, but for DOTMA the microfluidics method had a lower PDI, indicating a more uniform population. Additionally, the PDI of the lipid-peptide nanoparticles was similar to that of the hpDNA LNPs, indicating comparable uniformity.

Figure 5 shows the transfection efficiency of HEK293T cells 48 hours post transfection with DOTMA, ALC-0315 or DLin-MC3-DMA hpDNA lipid-peptide nanoparticles or LNPs, measured using flow cytometry. Figure 6 shows the corresponding fluorescent microscopy images 48 hours post-transfection, imaged with an EVOS M500 imaging system (ThermoFisher Scientific, Waltham, Massachusetts, USA) using an EVOS 4X objective (Cat no. AMP4980) and a GFP light cube (470/525 nm excitation/emission).

The commercially available transfection reagent Lipofectamine 2000 (L2K) was added to cells as a transfection control at the same dose as the nanoparticles (300 ng/well) at a ratio of 1:3 hpDNA:L2K, diluted in OptiMEM. Lipid-peptide nanoparticles prepared using the NanoAssemblr method demonstrated a comparable or greater number of GFP-positive cells and MFI than the same formulation prepared using a hand mixing method. The commercially available transfection reagent L2K demonstrated higher transfection efficiency compared to the lipid-peptide nanoparticles, however, was highly toxic and substantial cell death was observed on the microscopy images. No signs of cell death were observed with the lipid-peptide nanoparticles and their appearance was comparable to untransfected (UT) cells.

**Example 5: Stability of nanoparticles encapsulating mRNA**

mRNA lipid-peptide nanoparticles were prepared as per the microfluidic method described for hpDNA, Example 2. The liposome was 50.57% DOTMA, 40% Cholesterol, 8.43% DOPE and 1% DMG-PEG (DOTMA 406 1%) and the mRNA: lipid ratio was 1:15. The peptide was K16 and was added to bring the total N/P ratio of the mRNA lipid-peptide nanoparticle to either 5 or 9 (denoted 1 :15:(5) or 1 :15:(9)). The mRNA contained an eGFP reporter gene sequence. A no peptide control was also prepared by microfluidics mixing using the same DOTMA 406 1% liposome but omitting the peptide. This is termed LNP, and the N/P ratio was 6. After preparation all nanoparticles were diluted to an mRNA concentration of 40 μg/mL in 30% trehalose in water to bring the trehalose concentration to 15% and stored at either +4°C or -80°C for the duration of the experiment. Biophysical characteristics and HEK293T transfection efficiency were determined at 0, 1, 7, 28 and 42 days post-preparation. The T=0 time point was immediately post-preparation and prior to storage at +4°C or -80°C.

Figure 7 shows that the size and PDI of all nanoparticles remained constant over the 42-day duration of the experiment at both +4°C (Figure 8A) and -80°C (Figure 8B) storage, confirming the stability of the formulations. Further, the size and PDI of the two mRNA lipid-peptide nanoparticles was comparable to that of the LNP, which contained no peptide but a higher lipid concentration, confirming that including a peptide and reducing the lipid concentration does not affect nanoparticle stability. At - 80°C, the PDI of the 1:15:(9) lipid-peptide nanoparticle increased slightly upon freezing, but the PDI remained stable after this at approximately 0.2, indicating the population remained uniform. Biophysical traces of these formulations at Day 1 and Day 42 are shown in figures 8 (+4°C) and 9 (-80°C).

Figure 10 shows transfection efficiency of all nanoparticles also remained constant over the 42-day duration of the experiment at both +4°C (Figure 10A) and -80°C (Figure 10B) storage and, was consistently -90%. Lipid-peptide nanoparticles at ether 1:15:(5) or 1:15:(9) demonstrated the same transfection efficiency as the DOTMA LNP in terms of number of GFP-positive cells and MFI.

**Example 6: mRNA lipid-peptide nanoparticles containing both DOTMA and ALC-0315**

Liposomes containing DOTMA, ALC-0315 or a combination of both at a 10:1 ratio of DOTMA:ALC, as well as Cholesterol, DOPE and DMG-PEG at the molar ratios in Table 2 were prepared using a NanoAssemblr Ignite, a microfluidics device, as above. The molar ratio of the cationic/ionizable component was always 50.6. mRNA lipid-peptide nanoparticles were prepared by hand mixing as above with K16 peptide at an mRNA:lipid ratio of 1:11 and an N/P ratio of 6. mRNA LNPs were prepared as above with a conventional microfluidics mixing method.

Figure 11 shows the biophysical characteristics (Figure 11A) and HEK293T transfection efficiency (Figure 11B) of lipid-peptide nanoparticles prepared with DOTMA and ALC-0315 at a 1:10 molar ratio, as compared to those containing either DOTMA or ALC-0315 alone, or to LNP controls. Nanoparticles containing both DOTMA and ALC-0315 were of comparable size and PDI to those containing either DOTMA or ALC-0315 alone. As with hpDNA lipid-peptide hand mixed nanoparticles, the size was larger than the LNP controls, although they were still small, between 90 and 120 nm, and the PDI was comparable.

*Table 3: Lipid molar ratios of liposomes prepared with DOTMA, ALC-0315, or a combination of both and, the lipid molar ratios within DOTMA and ALC-0315 LNPs.*

| | Molar Ratio | | | | |
|---|---|---|---|---|---|
| | **DOTMA: ALC 10: 1** | **DOTMA only** | **ALC only** | **DOTMA LNP** | **ALC LNP** |
| **DOTMA** | 46.0 | 50.6 | 0.0 | 50 | 0 |
| **ALC** | 4.6 | 0.0 | 50.6 | 0 | 50 |
| **Chol** | 40.0 | 40.0 | 40.0 | 38.5 | 38.5 |
| **DOPE** | 8.4 | 8.4 | 8.4 | 10 | 10 |
| **DMG-PEG** | 1.0 | 1.0 | 1.0 | 1.5 | 1.5 |

98% of the cells transfected the DOTMA:ALC 10:1 nanoparticles were GFP-positive, compared to 82% and 66% of DOTMA only or ALC-0315 only nanoparticles, respectively. Similarly, the MFI of the DOTMA:ALC 10:1 nanoparticles was 3 and 18-fold greater than DOTMA or ALC-0315 nanoparticles, respectively. Compared to the LNPs, the DOTMA:ALC 10:1 nanoparticles had more GFP-positive cells than the ALC-0315 LNP and comparable to the DOTMA LNP. The MFI of the DOTMA:ALC 10:1 nanoparticles was greater than both ALC-0315 and DOTMA LNPs.

## Claims

**1.** A nanoparticle comprising:

(f) a cargo;
(g) a lipid component, wherein the lipid component is one or more ionizable lipids and/or one or more cationic lipids;
(h) a phospholipid;
(i) steroid lipid; and
(j) a cationic polymer.

**2.** The nanoparticle of claim 1, wherein the nanoparticle does not comprise a targeting moiety or a targeting peptide.

**3.** The nanoparticle of claim 1 or claim 2, wherein the nanoparticle further comprises a PEG lipid.

**4.** The nanoparticle of any one of claims 2 to 3, wherein the nanoparticle is a non-viral transfection complex.

**5.** The nanoparticle of any one of claims 1 to 4, wherein the cargo is RNA, optionally wherein the RNA is mRNA.

**6.** The nanoparticle of any one of claims 1 to 4, wherein the cargo is DNA, optionally wherein the DNA is: (i) a closed linear DNA molecule; (ii) a linear deoxyribonucleic acid (DNA) molecule comprising one or more nuclease-resistant nucleotides and a cassette, wherein one or more nuclease-resistant nucleotides in the linear DNA molecule are located outside of the cassette; or (iii) a partially closed linear deoxyribonucleic acid (DNA) molecule comprising a double-stranded DNA portion that is closed at a first end and open at a second end, wherein the partially closed linear DNA molecule comprises one or more nuclease-resistant nucleotides in an open end region adjacent to the second end.

**7.** The nanoparticle of any one of claims 1 to 6, wherein the cationic polymer is a polycationic peptide.

**8.** The nanoparticle of claim 7, wherein the polycationic peptide comprises a nucleic acid-binding cationic component, optionally wherein the nucleic acid-binding cationic component is oligolysine.

**9.** The nanoparticle of any one of claims 1 to 8 wherein the lipid component comprises one or more of DLin-MC3-DMA, DLin-KC2-DMA, DLin-DMA, TCL053, SM-102, ALC-0315, C12-200, DODMA, DODAP, Lipid A9, 9A1P9, Lipid C24, Lipid LP01, Lipid 5, DOTMA, DTDTMA or DHDTMA.

**10.** The nanoparticle of any one of claims 1 to 9, wherein the cationic polymer comprises an oligolysine (linear or branched) such as K16, K17 or K30, an oligohistidine (linear or branched) or an oligoarginine (linear or branched) or combination of an oligolysine and an oligohistidine, an oligohistidine and an oligoarginine, an oligoarginine and an oligolysine, or oligolysine, oligohistidine and oligoarginine, or PEI.

**11.** A pharmaceutical composition comprising the nanoparticle of any one of claims 1-10 and, optionally, a pharmaceutically suitable carrier, optionally wherein the pharmaceutical composition is a vaccine.

**12.** The nanoparticle of any one of claims 1-10 or the pharmaceutical composition of claim 11 for use in therapy.

**13.** A method for transfecting a cell comprising:

(a) contacting a cell with the nanoparticle of any one of claims 1-10 or the pharmaceutical composition of claim 11; and
(b) transfecting the nanoparticle or the pharmaceutical composition to the cell.

**14.** A library comprising two or more nanoparticles of any one of claims 1-10, wherein the polydispersity index (PDI) of the nanoparticles in the library is less than 0.2, preferably 0.15.

**15.** A method for forming the nanoparticle of any one of claims 1-10, the method comprising:

(a) contacting (i) a cargo, (ii) a lipid component, wherein the lipid component is one or more ionizable lipids and/or one or more cationic lipids; (iii) a phospholipid; (iv) a steroid lipid; and (v) a cationic polymer; and
(b) forming the nanoparticle.

Fig. 1

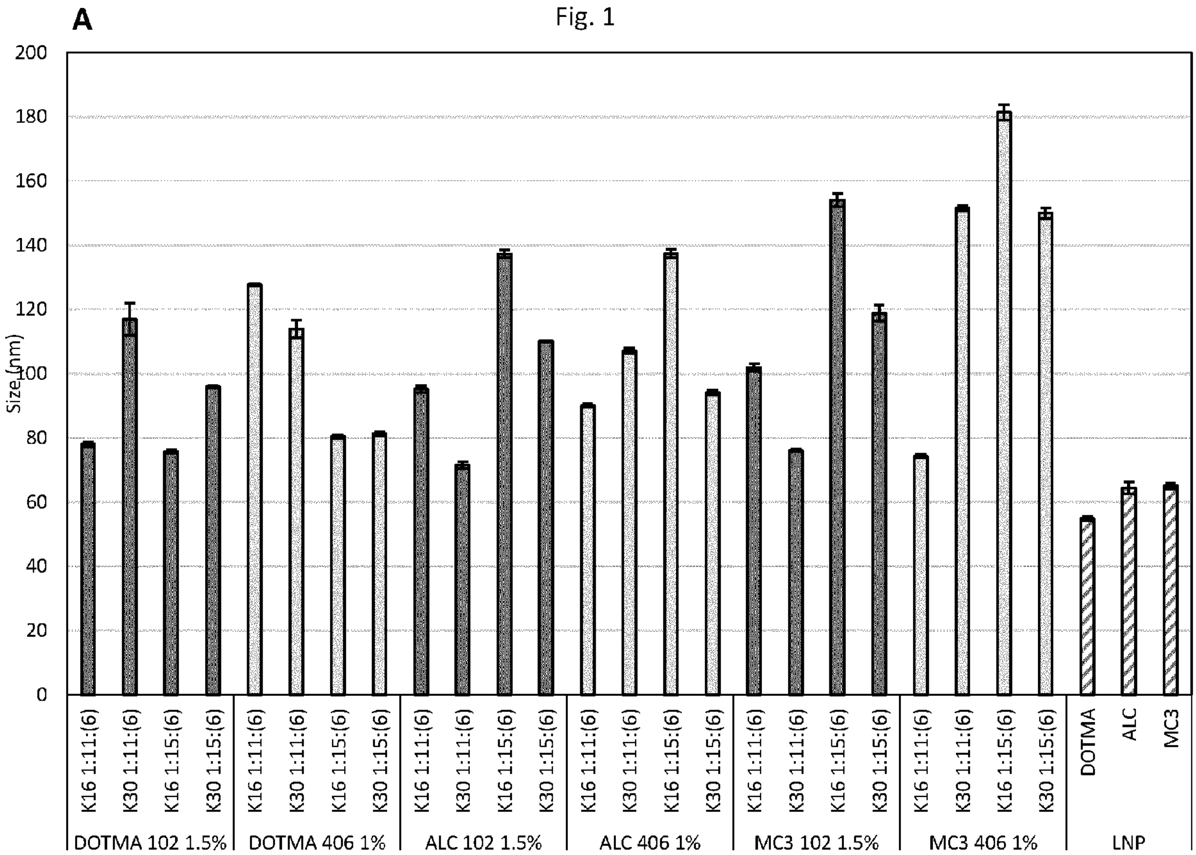
A
Size (nm)
200
180
160
140
120
100
80
60
40
20
0
K16 1:11:(6)
K30 1:11:(6)
K16 1:15:(6)
K30 1:15:(6)
DOTMA
ALC
MC3
DOTMA 102 1.5%
DOTMA 406 1%
ALC 102 1.5%
ALC 406 1%
MC3 102 1.5%
MC3 406 1%
LNP

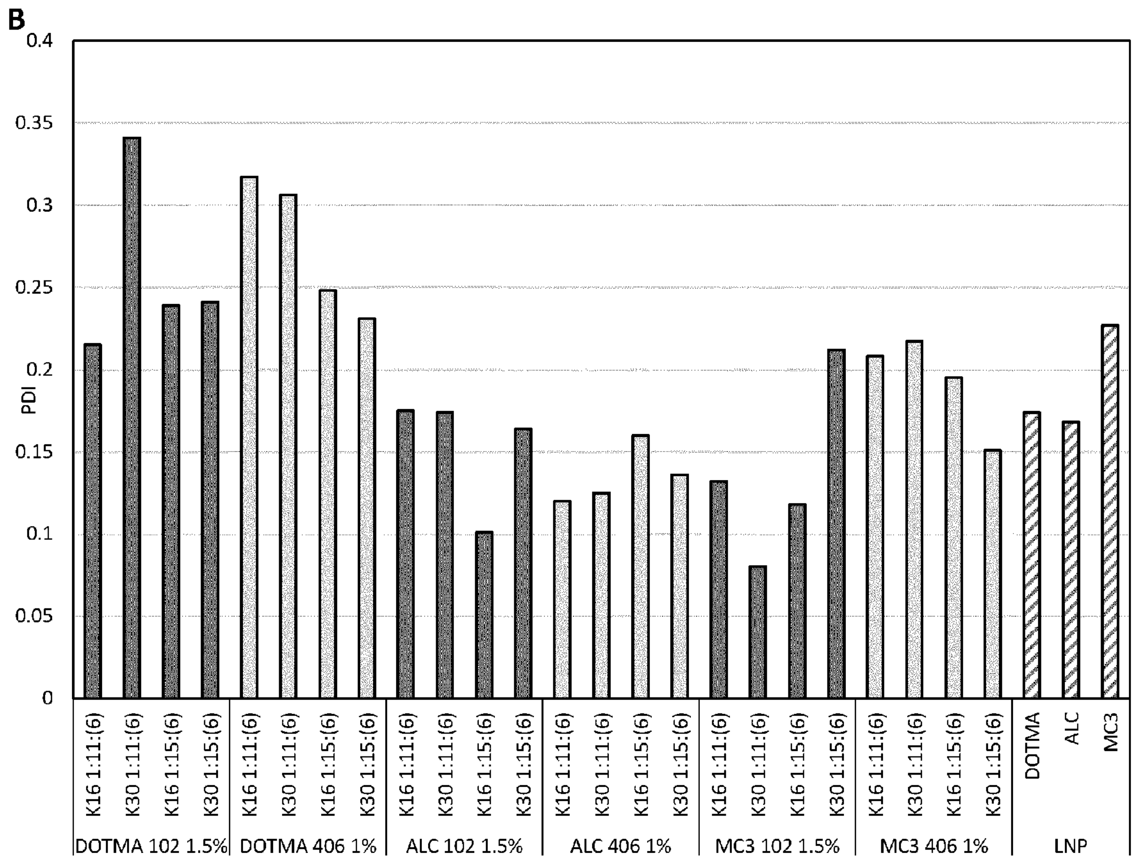
B
PDI
0.4
0.35
0.3
0.25
0.2
0.15
0.1
0.05
0
K16 1:11:(6)
K30 1:11:(6)
K16 1:15:(6)
K30 1:15:(6)
DOTMA
ALC
MC3
DOTMA 102 1.5%
DOTMA 406 1%
ALC 102 1.5%
ALC 406 1%
MC3 102 1.5%
MC3 406 1%
LNP

Fig. 2

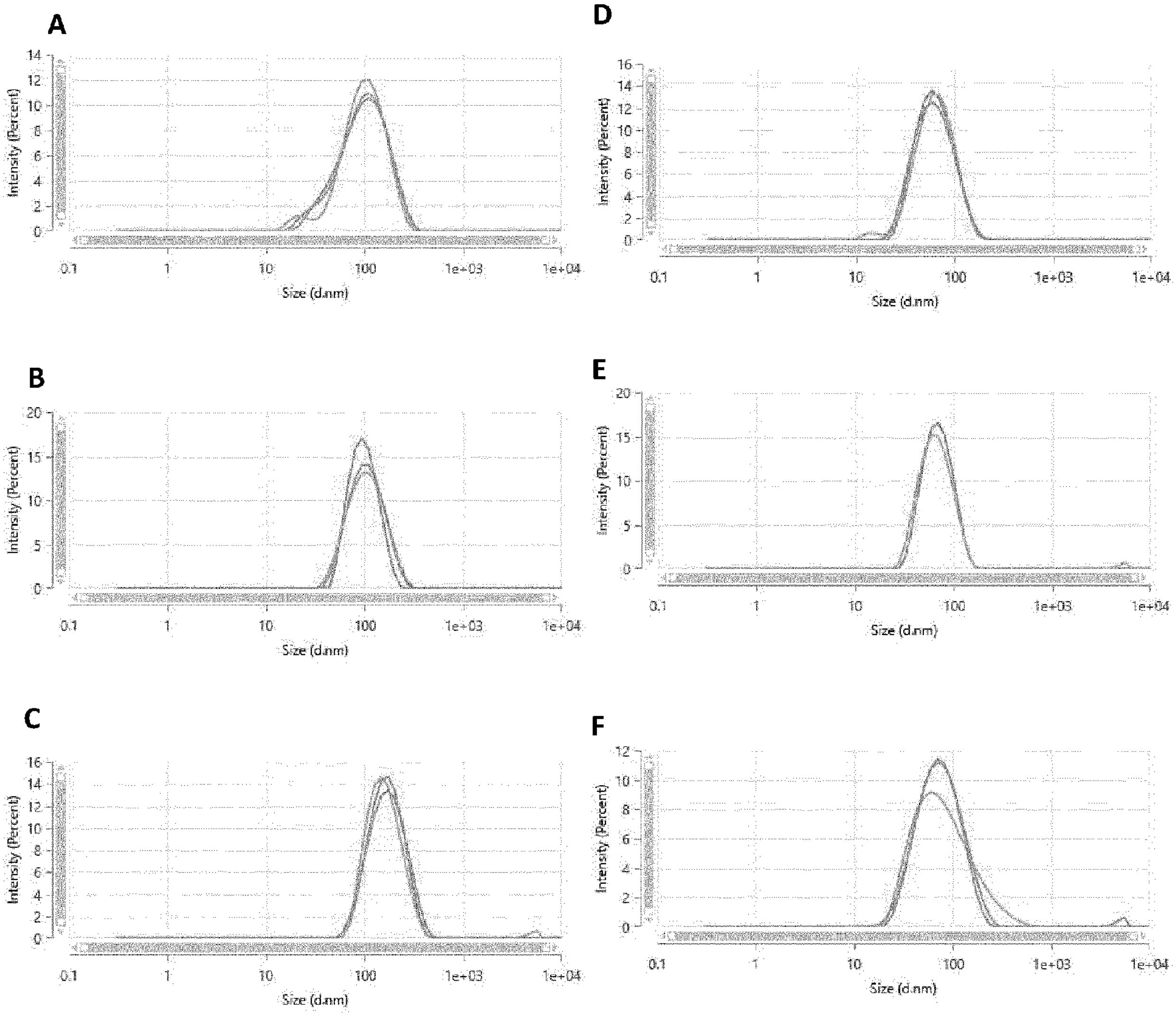

Fig. 3

A

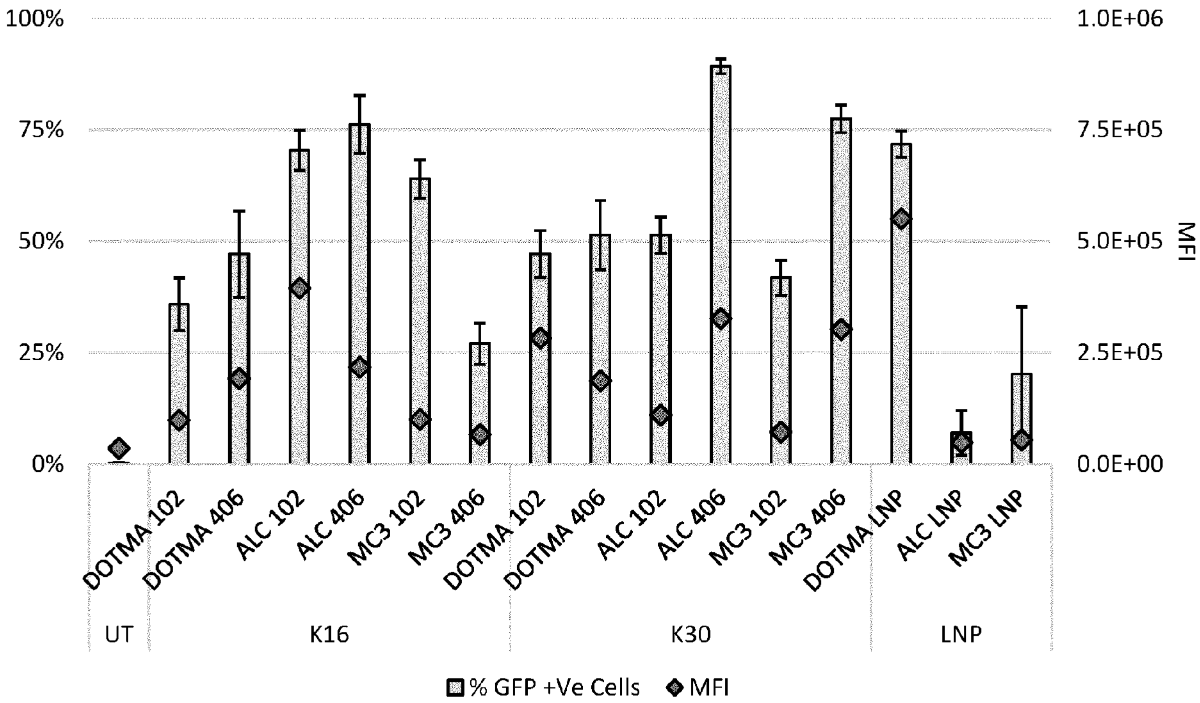
100%
75%
50%
25%
0%
%GFP +Ve Cells
1.0E+06
7.5E+05
5.0E+05
2.5E+05
0.0E+00
MFI
DOTMA 102
DOTMA 406
ALC 102
ALC 406
MC3 102
MC3 406
DOTMA 102
DOTMA 406
ALC 102
ALC 406
MC3 102
MC3 406
DOTMA LNP
ALC LNP
MC3 LNP
UT
K16
K30
LNP
% GFP +Ve Cells
MFI

B

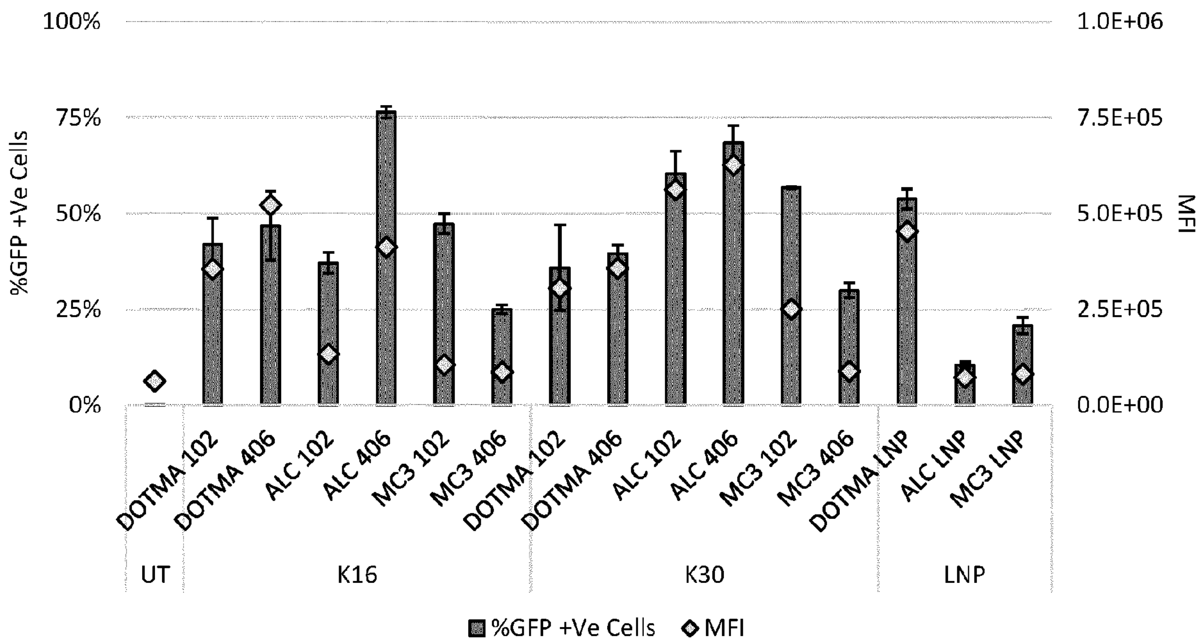
100%
75%
50%
25%
0%
%GFP +Ve Cells
1.0E+06
7.5E+05
5.0E+05
2.5E+05
0.0E+00
MFI
DOTMA 102
DOTMA 406
ALC 102
ALC 406
MC3 102
MC3 406
DOTMA 102
DOTMA 406
ALC 102
ALC 406
MC3 102
MC3 406
DOTMA LNP
ALC LNP
MC3 LNP
UT
K16
K30
LNP
%GFP +Ve Cells
MFI

Fig. 4

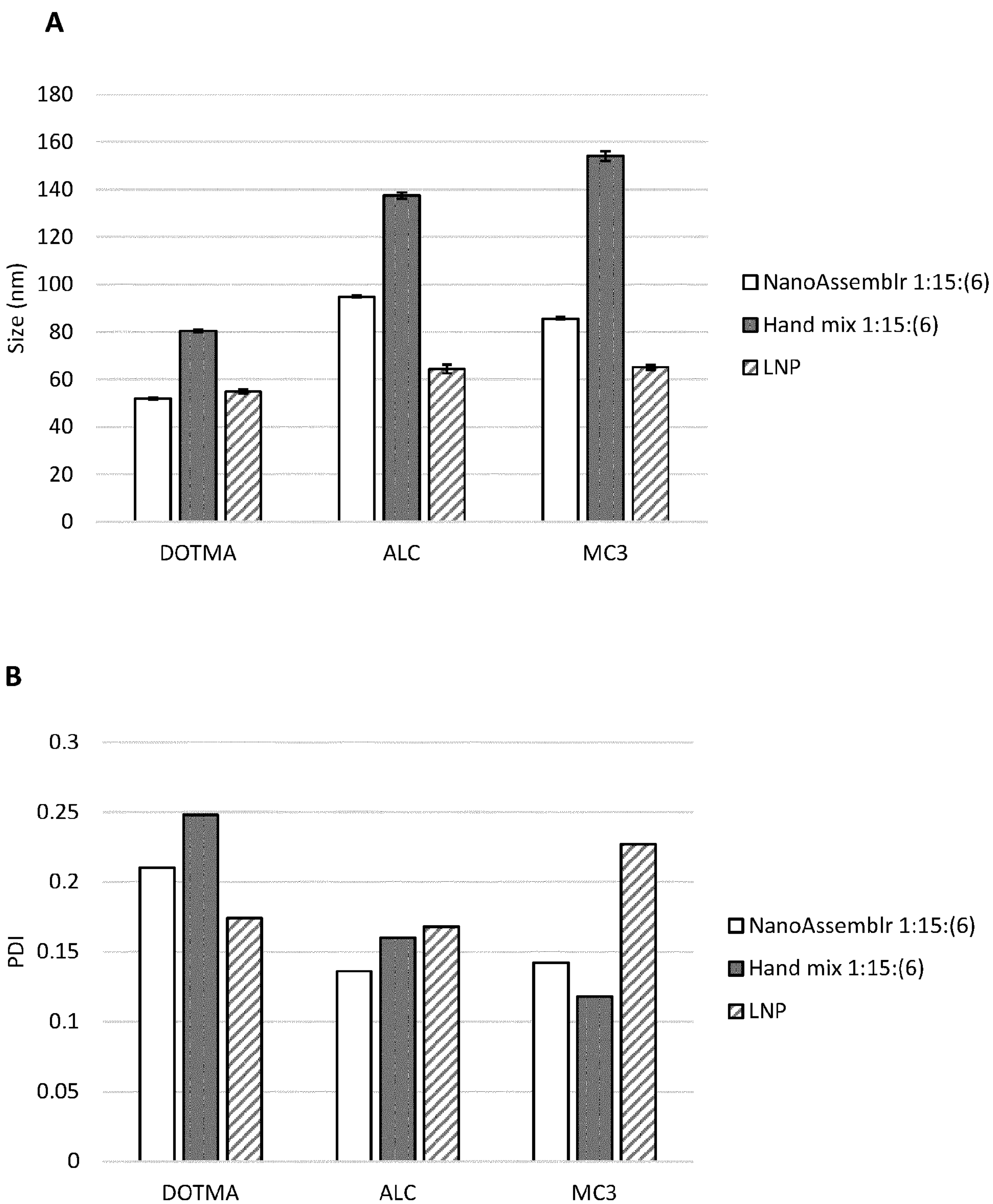
A
180
160
140
120
100
80
60
40
20
0
Size (nm)
DOTMA
ALC
MC3
NanoAssemblr 1:15:(6)
Hand mix 1:15:(6)
LNP
B
0.3
0.25
0.2
0.15
0.1
0.05
0
PDI
DOTMA
ALC
MC3
NanoAssemblr 1:15:(6)
Hand mix 1:15:(6)
LNP

Fig. 5

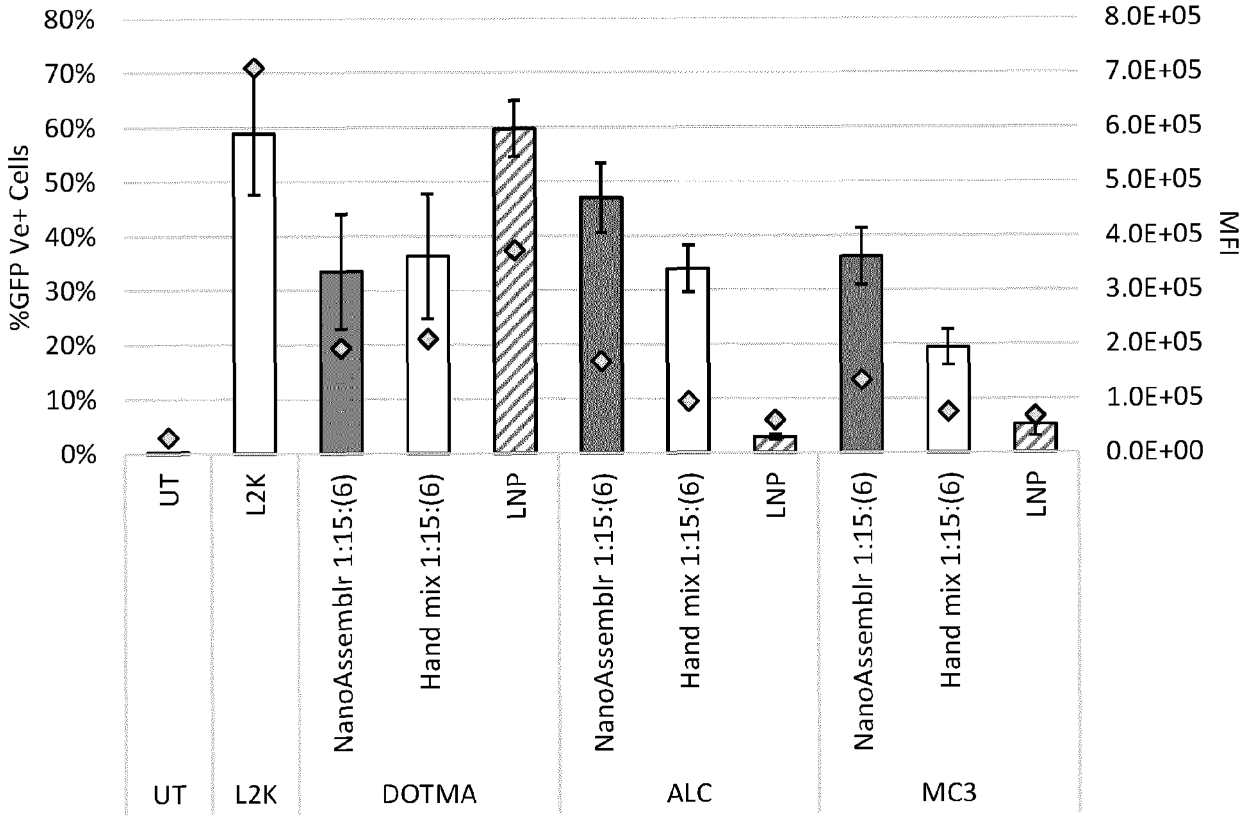
%GFP Ve+ Cells
80%
70%
60%
50%
40%
30%
20%
10%
0%
MFI
8.0E+05
7.0E+05
6.0E+05
5.0E+05
4.0E+05
3.0E+05
2.0E+05
1.0E+05
0.0E+00
UT
L2K
NanoAssemblr 1:15:(6)
Hand mix 1:15:(6)
LNP
NanoAssemblr 1:15:(6)
Hand mix 1:15:(6)
LNP
NanoAssemblr 1:15:(6)
Hand mix 1:15:(6)
LNP
UT
L2K
DOTMA
ALC
MC3

Fig. 6

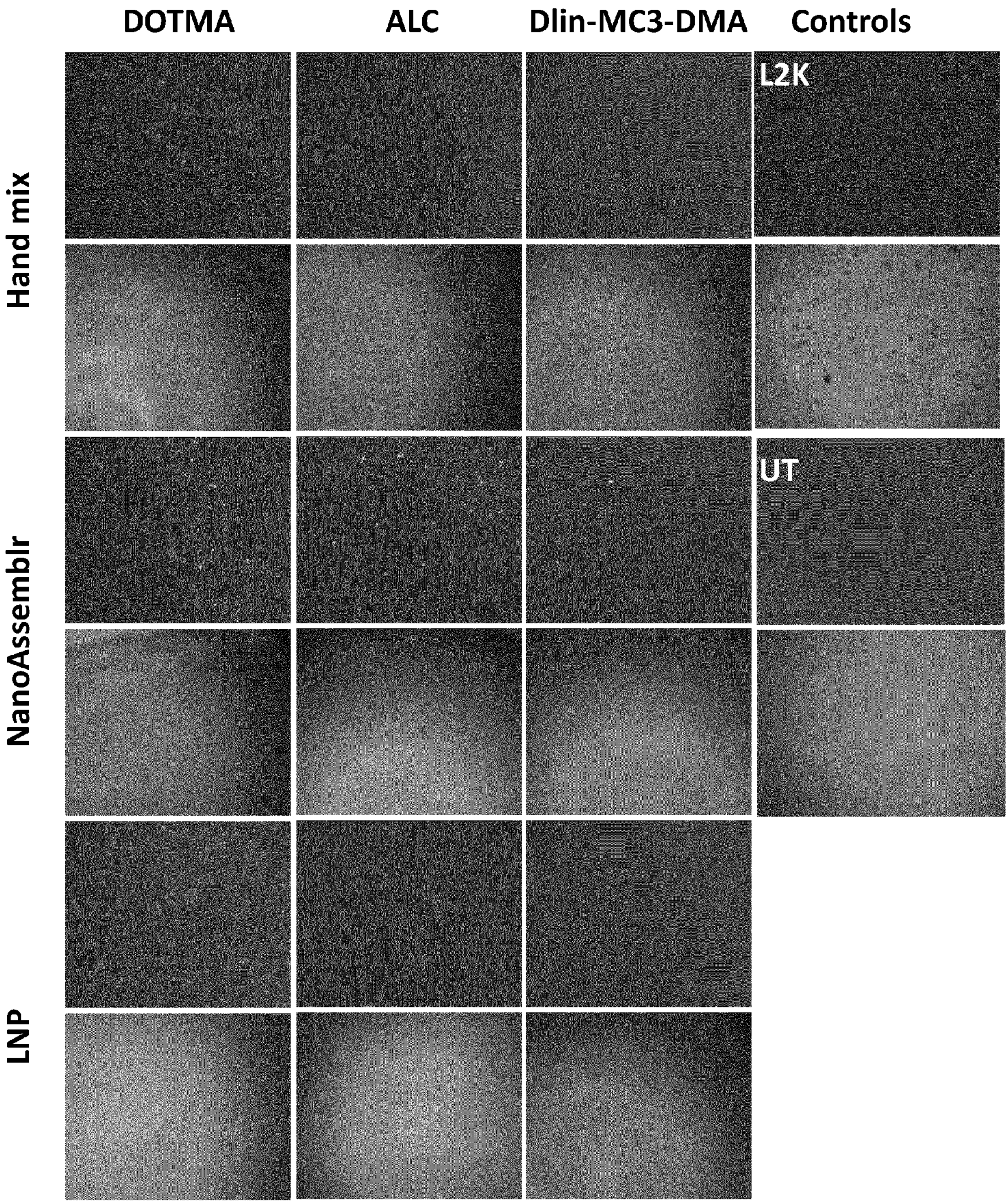
DOTMA
ALC
Dlin-MC3-DMA
Controls
Hand mix
NanoAssemblr
LNP
L2K
UT

Fig. 7

A

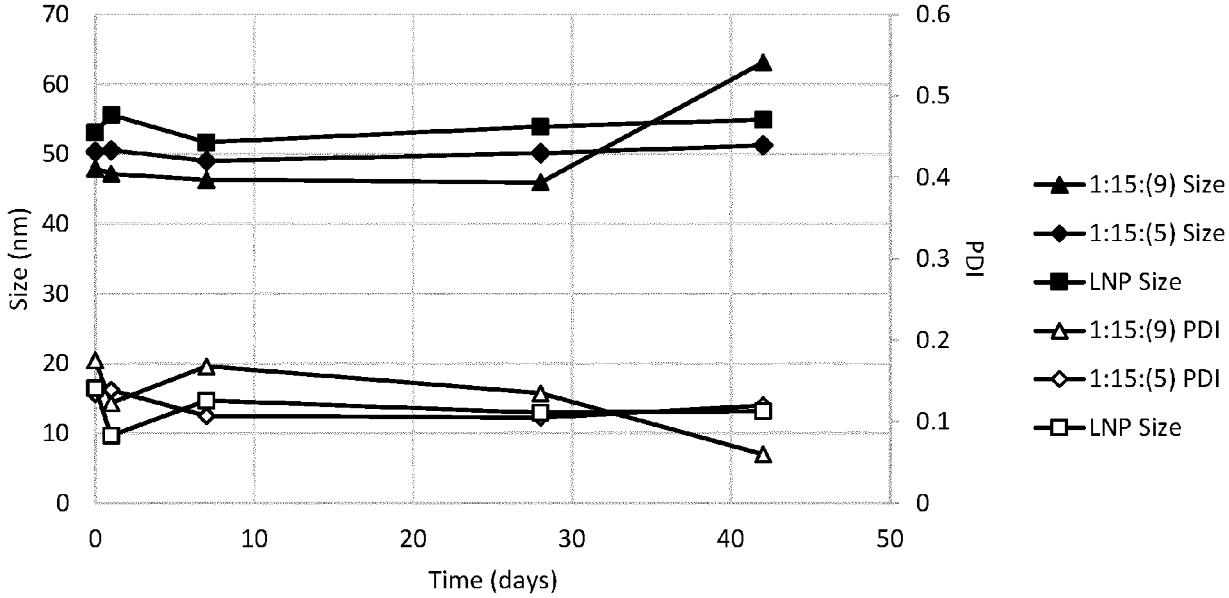
70
60
50
40
30
20
10
0
Size (nm)
0.6
0.5
0.4
0.3
0.2
0.1
0
PDI
0
10
20
30
40
50
Time (days)
1:15:(9) Size
1:15:(5) Size
LNP Size
1:15:(9) PDI
1:15:(5) PDI
LNP Size

B

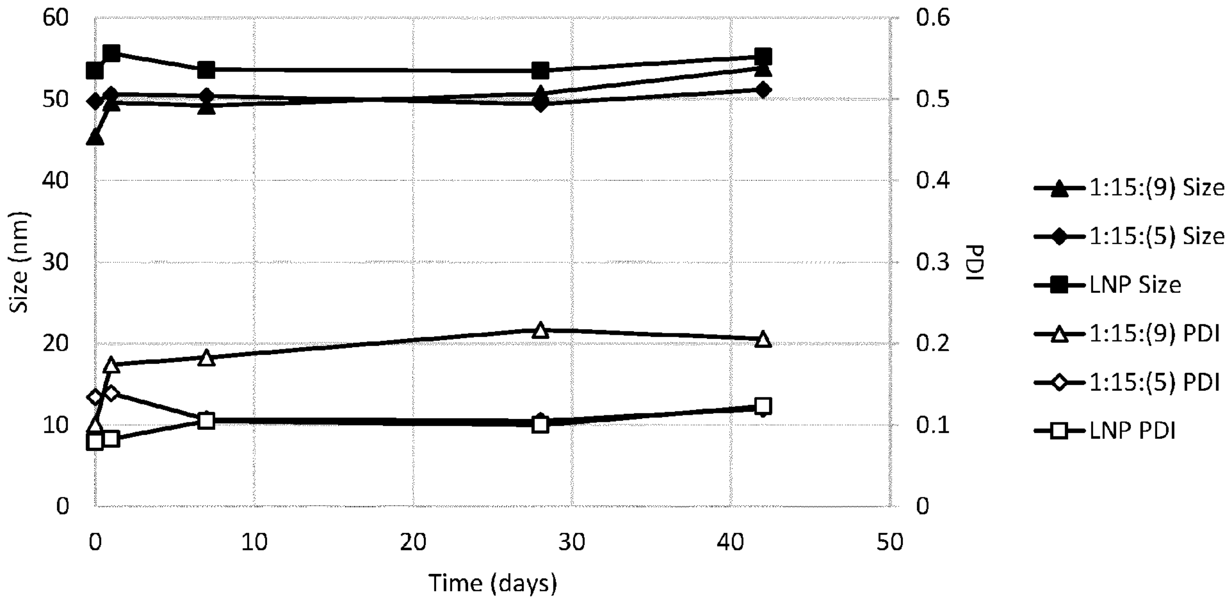
60
50
40
30
20
10
0
Size (nm)
0.6
0.5
0.4
0.3
0.2
0.1
0
PDI
0
10
20
30
40
50
Time (days)
1:15:(9) Size
1:15:(5) Size
LNP Size
1:15:(9) PDI
1:15:(5) PDI
LNP PDI

Fig. 8

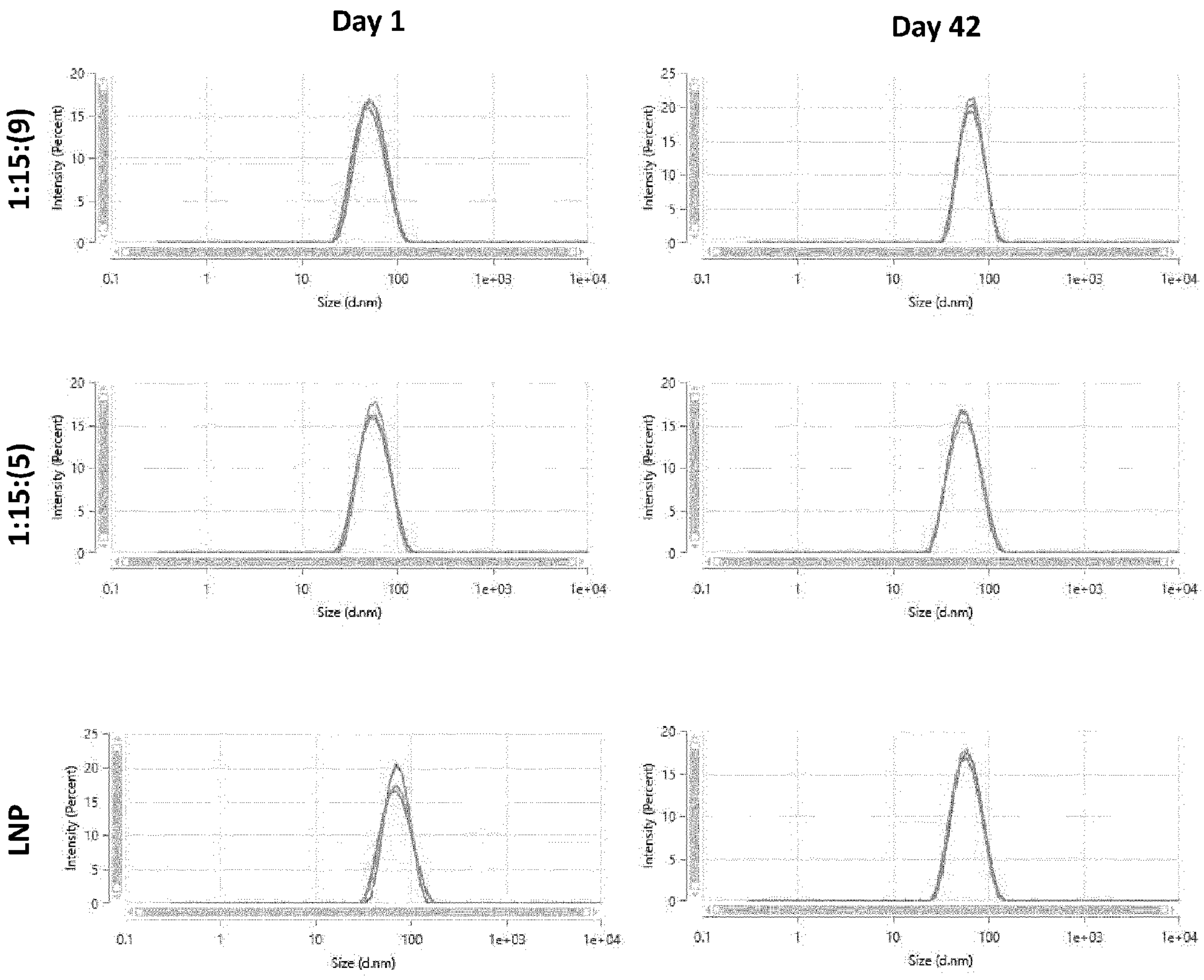
Day 1
Day 42
1:15:(9)
1:15:(5)
LNP
Intensity (Percent)
Size (d.nm)
0.1
1
10
100
1e+03
1e+04

Fig. 9

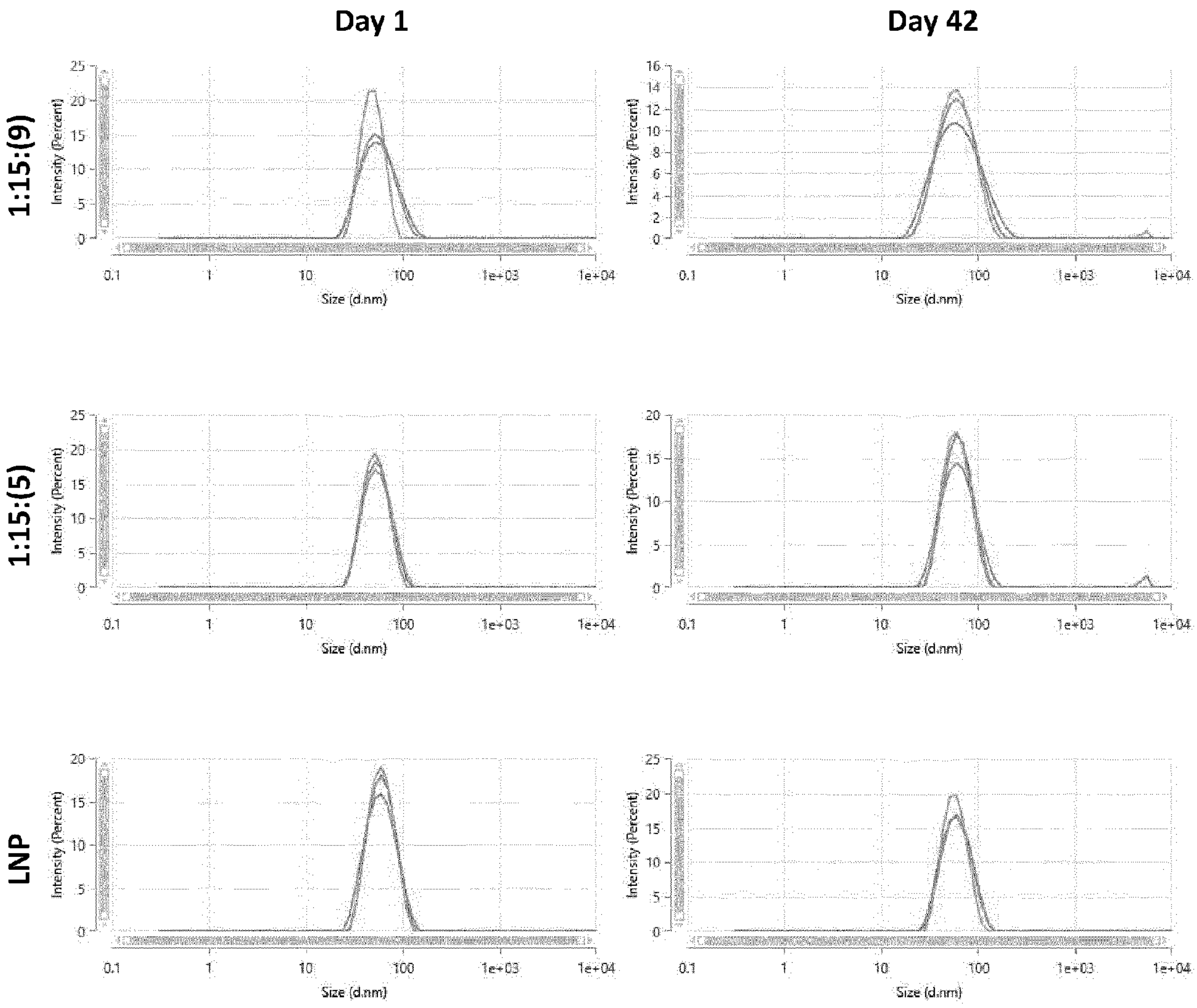
Day 1
Day 42
1:15:(9)
1:15:(5)
LNP
Intensity (Percent)
Size (d.nm)
0.1
1
10
100
1e+03
1e+04

Fig. 10

A

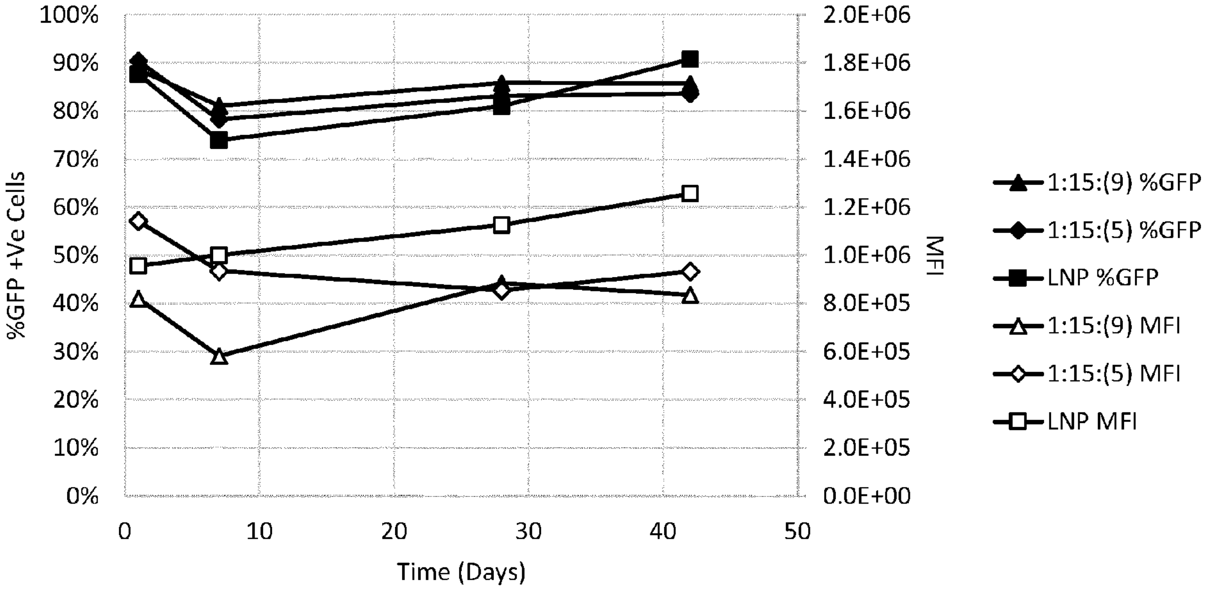
100%
90%
80%
70%
60%
50%
40%
30%
20%
10%
0%
%GFP +Ve Cells
2.0E+06
1.8E+06
1.6E+06
1.4E+06
1.2E+06
1.0E+06
8.0E+05
6.0E+05
4.0E+05
2.0E+05
0.0E+00
MFI
0
10
20
30
40
50
Time (Days)
1:15:(9) %GFP
1:15:(5) %GFP
LNP %GFP
1:15:(9) MFI
1:15:(5) MFI
LNP MFI

B

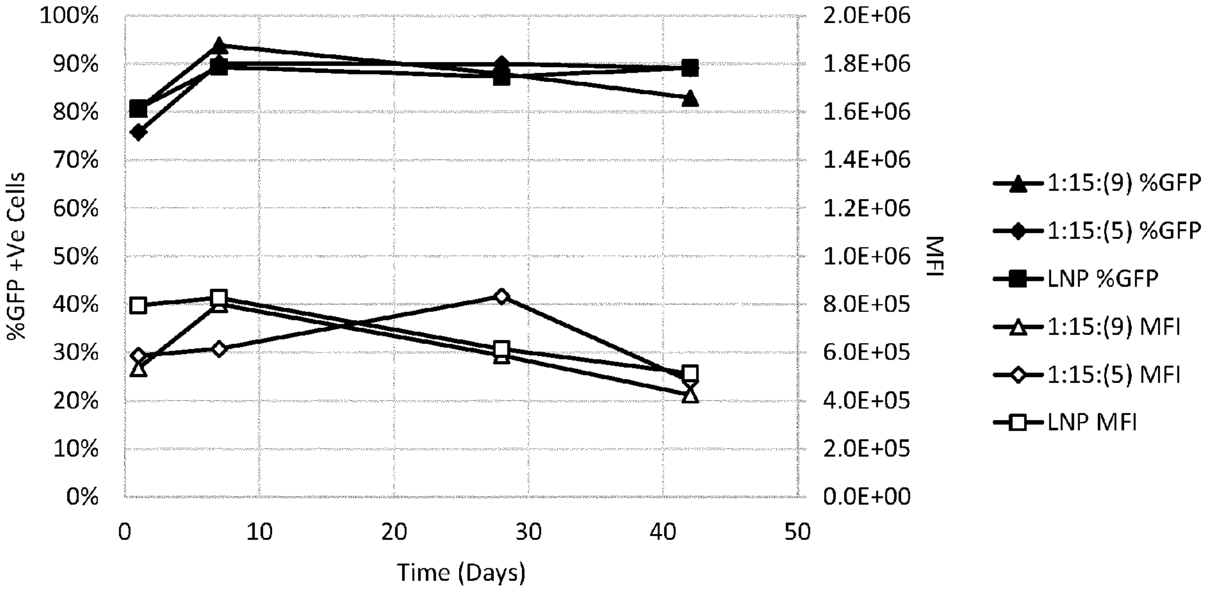
100%
90%
80%
70%
60%
50%
40%
30%
20%
10%
0%
%GFP +Ve Cells
2.0E+06
1.8E+06
1.6E+06
1.4E+06
1.2E+06
1.0E+06
8.0E+05
6.0E+05
4.0E+05
2.0E+05
0.0E+00
MFI
0
10
20
30
40
50
Time (Days)
1:15:(9) %GFP
1:15:(5) %GFP
LNP %GFP
1:15:(9) MFI
1:15:(5) MFI
LNP MFI

Fig. 11

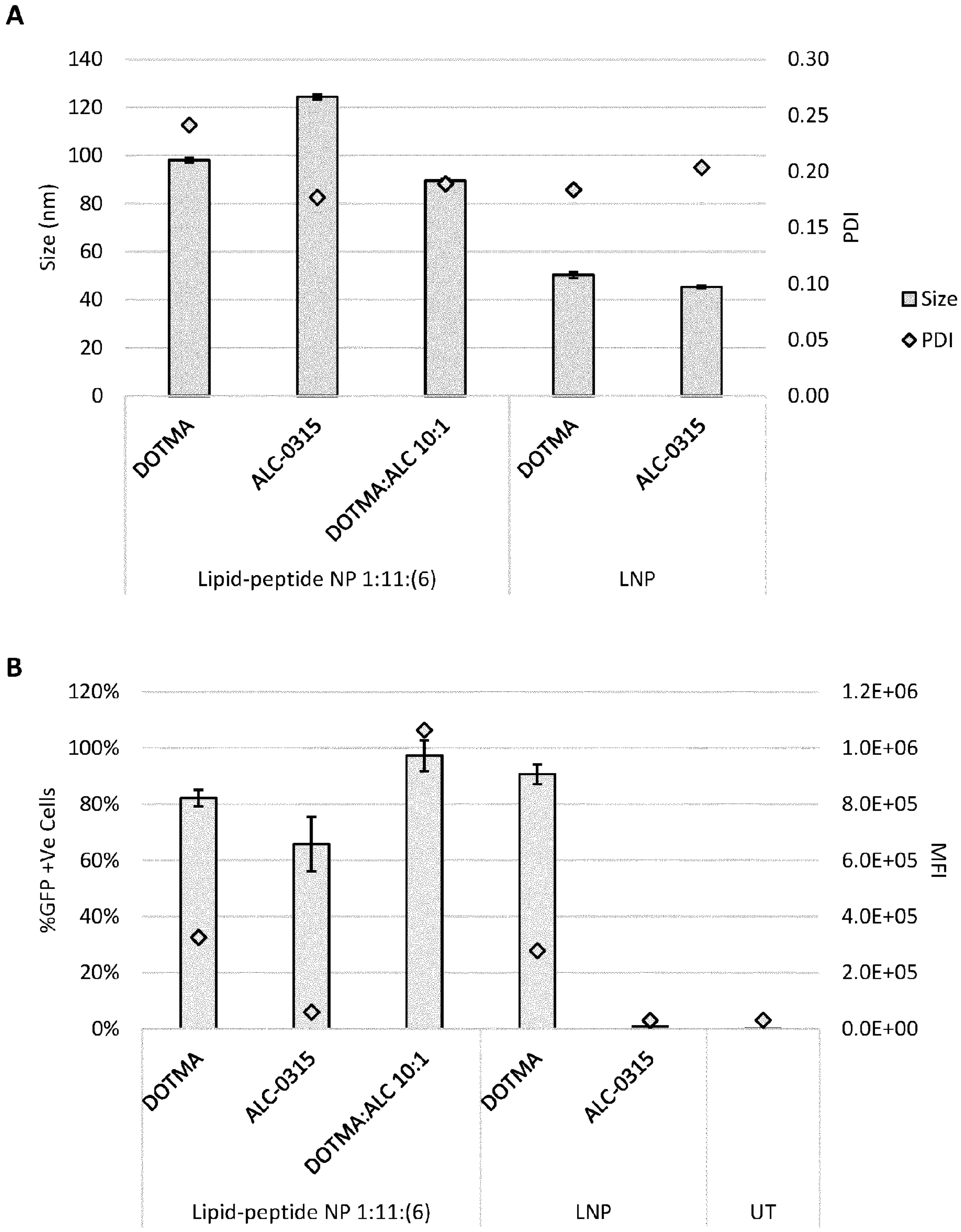
A
140
120
100
80
60
40
20
0
Size (nm)
0.30
0.25
0.20
0.15
0.10
0.05
0.00
PDI
Size
PDI
DOTMA
ALC-0315
DOTMA:ALC 10:1
DOTMA
ALC-0315
Lipid-peptide NP 1:11:(6)
LNP
B
120%
100%
80%
60%
40%
20%
0%
%GFP +Ve Cells
1.2E+06
1.0E+06
8.0E+05
6.0E+05
4.0E+05
2.0E+05
0.0E+00
MFI
DOTMA
ALC-0315
DOTMA:ALC 10:1
DOTMA
ALC-0315
Lipid-peptide NP 1:11:(6)
LNP
UT

Europäisches Patentamt
European Patent Office
Office européen des brevets

# EUROPEAN SEARCH REPORT

Application Number
EP 23 38 2091

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2019/015501 A1 (CIARAMELLA GIUSEPPE [US] ET AL) 17 January 2019 (2019-01-17)<br>* paragraph [0860]; claims 1-23 * | 1-7, 11-13,15<br>8-10,14 | INV.<br>A61K47/69 |
| A | US 2022/168222 A1 (HEYES JAMES [CA] ET AL) 2 June 2022 (2022-06-02)<br>* claims 70-77 * | 1-15 | |
| A | US 2020/247861 A1 (DE FOUGEROLLES ANTONIN [BE] ET AL) 6 August 2020 (2020-08-06)<br>* claims 1-10 * | 1-15 | |
| A | US 2021/346306 A1 (DIMITROV STOIL [US] ET AL) 11 November 2021 (2021-11-11)<br>* claims 1, 10, 12, 17 * | 1-15 | |
| A | US 10 772 975 B2 (MODERNATX INC [US]) 15 September 2020 (2020-09-15)<br>* claims 1-11 * | 1-15 | |

TECHNICAL FIELDS SEARCHED (IPC)

A61K

The present search report has been drawn up for all claims

1

| Place of search | Date of completion of the search | Examiner |
|---|---|---|
| The Hague | 18 July 2023 | Jetter, Sonya |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 38 2091

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| US 2019015501 A1 | 17-01-2019 | AU 2015249553 A1 | 10-11-2016 |
| | | AU 2021203492 A1 | 24-06-2021 |
| | | BR 112016024644 A2 | 10-10-2017 |
| | | CA 2946751 A1 | 29-10-2015 |
| | | CA 3177878 A1 | 29-10-2015 |
| | | CN 106659803 A | 10-05-2017 |
| | | DK 3134131 T3 | 07-02-2022 |
| | | EP 3134131 A1 | 01-03-2017 |
| | | EP 3981437 A1 | 13-04-2022 |
| | | EP 4023249 A1 | 06-07-2022 |
| | | ES 2909180 T3 | 05-05-2022 |
| | | HR P20220070 T1 | 01-04-2022 |
| | | HU E057800 T2 | 28-06-2022 |
| | | JP 6881813 B2 | 02-06-2021 |
| | | JP 2017513956 A | 01-06-2017 |
| | | JP 2021091689 A | 17-06-2021 |
| | | LT 3134131 T | 10-02-2022 |
| | | PL 3134131 T3 | 11-04-2022 |
| | | PT 3134131 T | 24-03-2022 |
| | | RS 63050 B1 | 29-04-2022 |
| | | RU 2021109685 A | 13-04-2021 |
| | | SG 10201912038T A | 27-02-2020 |
| | | SG 11201608798Y A | 29-11-2016 |
| | | SI 3134131 T1 | 29-04-2022 |
| | | US 2016317647 A1 | 03-11-2016 |
| | | US 2016331828 A1 | 17-11-2016 |
| | | US 2019000959 A1 | 03-01-2019 |
| | | US 2019008948 A1 | 10-01-2019 |
| | | US 2019015501 A1 | 17-01-2019 |
| | | US 2021220467 A1 | 22-07-2021 |
| | | US 2022193223 A1 | 23-06-2022 |
| | | WO 2015164674 A1 | 29-10-2015 |
| US 2022168222 A1 | 02-06-2022 | AU 2020262437 A1 | 23-12-2021 |
| | | CA 3137450 A1 | 29-10-2020 |
| | | CN 114026233 A | 08-02-2022 |
| | | EP 3959314 A1 | 02-03-2022 |
| | | JP 2022530018 A | 27-06-2022 |
| | | MA 55766 A | 02-03-2022 |
| | | US 2022168222 A1 | 02-06-2022 |
| | | WO 2020219941 A1 | 29-10-2020 |
| US 2020247861 A1 | 06-08-2020 | US 2018002393 A1 | 04-01-2018 |
| | | US 2020247861 A1 | 06-08-2020 |
| US 2021346306 A1 | 11-11-2021 | EP 3796893 A1 | 31-03-2021 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 38 2091

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| | | MA 52709 A | 31-03-2021 |
| | | US 2021346306 A1 | 11-11-2021 |
| | | WO 2019226650 A1 | 28-11-2019 |
| US 10772975 B2 | 15-09-2020 | AU 2013243834 A1 | 30-10-2014 |
| | | AU 2013243946 A1 | 30-10-2014 |
| | | AU 2013243947 A1 | 30-10-2014 |
| | | AU 2013243948 A1 | 30-10-2014 |
| | | AU 2013243950 A1 | 30-10-2014 |
| | | AU 2013243951 A1 | 30-10-2014 |
| | | AU 2013243952 A1 | 30-10-2014 |
| | | AU 2013243953 A1 | 30-10-2014 |
| | | AU 2013243954 A1 | 30-10-2014 |
| | | AU 2013243955 A1 | 30-10-2014 |
| | | AU 2017232121 A1 | 12-10-2017 |
| | | AU 2018200373 A1 | 22-03-2018 |
| | | AU 2018200375 A1 | 22-03-2018 |
| | | AU 2018200377 A1 | 22-03-2018 |
| | | AU 2018200379 A1 | 22-03-2018 |
| | | AU 2018200380 A1 | 22-03-2018 |
| | | AU 2018200381 A1 | 01-02-2018 |
| | | AU 2018247318 A1 | 08-11-2018 |
| | | AU 2018260928 A1 | 06-12-2018 |
| | | AU 2019202835 A1 | 16-05-2019 |
| | | AU 2020257150 A1 | 19-11-2020 |
| | | CA 2868393 A1 | 10-10-2013 |
| | | CA 2868398 A1 | 10-10-2013 |
| | | CA 2868418 A1 | 10-10-2013 |
| | | CA 2868422 A1 | 10-10-2013 |
| | | CA 2868429 A1 | 10-10-2013 |
| | | CA 2868434 A1 | 10-10-2013 |
| | | CA 2868438 A1 | 10-10-2013 |
| | | CA 2868440 A1 | 10-10-2013 |
| | | CA 2868996 A1 | 10-10-2013 |
| | | CA 2869005 A1 | 10-10-2013 |
| | | CN 104870022 A | 26-08-2015 |
| | | CN 112390871 A | 23-02-2021 |
| | | DE 18200782 T1 | 21-10-2021 |
| | | DE 18203666 T1 | 07-10-2021 |
| | | EP 2833892 A2 | 11-02-2015 |
| | | EP 2833894 A1 | 11-02-2015 |
| | | EP 2833920 A2 | 11-02-2015 |
| | | EP 2833921 A2 | 11-02-2015 |
| | | EP 2833922 A2 | 11-02-2015 |
| | | EP 2833923 A1 | 11-02-2015 |
| | | EP 2834259 A1 | 11-02-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 23 38 2091

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| | | EP 2834260 A1 | 11-02-2015 |
| | | EP 2834358 A2 | 11-02-2015 |
| | | EP 2847329 A1 | 18-03-2015 |
| | | EP 2849799 A2 | 25-03-2015 |
| | | EP 3501550 A1 | 26-06-2019 |
| | | EP 3505176 A1 | 03-07-2019 |
| | | EP 3520820 A1 | 07-08-2019 |
| | | EP 3520821 A1 | 07-08-2019 |
| | | EP 3978030 A1 | 06-04-2022 |
| | | HK 1206601 A1 | 15-01-2016 |
| | | HK 1206635 A1 | 15-01-2016 |
| | | HK 1206636 A1 | 15-01-2016 |
| | | HK 1206638 A1 | 15-01-2016 |
| | | HK 1206639 A1 | 15-01-2016 |
| | | HK 1206748 A1 | 15-01-2016 |
| | | HK 1206779 A1 | 15-01-2016 |
| | | HK 1206780 A1 | 12-02-2016 |
| | | HK 1207306 A1 | 29-01-2016 |
| | | JP 6189415 B2 | 30-08-2017 |
| | | JP 6348482 B2 | 27-06-2018 |
| | | JP 6424156 B2 | 14-11-2018 |
| | | JP 6426217 B2 | 21-11-2018 |
| | | JP 6449356 B2 | 09-01-2019 |
| | | JP 6553104 B2 | 31-07-2019 |
| | | JP 6946384 B2 | 06-10-2021 |
| | | JP 6953135 B2 | 27-10-2021 |
| | | JP 6953135 B6 | 14-01-2022 |
| | | JP 6953135 B6 | 21-01-2022 |
| | | JP 6953135 B6 | 24-11-2021 |
| | | JP 6971953 B2 | 24-11-2021 |
| | | JP 6971953 B6 | 17-01-2022 |
| | | JP 2015513912 A | 18-05-2015 |
| | | JP 2015513913 A | 18-05-2015 |
| | | JP 2015513914 A | 18-05-2015 |
| | | JP 2015513916 A | 18-05-2015 |
| | | JP 2015516143 A | 08-06-2015 |
| | | JP 2015517995 A | 25-06-2015 |
| | | JP 2015518704 A | 06-07-2015 |
| | | JP 2015518816 A | 06-07-2015 |
| | | JP 2015519040 A | 09-07-2015 |
| | | JP 2015519881 A | 16-07-2015 |
| | | JP 2017121239 A | 13-07-2017 |
| | | JP 2017121240 A | 13-07-2017 |
| | | JP 2017121241 A | 13-07-2017 |
| | | JP 2017121243 A | 13-07-2017 |
| | | JP 2017123847 A | 20-07-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 23 38 2091

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| | | JP 2017123853 A | 20-07-2017 |
| | | JP 2017141230 A | 17-08-2017 |
| | | JP 2017197545 A | 02-11-2017 |
| | | JP 2018023373 A | 15-02-2018 |
| | | JP 2019033757 A | 07-03-2019 |
| | | JP 2019054813 A | 11-04-2019 |
| | | JP 2019107022 A | 04-07-2019 |
| | | JP 2019216733 A | 26-12-2019 |
| | | JP 2020072670 A | 14-05-2020 |
| | | JP 2020158508 A | 01-10-2020 |
| | | JP 2021045163 A | 25-03-2021 |
| | | JP 2022023036 A | 07-02-2022 |
| | | JP 2022036938 A | 08-03-2022 |
| | | JP 2022078081 A | 24-05-2022 |
| | | JP 2022122923 A | 23-08-2022 |
| | | JP 2023021215 A | 10-02-2023 |
| | | US 2013259923 A1 | 03-10-2013 |
| | | US 2013259924 A1 | 03-10-2013 |
| | | US 2014010861 A1 | 09-01-2014 |
| | | US 2014105964 A1 | 17-04-2014 |
| | | US 2014105965 A1 | 17-04-2014 |
| | | US 2014105966 A1 | 17-04-2014 |
| | | US 2014107189 A1 | 17-04-2014 |
| | | US 2014113959 A1 | 24-04-2014 |
| | | US 2014141067 A1 | 22-05-2014 |
| | | US 2014148502 A1 | 29-05-2014 |
| | | US 2014155472 A1 | 05-06-2014 |
| | | US 2014155473 A1 | 05-06-2014 |
| | | US 2014155474 A1 | 05-06-2014 |
| | | US 2014155475 A1 | 05-06-2014 |
| | | US 2014161873 A1 | 12-06-2014 |
| | | US 2014171485 A1 | 19-06-2014 |
| | | US 2014179771 A1 | 26-06-2014 |
| | | US 2014186432 A1 | 03-07-2014 |
| | | US 2014193482 A1 | 10-07-2014 |
| | | US 2014194494 A1 | 10-07-2014 |
| | | US 2014200262 A1 | 17-07-2014 |
| | | US 2014200263 A1 | 17-07-2014 |
| | | US 2014200264 A1 | 17-07-2014 |
| | | US 2014206755 A1 | 24-07-2014 |
| | | US 2014221465 A1 | 07-08-2014 |
| | | US 2014249208 A1 | 04-09-2014 |
| | | US 2014255467 A1 | 11-09-2014 |
| | | US 2014255468 A1 | 11-09-2014 |
| | | US 2015044277 A1 | 12-02-2015 |
| | | US 2016075733 A1 | 17-03-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 23 38 2091

This annex lists the patent family members relating to the patent documents cited in the above-mentioned European search report. The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| | | US 2017348436 A1 | 07-12-2017 |
| | | US 2017368200 A1 | 28-12-2017 |
| | | US 2018311381 A1 | 01-11-2018 |
| | | US 2019240351 A1 | 08-08-2019 |
| | | US 2019314527 A1 | 17-10-2019 |
| | | US 2020155706 A1 | 21-05-2020 |
| | | US 2021077634 A1 | 18-03-2021 |
| | | US 2021299278 A1 | 30-09-2021 |
| | | WO 2013151663 A1 | 10-10-2013 |
| | | WO 2013151664 A1 | 10-10-2013 |
| | | WO 2013151665 A2 | 10-10-2013 |
| | | WO 2013151667 A1 | 10-10-2013 |
| | | WO 2013151668 A2 | 10-10-2013 |
| | | WO 2013151669 A1 | 10-10-2013 |
| | | WO 2013151670 A2 | 10-10-2013 |
| | | WO 2013151671 A1 | 10-10-2013 |
| | | WO 2013151672 A2 | 10-10-2013 |
| | | WO 2013151736 A2 | 10-10-2013 |

---

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ERBACHER, P. et al.** *Gene Therapy,* 1999, vol. 6, 138-145
- **FELGNER et al.** *Human Gene Therapy,* 1997, vol. 8, 511-512
- *Nano Lett.,* 2020, vol. 20 (6), 4543-4549